# EUROPEAN PATENT APPLICATION

(11) **EP 2 157 100 A1**
(43) Date of publication of application: **24.02.2010**
(21) Application number: 08162644.2
(22) Date of filing: 19.08.2008
(51) Int. Cl.: C07K 14/435, C12N 9/14

(54) **Artificial peptidoglycan lysing enzymes and peptidoglycan binding proteins**

(71) Applicant: Profos AG, 93053 Regensburg (DE)
(72) Inventor: Loessner, Martin, 8123 Ebmatingen (CH); Schmelcher, Mathias, 86947 Schwabhausen (DE); Grallert, Holger, 93053 Regensburg (DE); Bretfeld, Falko, 93047 Regensburg (DE)
(74) Representative: Dehmel, Albrecht

(57) **Abstract**

The present invention relates to recombinant polypeptides having the activity of binding and lysing of bacteria, comprising at least one enzymatically active domain and at least two bacterial cell binding domains. The present invention further relates to recombinant polypeptide having the activity of binding bacteria, comprising at least two bacterial cell binding domain. Further the present inventions relates to nucleic acid molecules comprising a nucleotide sequence encoding the recombinant polypeptides, vectors and host cells.

SEQUENCE LISTING

<110>  PROFOS AG

<120>  Artificial peptidoglycan lysing enzymes and peptidoglycan binding
        proteins

<130>  PRO-031 EP

<140>  unknown
<141>  2008-08-19

<160>  102

<170>  PatentIn version 3.3


<210>  1
<211>  289
<212>  PRT
<213>  Bacteriophage A500

<400>  1

Met Ala Leu Thr Glu Ala Trp Leu Ile Glu Lys Ala Asn Arg Lys Leu
1               5                   10                  15
Asn Ala Gly Gly Met Tyr Lys Ile Thr Ser Asp Lys Thr Arg Asn Val
            20                  25                  30
Ile Lys Lys Met Ala Lys Glu Gly Ile Tyr Leu Cys Val Ala Gln Gly
        35                  40                  45
Tyr Arg Ser Thr Ala Glu Gln Asn Ala Leu Tyr Ala Gln Gly Arg Thr
    50                  55                  60
Lys Pro Gly Ala Ile Val Thr Asn Ala Lys Gly Gly Gln Ser Asn His
65                  70                  75                  80
Asn Tyr Gly Val Ala Val Asp Leu Cys Leu Tyr Thr Asn Asp Gly Lys
                85                  90                  95
Asp Val Ile Trp Glu Ser Thr Thr Ser Arg Trp Lys Lys Val Val Ala
            100                 105                 110
Ala Met Lys Ala Glu Gly Phe Lys Trp Gly Gly Asp Trp Lys Ser Phe
        115                 120                 125
Lys Asp Tyr Pro His Phe Glu Leu Cys Asp Ala Val Ser Gly Glu Lys
    130                 135                 140
Ile Pro Ala Ala Thr Gln Asn Thr Asn Thr Asn Ser Asn Arg Tyr Glu
145                 150                 155                 160
Gly Lys Val Ile Asp Ser Ala Pro Leu Leu Pro Lys Met Asp Phe Lys
                165                 170                 175
Ser Ser Pro Phe Arg Met Tyr Lys Val Gly Thr Glu Phe Leu Val Tyr
            180                 185                 190
Asp His Asn Gln Tyr Trp Tyr Lys Thr Tyr Ile Asp Asp Lys Leu Tyr
        195                 200                 205
Tyr Met Tyr Lys Ser Phe Cys Asp Val Val Ala Lys Lys Asp Ala Lys
    210                 215                 220
Gly Arg Ile Lys Val Arg Ile Lys Ser Ala Lys Asp Leu Arg Ile Pro
225                 230                 235                 240
Val Trp Asn Asn Ile Lys Leu Asn Ser Gly Lys Ile Lys Trp Tyr Ala
                245                 250                 255
Pro Asn Val Lys Leu Ala Trp Tyr Asn Tyr Arg Arg Gly Tyr Leu Glu
            260                 265                 270
Leu Trp Tyr Pro Asn Asp Gly Trp Tyr Tyr Thr Ala Glu Tyr Phe Leu
        275                 280                 285
Lys

<210>  2
<211>  870
<212>  DNA
<213>  Bacteriophage A500

<400>  2
atggcattaa cagaggcatg gctaattgaa aaagcaaatc gcaaattgaa tgctggggga      60
atgtataaaa ttacatcgga taaaacacga aatgtaatta aaaaaatggc aaaagaaggt     120
atttatcttt gtgttgcgca aggttaccgc tcaacagcgg aacaaaatgc gctatatgca     180
caagggagaa ccaaacctgg agcaattgtt actaatgcca agggcgggca atctaatcac     240
aactacgggg tagctgttga cttgtgcttg tatacaaatg acggaaaaga tgttatttgg     300
gagtcaacaa cttcccggtg gaaaaaggtt gttgctgcta tgaaagcaga agggtttaaa     360
tggggcggag actggaaaag ttttaaagac tatccgcatt ttgaactatg tgatgctgta     420
agtggtgaga aaatccctgc tgcaacacaa aacactaata caaattcaaa tcgttacgag     480
ggtaaagtca ttgatagcgc accactgcta ccgaaaatgg actttaaatc atcaccattc     540
cgcatgtata aggtaggaac tgagttctta gtatatgatc ataatcaata ttggtacaag     600
acatacattg atgacaaact ttactacatg tataaaagct tttgcgatgt tgtagctaaa     660
aaagacgcaa aaggtcgcat caaagttcga attaaaagcg cgaaagactt gcgtattcca     720
gtctggaata acataaaatt gaattctggg aaaattaaat ggtatgcacc caatgtaaaa     780
ctagcgtggt acaactatcg aagaggatat ttagagctat ggtatccgaa cgacggctgg     840
tattacacag cagaatactt cttaaaataa                                      870

<210>  3
<211>  281
<212>  PRT
<213>  Bacteriophage A118

<400>  3
Met Thr Ser Tyr Tyr Tyr Ser Arg Ser Leu Ala Asn Val Asn Lys Leu
1               5                   10                  15
Ala Asp Asn Thr Lys Ala Ala Ala Arg Lys Leu Leu Asp Trp Ser Glu
            20                  25                  30
Ser Asn Gly Ile Glu Val Leu Ile Tyr Glu Thr Ile Arg Thr Lys Glu
        35                  40                  45
Gln Gln Ala Ala Asn Val Asn Ser Gly Ala Ser Gln Thr Met Arg Ser
    50                  55                  60
Tyr His Leu Val Gly Gln Ala Leu Asp Phe Val Met Ala Lys Gly Lys
65                  70                  75                  80
Thr Val Asp Trp Gly Ala Tyr Arg Ser Asp Lys Gly Lys Lys Phe Val
                85                  90                  95
Ala Lys Ala Lys Ser Leu Gly Phe Glu Trp Gly Gly Asp Trp Ser Gly
            100                 105                 110
Phe Val Asp Asn Pro His Leu Gln Phe Asn Tyr Lys Gly Tyr Gly Thr
        115                 120                 125
Asp Thr Phe Gly Lys Gly Ala Ser Thr Ser Asn Ser Ser Lys Pro Ser
    130                 135                 140
Ala Asp Thr Asn Thr Asn Ser Leu Gly Leu Val Asp Tyr Met Asn Leu
145                 150                 155                 160
Asn Lys Leu Asp Ser Ser Phe Ala Asn Arg Lys Lys Leu Ala Thr Ser
                165                 170                 175
Tyr Gly Ile Lys Asn Tyr Ser Gly Thr Ala Thr Gln Asn Thr Thr Leu
            180                 185                 190
Leu Ala Lys Leu Lys Ala Gly Lys Pro His Thr Pro Ala Ser Lys Asn
        195                 200                 205
Thr Tyr Tyr Thr Glu Asn Pro Arg Lys Val Lys Thr Leu Val Gln Cys
    210                 215                 220
Asp Leu Tyr Lys Ser Val Asp Phe Thr Thr Lys Asn Gln Thr Gly Gly
225                 230                 235                 240
Thr Phe Pro Pro Gly Thr Val Phe Thr Ile Ser Gly Met Gly Lys Thr
                245                 250                 255
Lys Gly Gly Thr Pro Arg Leu Lys Thr Lys Ser Gly Tyr Tyr Leu Thr

260                 265                 270
Ala Asn Thr Lys Phe Val Lys Lys Ile
        275                 280

<210>  4
<211>  846
<212>  DNA
<213>  Bacteriophage A118

<400>  4
atgacaagtt attattatag tagaagttta gcgaatgtaa ataagttagc agacaatacg      60
aaagcggcac gtagaaaatt gctagattgg tctgaaagca acgggattga agtattaatc     120
tacgaaacaa ttagaacgaa agaacaacaa gccgcaaatg ttaacagtgg agcgtctcaa     180
acaatgcgct cttatcactt ggtaggacaa gcgctagatt tcgtcatggc gaaaggtaaa     240
acggtcgatt ggggtgctta tcgttcagac aaaggcaaga aatttgtggc aaaggcaaaa     300
tctttaggtt ttgagtgggg tggtgattgg tctggatttg tagacaatcc gcaccttcaa     360
tttaattata aaggctatgg gactgatact tttggaaaag gagctagtac tagtaattca     420
tctaaaccga gcgcagacac aaacacaaac agtctaggat tagtagatta tatgaattta     480
aataaactag attcaagctt tgcgaatcgc aaaaaactag cgacaagtta cggaattaaa     540
aattacagtg gaacagcaac gcagaacaca acattattag cgaagttaaa agcaggaaaa     600
ccacacacac cagcaagcaa aaacacatac tacacagaaa atccgcgaaa agttaaaaca     660
ctagtacaat gtgatctata caaatcagta gactttacaa caaaaaacca aacaggtgga     720
acatttccgc caggcacagt cttcacgatt tcagggatgg ggaaaacgaa aggcggaaca     780
cctcgcttga agacgaagag cggttactat ctcactgcta acacgaagtt tgttaaaaag     840
atttag                                                                846

<210>  5
<211>  291
<212>  PRT
<213>  Bacteriophage P35

<400>  5
Met Ala Arg Lys Phe Thr Lys Ala Glu Leu Val Ala Lys Ala Glu Lys
1               5                   10                  15
Lys Val Gly Gly Leu Lys Pro Asp Val Lys Lys Ala Val Leu Ser Ala
            20                  25                  30
Val Lys Glu Ala Tyr Asp Arg Tyr Gly Ile Gly Ile Ile Val Ser Gln
        35                  40                  45
Gly Tyr Arg Ser Ile Ala Glu Gln Asn Gly Leu Tyr Ala Gln Gly Arg
    50                  55                  60
Thr Lys Pro Gly Asn Ile Val Thr Asn Ala Lys Gly Gly Gln Ser Asn
65                  70                  75                  80
His Asn Phe Gly Val Ala Val Asp Phe Ala Ile Asp Leu Ile Asp Asp
                85                  90                  95
Gly Lys Ile Asp Ser Trp Gln Pro Ser Ala Thr Ile Val Asn Met Met
            100                 105                 110
Lys Arg Arg Gly Phe Lys Trp Gly Gly Asp Trp Lys Ser Phe Thr Asp
        115                 120                 125
Leu Pro His Phe Glu Ala Cys Asp Trp Tyr Arg Gly Glu Arg Lys Tyr
    130                 135                 140
Lys Val Asp Thr Ser Glu Trp Lys Lys Lys Glu Asn Ile Asn Ile Val
145                 150                 155                 160
Ile Lys Asp Val Gly Tyr Phe Gln Asp Lys Pro Gln Phe Leu Asn Ser
                165                 170                 175
Lys Ser Val Arg Gln Trp Lys His Gly Thr Lys Val Lys Leu Thr Lys
            180                 185                 190
His Asn Ser His Trp Tyr Thr Gly Val Val Lys Asp Gly Asn Lys Ser
        195                 200                 205
Val Arg Gly Tyr Ile Tyr His Ser Met Ala Lys Val Thr Ser Lys Asn
    210                 215                 220
Ser Asp Gly Ser Val Asn Ala Thr Ile Asn Ala His Ala Phe Cys Trp

225                 230                 235                 240
Asp Asn Lys Lys Leu Asn Gly Gly Asp Phe Ile Asn Leu Lys Arg Gly
                245                 250                 255
Phe Lys Gly Ile Thr His Pro Ala Ser Asp Gly Phe Tyr Pro Leu Tyr
            260                 265                 270
Phe Ala Ser Arg Lys Lys Thr Phe Tyr Ile Pro Arg Tyr Met Phe Asp
        275                 280                 285
Ile Lys Lys
    290

<210>  6
<211>  876
<212>  DNA
<213>  Bacteriophage P35

<400>  6
atggcacgaa aatttacaaa agctgaactg gtagctaaag cagaaaagaa agtcggtgga      60
ttaaaacccg acgtaaagaa agcagtattg tccgcagtga aggaagcata tgaccgctat     120
ggtattggga ttatcgtatc acagggttat cgttcaattg ctgaacaaaa cggattgtat     180
gcacaaggtc ggaccaaacc ggggaacatt gtgaccaacg caaaaggtgg acaatctaac     240
cataactttg gtgttgccgt tgactttgct attgacttga ttgacgatgg taaaatcgac     300
tcttggcaac catcagcaac cattgtgaac atgatgaaac gtcgtgggtt caaatggggc     360
ggagattgga aaagctttac tgaccttcca cattttgaag cttgtgactg gtatcgcggg     420
gaacgcaagt ataaagtgga cacatctgaa tggaaaaaga aagagaatat caatatcgtt     480
attaaagatg ttggttactt ccaagacaaa cctcaattct taaactccaa atcggttcgt     540
cagtggaagc atggcacgaa agtgaagctt actaaacata actcacattg gtacactggt     600
gtggtcaagg atggtaacaa atcagtcagg ggatatattt atcattcgat ggctaaggtc     660
acaagcaaga atagcgacgg ttcggttaac gcaacgatta acgcccacgc attttgttgg     720
gacaataaaa aacttaatgg tggcgacttt atcaacttga agcgtggttt taaaggtatc     780
acccatcccg ctagtgacgg tttctatcca ctgtatttcg cttctaggaa aaaaactttc     840
tacattccgc gttacatgtt tgacatcaag aaatga                               876

<210>  7
<211>  363
<212>  PRT
<213>  artificial sequence

<220>
<223>  CBD500-118:

<400>  7

Ile Thr His Gly Met Asp Glu Leu Tyr Lys Glu Leu His Phe Glu Leu
1               5                   10                  15
Cys Asp Ala Val Ser Gly Glu Lys Ile Pro Ala Ala Thr Gln Asn Thr
            20                  25                  30
Asn Thr Asn Ser Asn Arg Tyr Glu Gly Lys Val Ile Asp Ser Ala Pro
        35                  40                  45
Leu Leu Pro Lys Met Asp Phe Lys Ser Ser Pro Phe Arg Met Tyr Lys
    50                  55                  60
Val Gly Thr Glu Phe Leu Val Tyr Asp His Asn Gln Tyr Trp Tyr Lys
65                  70                  75                  80
Thr Tyr Ile Asp Asp Lys Leu Tyr Tyr Met Tyr Lys Ser Phe Cys Asp
                85                  90                  95
Val Val Ala Lys Lys Asp Ala Lys Gly Arg Ile Lys Val Arg Ile Lys
            100                 105                 110
Ser Ala Lys Asp Leu Arg Ile Pro Val Trp Asn Asn Ile Lys Leu Asn
        115                 120                 125
Ser Gly Lys Ile Lys Trp Tyr Ala Pro Asn Val Lys Leu Ala Trp Tyr
    130                 135                 140
Asn Tyr Arg Arg Gly Tyr Leu Glu Leu Trp Tyr Pro Asn Asp Gly Trp

145                 150                 155                 160
Tyr Tyr Thr Ala Glu Tyr Phe Leu Lys Gln Phe Asp Lys Gly Lys Lys
                165                 170                 175
Phe Val Ala Lys Ala Lys Ser Leu Gly Phe Glu Trp Gly Gly Asp Trp
            180                 185                 190
Ser Gly Phe Val Asp Asn Pro His Leu Gln Phe Asn Tyr Lys Gly Tyr
        195                 200                 205
Gly Thr Asp Thr Phe Gly Lys Gly Ala Ser Thr Ser Asn Ser Ser Lys
    210                 215                 220
Pro Ser Ala Asp Thr Asn Thr Asn Ser Leu Gly Leu Val Asp Tyr Met
225                 230                 235                 240
Asn Leu Asn Lys Leu Asp Ser Ser Phe Ala Asn Arg Lys Lys Leu Ala
                245                 250                 255
Thr Ser Tyr Gly Ile Lys Asn Tyr Ser Gly Thr Ala Thr Gln Asn Thr
            260                 265                 270
Thr Leu Leu Ala Lys Leu Lys Ala Gly Lys Pro His Thr Pro Ala Ser
        275                 280                 285
Lys Asn Thr Tyr Tyr Thr Glu Asn Pro Arg Lys Val Lys Thr Leu Val
    290                 295                 300
Gln Cys Asp Leu Tyr Lys Ser Val Asp Phe Thr Thr Lys Asn Gln Thr
305                 310                 315                 320
Gly Gly Thr Phe Pro Pro Gly Thr Val Phe Thr Ile Ser Gly Met Gly
                325                 330                 335
Lys Thr Lys Gly Gly Thr Pro Arg Leu Lys Thr Lys Ser Gly Tyr Tyr
            340                 345                 350
Leu Thr Ala Asn Thr Lys Phe Val Lys Lys Ile
        355                 360

<210>  8
<211>  1092
<212>  DNA
<213>  artificial sequence

<220>
<223>  CBD500-118:

<400>  8
attacacatg gcatggatga actatacaaa gagctccatt ttgaactatg tgatgctgta      60
agtggtgaga aaatccctgc tgcaacacaa aacactaata caaattcaaa tcgttacgag     120
ggtaaagtca ttgatagcgc accactgcta ccgaaaatgg actttaaatc atcaccattc     180
cgcatgtata aggtaggaac tgagttctta gtatatgatc ataatcaata ttggtacaag     240
acatacattg atgacaaact ttactacatg tataaaagct tttgcgatgt tgtagctaaa     300
aaagacgcaa aaggtcgcat caaagttcga attaaaagcg cgaaagactt gcgtattcca     360
gtctggaata acataaaatt gaattctggg aaaattaaat ggtatgcacc caatgtaaaa     420
ctagcgtggt acaactatcg aagaggatat ttagagctat ggtatccgaa cgacggctgg     480
tattacacag cagaatactt cttaaaacaa ttcgacaaag gcaagaaatt tgtggcaaag     540
gcaaaatctt taggttttga gtggggtggt gattggtctg gatttgtaga caatccgcac     600
cttcaattta attataaagg ctatgggact gatacttttg gaaaaggagc tagtactagt     660
aattcatcta aaccgagcgc agacacaaac acaaacagtc taggattagt agattatatg     720
aatttaaata aactagattc aagctttgcg aatcgcaaaa aactagcgac aagttacgga     780
attaaaaatt acagtggaac agcaacgcag aacacaacat tattagcgaa gttaaaagca     840
ggaaaaccac acacaccagc aagcaaaaac acatactaca cagaaaatcc gcgaaaagtt     900
aaaacactag tacaatgtga tctatacaaa tcagtagact ttacaacaaa aaaccaaaca     960
ggtggaacat ttccgccagg cacagtcttc acgatttcag ggatggggaa aacgaaaggc    1020
ggaacacctc gcttgaagac gaagagcggt tactatctca ctgctaacac gaagtttgtt    1080
aaaaagattt aa                                                        1092

<210>  9
<211>  348
<212>  PRT
<213>  artificial sequence

<220>
<223>  CBD500L118:

<400>  9
Ile Thr His Gly Met Asp Glu Leu Tyr Lys Glu Leu Gln Asn Thr Asn
1               5                   10                  15
Thr Asn Ser Asn Arg Tyr Glu Gly Lys Val Ile Asp Ser Ala Pro Leu
            20                  25                  30
Leu Pro Lys Met Asp Phe Lys Ser Ser Pro Phe Arg Met Tyr Lys Val
        35                  40                  45
Gly Thr Glu Phe Leu Val Tyr Asp His Asn Gln Tyr Trp Tyr Lys Thr
    50                  55                  60
Tyr Ile Asp Asp Lys Leu Tyr Tyr Met Tyr Lys Ser Phe Cys Asp Val
65                  70                  75                  80
Val Ala Lys Lys Asp Ala Lys Gly Arg Ile Lys Val Arg Ile Lys Ser
                85                  90                  95
Ala Lys Asp Leu Arg Ile Pro Val Trp Asn Asn Ile Lys Leu Asn Ser
            100                 105                 110
Gly Lys Ile Lys Trp Tyr Ala Pro Asn Val Lys Leu Ala Trp Tyr Asn
        115                 120                 125
Tyr Arg Arg Gly Tyr Leu Glu Leu Trp Tyr Pro Asn Asp Gly Trp Tyr
    130                 135                 140
Tyr Thr Ala Glu Tyr Phe Leu Lys Thr Gly Lys Thr Val Ala Ala Lys
145                 150                 155                 160
Asn Pro Asn Arg His Ser Lys Ser Leu Gly Phe Glu Trp Gly Gly Asp
                165                 170                 175
Trp Ser Gly Phe Val Asp Asn Pro His Leu Gln Phe Asn Tyr Lys Gly
            180                 185                 190
Tyr Gly Thr Asp Thr Phe Gly Lys Gly Ala Ser Thr Ser Asn Ser Ser
        195                 200                 205
Lys Pro Ser Ala Asp Thr Asn Thr Asn Ser Leu Gly Leu Val Asp Tyr
    210                 215                 220
Met Asn Leu Asn Lys Leu Asp Ser Ser Phe Ala Asn Arg Lys Lys Leu
225                 230                 235                 240
Ala Thr Ser Tyr Gly Ile Lys Asn Tyr Ser Gly Thr Ala Thr Gln Asn
                245                 250                 255
Thr Thr Leu Leu Ala Lys Leu Lys Ala Gly Lys Pro His Thr Pro Ala
            260                 265                 270
Ser Lys Asn Thr Tyr Tyr Thr Glu Asn Pro Arg Lys Val Lys Thr Leu
        275                 280                 285
Val Gln Cys Asp Leu Tyr Lys Ser Val Asp Phe Thr Thr Lys Asn Gln
    290                 295                 300
Thr Gly Gly Thr Phe Pro Pro Gly Thr Val Phe Thr Ile Ser Gly Met
305                 310                 315                 320
Gly Lys Thr Lys Gly Gly Thr Pro Arg Leu Lys Thr Lys Ser Gly Tyr
                325                 330                 335
Tyr Leu Thr Ala Asn Thr Lys Phe Val Lys Lys Ile
            340                 345


<210>  10
<211>  1047
<212>  DNA
<213>  artificial sequence

<220>
<223>  CBD500L118:

<400>  10
attacacatg gcatggatga actatacaaa gagctccaaa acactaatac aaattcaaat      60
cgttacgagg gtaaagtcat tgatagcgca ccactgctac cgaaaatgga ctttaaatca     120
tcaccattcc gcatgtataa ggtaggaact gagttcttag tatatgatca taatcaatat     180
tggtacaaga catacattga tgacaaactt tactacatgt ataaaagctt ttgcgatgtt     240

gtagctaaaa aagacgcaaa aggtcgcatc aaagttcgaa ttaaaagcgc gaaagacttg 300
cgtattccag tctggaataa cataaaattg aattctggga aaattaaatg gtatgcaccc 360
aatgtaaaac tagcgtggta caactatcga agaggatatt tagagctatg gtatccgaac 420
gacggctggt attacacagc agaatacttc ttaaaaactg gtaaaacagt agccgcaaaa 480
aatccaaacc gccattctaa atctttaggt tttgagtggg gtggtgattg gtctggattt 540
gtagacaatc cgcaccttca atttaattat aaaggctatg ggactgatac ttttggaaaa 600
ggagctagta ctagtaattc atctaaaccg agcgcagaca caaacacaaa cagtctagga 660
ttagtagatt atatgaattt aaataaacta gattcaagct ttgcgaatcg caaaaaacta 720
gcgacaagtt acggaattaa aaattacagt ggaacagcaa cgcagaacac aacattatta 780
gcgaagttaa aagcaggaaa accacacaca ccagcaagca aaaacacata ctacacagaa 840
aatccgcgaa aagttaaaac actagtacaa tgtgatctat acaaatcagt agactttaca 900
acaaaaaacc aaacaggtgg aacatttccg ccaggcacag tcttcacgat ttcagggatg 960
gggaaaacga aaggcggaac acctcgcttg aagacgaaga gcggttacta tctcactgct 1020
aacacgaagt ttgttaaaaa gatttaa 1047

<210> 11
<211> 14
<212> PRT
<213> artificial sequence

<220>
<223> PlyPSA linker (L)

<400> 11
Thr Gly Lys Thr Val Ala Ala Lys Asn Pro Asn Arg His Ser
1               5                   10

<210> 12
<211> 42
<212> DNA
<213> artificial sequence

<220>
<223> PlyPSA linker (L)

<400> 12
actggtaaaa cagtagccgc aaaaaatcca aaccgccatt ct 42

<210> 13
<211> 363
<212> PRT
<213> artificial sequence

<220>
<223> CBD118-500:

<400> 13
Ile Thr His Gly Met Asp Glu Leu Tyr Lys Glu Leu Asp Lys Gly Lys
1               5                   10                  15
Lys Phe Val Ala Lys Ala Lys Ser Leu Gly Phe Glu Trp Gly Gly Asp
            20                  25                  30
Trp Ser Gly Phe Val Asp Asn Pro His Leu Gln Phe Asn Tyr Lys Gly
        35                  40                  45
Tyr Gly Thr Asp Thr Phe Gly Lys Gly Ala Ser Thr Ser Asn Ser Ser
    50                  55                  60
Lys Pro Ser Ala Asp Thr Asn Thr Asn Ser Leu Gly Leu Val Asp Tyr
65                  70                  75                  80
Met Asn Leu Asn Lys Leu Asp Ser Ser Phe Ala Asn Arg Lys Lys Leu
                85                  90                  95
Ala Thr Ser Tyr Gly Ile Lys Asn Tyr Ser Gly Thr Ala Thr Gln Asn

                100                 105                 110
Thr Thr Leu Leu Ala Lys Leu Lys Ala Gly Lys Pro His Thr Pro Ala
        115                 120                 125
Ser Lys Asn Thr Tyr Tyr Thr Glu Asn Pro Arg Lys Val Lys Thr Leu
    130                 135                 140
Val Gln Cys Asp Leu Tyr Lys Ser Val Asp Phe Thr Thr Lys Asn Gln
145                 150                 155                 160
Thr Gly Gly Thr Phe Pro Pro Gly Thr Val Phe Thr Ile Ser Gly Met
                165                 170                 175
Gly Lys Thr Lys Gly Gly Thr Pro Arg Leu Lys Thr Lys Ser Gly Tyr
            180                 185                 190
Tyr Leu Thr Ala Asn Thr Lys Phe Val Lys Lys Ile Glu Leu His Phe
        195                 200                 205
Glu Leu Cys Asp Ala Val Ser Gly Glu Lys Ile Pro Ala Ala Thr Gln
    210                 215                 220
Asn Thr Asn Thr Asn Ser Asn Arg Tyr Glu Gly Lys Val Ile Asp Ser
225                 230                 235                 240
Ala Pro Leu Leu Pro Lys Met Asp Phe Lys Ser Ser Pro Phe Arg Met
                245                 250                 255
Tyr Lys Val Gly Thr Glu Phe Leu Val Tyr Asp His Asn Gln Tyr Trp
            260                 265                 270
Tyr Lys Thr Tyr Ile Asp Asp Lys Leu Tyr Tyr Met Tyr Lys Ser Phe
        275                 280                 285
Cys Asp Val Val Ala Lys Lys Asp Ala Lys Gly Arg Ile Lys Val Arg
    290                 295                 300
Ile Lys Ser Ala Lys Asp Leu Arg Ile Pro Val Trp Asn Asn Ile Lys
305                 310                 315                 320
Leu Asn Ser Gly Lys Ile Lys Trp Tyr Ala Pro Asn Val Lys Leu Ala
                325                 330                 335
Trp Tyr Asn Tyr Arg Arg Gly Tyr Leu Glu Leu Trp Tyr Pro Asn Asp
            340                 345                 350
Gly Trp Tyr Tyr Thr Ala Glu Tyr Phe Leu Lys
        355                 360


<210>  14
<211>  1092
<212>  DNA
<213>  artificial sequence

<220>
<223>  CBD118-500:

<400>  14
attacacatg gcatggatga actatacaaa gagctcgaca aaggcaagaa atttgtggca      60
aaggcaaaat ctttaggttt tgagtggggt ggtgattggt ctggatttgt agacaatccg     120
caccttcaat ttaattataa aggctatggg actgatactt ttggaaaagg agctagtact     180
agtaattcat ctaaaccgag cgcagacaca aacacaaaca gtctaggatt agtagattat     240
atgaatttaa ataaactaga ttcaagcttt gcgaatcgca aaaaactagc gacaagttac     300
ggaattaaaa attacagtgg aacagcaacg cagaacacaa cattattagc gaagttaaaa     360
gcaggaaaac cacacacacc agcaagcaaa aacacatact acacagaaaa tccgcgaaaa     420
gttaaaacac tagtacaatg tgatctatac aaatcagtag actttacaac aaaaaaccaa     480
acaggtggaa catttccgcc aggcacagtc ttcacgattt cagggatggg gaaaacgaaa     540
ggcggaacac ctcgcttgaa gacgaagagc ggttactatc tcactgctaa cacgaagttt     600
gttaaaaaga ttgaattgca ttttgaacta tgtgatgctg taagtggtga gaaaatccct     660
gctgcaacac aaaacactaa tacaaattca aatcgttacg agggtaaagt cattgatagc     720
gcaccactgc taccgaaaat ggactttaaa tcatcaccat tccgcatgta taaggtagga     780
actgagttct tagtatatga tcataatcaa tattggtaca agacatacat tgatgacaaa     840
ctttactaca tgtataaaag cttttgcgat gttgtagcta aaaaagacgc aaaaggtcgc     900
atcaaagttc gaattaaaag cgcgaaagac ttgcgtattc cagtctggaa taacataaaa     960
ttgaattctg gaaaaattaa atggtatgca cccaatgtaa aactagcgtg gtacaactat    1020
cgaagaggat atttagagct atggtatccg aacgacggct ggtattacac agcagaatac    1080
ttcttaaaat aa                                                        1092

<210>  15
<211>  348
<212>  PRT
<213>  artificial sequence

<220>
<223>  CBD118L500:

<400>  15
Ile Thr His Gly Met Asp Glu Leu Tyr Lys Glu Leu Lys Ser Leu Gly
1               5                   10                  15
Phe Glu Trp Gly Gly Asp Trp Ser Gly Phe Val Asp Asn Pro His Leu
            20                  25                  30
Gln Phe Asn Tyr Lys Gly Tyr Gly Thr Asp Thr Phe Gly Lys Gly Ala
        35                  40                  45
Ser Thr Ser Asn Ser Ser Lys Pro Ser Ala Asp Thr Asn Thr Asn Ser
    50                  55                  60
Leu Gly Leu Val Asp Tyr Met Asn Leu Asn Lys Leu Asp Ser Ser Phe
65                  70                  75                  80
Ala Asn Arg Lys Lys Leu Ala Thr Ser Tyr Gly Ile Lys Asn Tyr Ser
                85                  90                  95
Gly Thr Ala Thr Gln Asn Thr Thr Leu Leu Ala Lys Leu Lys Ala Gly
            100                 105                 110
Lys Pro His Thr Pro Ala Ser Lys Asn Thr Tyr Tyr Thr Glu Asn Pro
        115                 120                 125
Arg Lys Val Lys Thr Leu Val Gln Cys Asp Leu Tyr Lys Ser Val Asp
    130                 135                 140
Phe Thr Thr Lys Asn Gln Thr Gly Gly Thr Phe Pro Pro Gly Thr Val
145                 150                 155                 160
Phe Thr Ile Ser Gly Met Gly Lys Thr Lys Gly Gly Thr Pro Arg Leu
                165                 170                 175
Lys Thr Lys Ser Gly Tyr Tyr Leu Thr Ala Asn Thr Lys Phe Val Lys
            180                 185                 190
Lys Ile Thr Gly Lys Thr Val Ala Ala Lys Asn Pro Asn Arg His Ser
        195                 200                 205
Gln Asn Thr Asn Thr Asn Ser Asn Arg Tyr Glu Gly Lys Val Ile Asp
    210                 215                 220
Ser Ala Pro Leu Leu Pro Lys Met Asp Phe Lys Ser Ser Pro Phe Arg
225                 230                 235                 240
Met Tyr Lys Val Gly Thr Glu Phe Leu Val Tyr Asp His Asn Gln Tyr
                245                 250                 255
Trp Tyr Lys Thr Tyr Ile Asp Asp Lys Leu Tyr Tyr Met Tyr Lys Ser
            260                 265                 270
Phe Cys Asp Val Val Ala Lys Lys Asp Ala Lys Gly Arg Ile Lys Val
        275                 280                 285
Arg Ile Lys Ser Ala Lys Asp Leu Arg Ile Pro Val Trp Asn Asn Ile
    290                 295                 300
Lys Leu Asn Ser Gly Lys Ile Lys Trp Tyr Ala Pro Asn Val Lys Leu
305                 310                 315                 320
Ala Trp Tyr Asn Tyr Arg Arg Gly Tyr Leu Glu Leu Trp Tyr Pro Asn
                325                 330                 335
Asp Gly Trp Tyr Tyr Thr Ala Glu Tyr Phe Leu Lys
            340                 345


<210>  16
<211>  1047
<212>  DNA
<213>  artificial sequence

<220>

<223>  CBD118L500:

<400>  16
attacacatg gcatggatga actatacaaa gagctcaaat ctttaggttt tgagtggggt      60
ggtgattggt ctggatttgt agacaatccg caccttcaat ttaattataa aggctatggg     120
actgatactt ttggaaaagg agctagtact agtaattcat ctaaaccgag cgcagacaca     180
aacacaaaca gtctaggatt agtagattat atgaatttaa ataaactaga ttcaagcttt     240
gcgaatcgca aaaaactagc gacaagttac ggaattaaaa attacagtgg aacagcaacg     300
cagaacacaa cattattagc gaagttaaaa gcaggaaaac cacacacacc agcaagcaaa     360
aacacatact acacagaaaa tccgcgaaaa gttaaaacac tagtacaatg tgatctatac     420
aaatcagtag actttacaac aaaaaaccaa acaggtggaa catttccgcc aggcacagtc     480
ttcacgattt cagggatggg gaaaacgaaa ggcggaacac ctcgcttgaa gacgaagagc     540
ggttactatc tcactgctaa cacgaagttt gttaaaaaga ttactggtaa aacagtagcc     600
gcaaaaaatc caaaccgcca ttctcaaaac actaatacaa attcaaatcg ttacgagggt     660
aaagtcattg atagcgcacc actgctaccg aaaatggact ttaaatcatc accattccgc     720
atgtataagg taggaactga gttcttagta tatgatcata atcaatattg gtacaagaca     780
tacattgatg acaaacttta ctacatgtat aaaagctttt gcgatgttgt agctaaaaaa     840
gacgcaaaag gtcgcatcaa agttcgaatt aaaagcgcga aagacttgcg tattccagtc     900
tggaataaca taaaattgaa ttctgggaaa attaaatggt atgcacccaa tgtaaaacta     960
gcgtggtaca actatcgaag aggatattta gagctatggt atccgaacga cggctggtat    1020
tacacagcag aatacttctt aaaataa                                        1047


<210>  17
<211>  316
<212>  PRT
<213>  artificial sequence

<220>
<223>  CBD500-P35:

<400>  17

Ile Thr His Gly Met Asp Glu Leu Tyr Lys Glu Leu Gln Asn Thr Asn
1               5                   10                  15
Thr Asn Ser Asn Arg Tyr Glu Gly Lys Val Ile Asp Ser Ala Pro Leu
            20                  25                  30
Leu Pro Lys Met Asp Phe Lys Ser Ser Pro Phe Arg Met Tyr Lys Val
        35                  40                  45
Gly Thr Glu Phe Leu Val Tyr Asp His Asn Gln Tyr Trp Tyr Lys Thr
    50                  55                  60
Tyr Ile Asp Asp Lys Leu Tyr Tyr Met Tyr Lys Ser Phe Cys Asp Val
65                  70                  75                  80
Val Ala Lys Lys Asp Ala Lys Gly Arg Ile Lys Val Arg Ile Lys Ser
                85                  90                  95
Ala Lys Asp Leu Arg Ile Pro Val Trp Asn Asn Ile Lys Leu Asn Ser
            100                 105                 110
Gly Lys Ile Lys Trp Tyr Ala Pro Asn Val Lys Leu Ala Trp Tyr Asn
        115                 120                 125
Tyr Arg Arg Gly Tyr Leu Glu Leu Trp Tyr Pro Asn Asp Gly Trp Tyr
    130                 135                 140
Tyr Thr Ala Glu Tyr Phe Leu Lys Gln Phe Pro His Phe Glu Ala Cys
145                 150                 155                 160
Asp Trp Tyr Arg Gly Glu Arg Lys Tyr Lys Val Asp Thr Ser Glu Trp
                165                 170                 175
Lys Lys Lys Glu Asn Ile Asn Ile Val Ile Lys Asp Val Gly Tyr Phe
            180                 185                 190
Gln Asp Lys Pro Gln Phe Leu Asn Ser Lys Ser Val Arg Gln Trp Lys
        195                 200                 205
His Gly Thr Lys Val Lys Leu Thr Lys His Asn Ser His Trp Tyr Thr
    210                 215                 220
Gly Val Val Lys Asp Gly Asn Lys Ser Val Arg Gly Tyr Ile Tyr His
225                 230                 235                 240

Ser Met Ala Lys Val Thr Ser Lys Asn Ser Asp Gly Ser Val Asn Ala
                245                 250                 255
Thr Ile Asn Ala His Ala Phe Cys Trp Asp Asn Lys Lys Leu Asn Gly
            260                 265                 270
Gly Asp Phe Ile Asn Leu Lys Arg Gly Phe Lys Gly Ile Thr His Pro
        275                 280                 285
Ala Ser Asp Gly Phe Tyr Pro Leu Tyr Phe Ala Ser Arg Lys Lys Thr
    290                 295                 300
Phe Tyr Ile Pro Arg Tyr Met Phe Asp Ile Lys Lys
305                 310                 315

<210> 18
<211> 951
<212> DNA
<213> artificial sequence

<220>
<223> CBD500-P35:

<400> 18
attacacatg gcatggatga actatacaaa gagctccaaa acactaatac aaattcaaat 60
cgttacgagg gtaaagtcat tgatagcgca ccactgctac cgaaaatgga ctttaaatca 120
tcaccattcc gcatgtataa ggtaggaact gagttcttag tatatgatca taatcaatat 180
tggtacaaga catacattga tgacaaactt tactacatgt ataaaagctt ttgcgatgtt 240
gtagctaaaa aagacgcaaa aggtcgcatc aaagttcgaa ttaaaagcgc gaaagacttg 300
cgtattccag tctggaataa cataaaattg aattctggga aaattaaatg gtatgcaccc 360
aatgtaaaac tagcgtggta caactatcga agaggatatt tagagctatg gtatccgaac 420
gacggctggt attacacagc agaatacttc ttaaaacaat tcccacattt tgaagcttgt 480
gactggtatc gcggggaacg caagtataaa gtggacacat ctgaatggaa aaagaaagag 540
aatatcaata tcgttattaa agatgttggt tacttccaag acaaacctca attcttaaac 600
tccaaatcgg ttcgtcagtg gaagcatggc acgaaagtga agcttactaa acataactca 660
cattggtaca ctggtgtggt caaggatggt aacaaatcag tcaggggata tatttatcat 720
tcgatggcta aggtcacaag caagaatagc gacggttcgg ttaacgcaac gattaacgcc 780
cacgcatttt gttgggacaa taaaaaactt aatggtggcg actttatcaa cttgaagcgt 840
ggttttaaag gtatcaccca tcccgctagt gacggtttct atccactgta tttcgcttct 900
aggaaaaaaa ctttctacat tccgcgttac atgtttgaca tcaagaaata a 951

<210> 19
<211> 316
<212> PRT
<213> artificial sequence

<220>
<223> CBDP35-500:

<400> 19
Ile Thr His Gly Met Asp Glu Leu Tyr Lys Glu Leu Pro His Phe Glu
1               5                   10                  15
Ala Cys Asp Trp Tyr Arg Gly Glu Arg Lys Tyr Lys Val Asp Thr Ser
            20                  25                  30
Glu Trp Lys Lys Lys Glu Asn Ile Asn Ile Val Ile Lys Asp Val Gly
        35                  40                  45
Tyr Phe Gln Asp Lys Pro Gln Phe Leu Asn Ser Lys Ser Val Arg Gln
    50                  55                  60
Trp Lys His Gly Thr Lys Val Lys Leu Thr Lys His Asn Ser His Trp
65                  70                  75                  80
Tyr Thr Gly Val Val Lys Asp Gly Asn Lys Ser Val Arg Gly Tyr Ile
                85                  90                  95
Tyr His Ser Met Ala Lys Val Thr Ser Lys Asn Ser Asp Gly Ser Val
            100                 105                 110
Asn Ala Thr Ile Asn Ala His Ala Phe Cys Trp Asp Asn Lys Lys Leu

115                 120                 125
Asn Gly Gly Asp Phe Ile Asn Leu Lys Arg Gly Phe Lys Gly Ile Thr
    130                 135                 140
His Pro Ala Ser Asp Gly Phe Tyr Pro Leu Tyr Phe Ala Ser Arg Lys
145                 150                 155                 160
Lys Thr Phe Tyr Ile Pro Arg Tyr Met Phe Asp Ile Lys Lys Gln Phe
                165                 170                 175
Gln Asn Thr Asn Thr Asn Ser Asn Arg Tyr Glu Gly Lys Val Ile Asp
            180                 185                 190
Ser Ala Pro Leu Leu Pro Lys Met Asp Phe Lys Ser Ser Pro Phe Arg
        195                 200                 205
Met Tyr Lys Val Gly Thr Glu Phe Leu Val Tyr Asp His Asn Gln Tyr
    210                 215                 220
Trp Tyr Lys Thr Tyr Ile Asp Asp Lys Leu Tyr Tyr Met Tyr Lys Ser
225                 230                 235                 240
Phe Cys Asp Val Val Ala Lys Lys Asp Ala Lys Gly Arg Ile Lys Val
                245                 250                 255
Arg Ile Lys Ser Ala Lys Asp Leu Arg Ile Pro Val Trp Asn Asn Ile
            260                 265                 270
Lys Leu Asn Ser Gly Lys Ile Lys Trp Tyr Ala Pro Asn Val Lys Leu
        275                 280                 285
Ala Trp Tyr Asn Tyr Arg Arg Gly Tyr Leu Glu Leu Trp Tyr Pro Asn
    290                 295                 300
Asp Gly Trp Tyr Tyr Thr Ala Glu Tyr Phe Leu Lys
305                 310                 315


<210>  20
<211>  951
<212>  DNA
<213>  artificial sequence

<220>
<223>  CBDP35-500:

<400>  20
attacacatg gcatggatga actatacaaa gagctcccac attttgaagc ttgtgactgg      60
tatcgcgggg aacgcaagta taaagtggac acatctgaat ggaaaaagaa agagaatatc     120
aatatcgtta ttaaagatgt tggttacttc caagacaaac ctcaattctt aaactccaaa     180
tcggttcgtc agtggaagca tggcacgaaa gtgaagctta ctaaacataa ctcacattgg     240
tacactggtg tggtcaagga tggtaacaaa tcagtcaggg gatatattta tcattcgatg     300
gctaaggtca caagcaagaa tagcgacggt tcggttaacg caacgattaa cgcccacgca     360
ttttgttggg acaataaaaa acttaatggt ggcgacttta tcaacttgaa gcgtggtttt     420
aaaggtatca cccatcccgc tagtgacggt ttctatccac tgtatttcgc ttctaggaaa     480
aaaactttct acattccgcg ttacatgttt gacatcaaga aacaattcca aaacactaat     540
acaaattcaa atcgttacga gggtaaagtc attgatagcg caccactgct accgaaaatg     600
gactttaaat catcaccatt ccgcatgtat aaggtaggaa ctgagttctt agtatatgat     660
cataatcaat attggtacaa gacatacatt gatgacaaac tttactacat gtataaaagc     720
ttttgcgatg ttgtagctaa aaaagacgca aaaggtcgca tcaaagttcg aattaaaagc     780
gcgaaagact tgcgtattcc agtctggaat aacataaaat tgaattctgg gaaaattaaa     840
tggtatgcac ccaatgtaaa actagcgtgg tacaactatc gaagaggata tttagagcta     900
tggtatccga acgacggctg gtattacaca gcagaatact tcttaaaata a              951


<210>  21
<211>  311
<212>  PRT
<213>  artificial sequence

<220>
<223>  CBD500-500:

<400>  21

Ile Thr His Gly Met Asp Glu Leu Tyr Lys Glu Leu Gln Asn Thr Asn
1               5                   10                  15
Thr Asn Ser Asn Arg Tyr Glu Gly Lys Val Ile Asp Ser Ala Pro Leu
            20                  25                  30
Leu Pro Lys Met Asp Phe Lys Ser Ser Pro Phe Arg Met Tyr Lys Val
        35                  40                  45
Gly Thr Glu Phe Leu Val Tyr Asp His Asn Gln Tyr Trp Tyr Lys Thr
    50                  55                  60
Tyr Ile Asp Asp Lys Leu Tyr Tyr Met Tyr Lys Ser Phe Cys Asp Val
65                  70                  75                  80
Val Ala Lys Lys Asp Ala Lys Gly Arg Ile Lys Val Arg Ile Lys Ser
                85                  90                  95
Ala Lys Asp Leu Arg Ile Pro Val Trp Asn Asn Ile Lys Leu Asn Ser
            100                 105                 110
Gly Lys Ile Lys Trp Tyr Ala Pro Asn Val Lys Leu Ala Trp Tyr Asn
        115                 120                 125
Tyr Arg Arg Gly Tyr Leu Glu Leu Trp Tyr Pro Asn Asp Gly Trp Tyr
    130                 135                 140
Tyr Thr Ala Glu Tyr Phe Leu Lys Glu Leu His Phe Glu Leu Cys Asp
145                 150                 155                 160
Ala Val Ser Gly Glu Lys Ile Pro Ala Ala Thr Gln Asn Thr Asn Thr
                165                 170                 175
Asn Ser Asn Arg Tyr Glu Gly Lys Val Ile Asp Ser Ala Pro Leu Leu
            180                 185                 190
Pro Lys Met Asp Phe Lys Ser Ser Pro Phe Arg Met Tyr Lys Val Gly
        195                 200                 205
Thr Glu Phe Leu Val Tyr Asp His Asn Gln Tyr Trp Tyr Lys Thr Tyr
    210                 215                 220
Ile Asp Asp Lys Leu Tyr Tyr Met Tyr Lys Ser Phe Cys Asp Val Val
225                 230                 235                 240
Ala Lys Lys Asp Ala Lys Gly Arg Ile Lys Val Arg Ile Lys Ser Ala
                245                 250                 255
Lys Asp Leu Arg Ile Pro Val Trp Asn Asn Ile Lys Leu Asn Ser Gly
            260                 265                 270
Lys Ile Lys Trp Tyr Ala Pro Asn Val Lys Leu Ala Trp Tyr Asn Tyr
        275                 280                 285
Arg Arg Gly Tyr Leu Glu Leu Trp Tyr Pro Asn Asp Gly Trp Tyr Tyr
    290                 295                 300
Thr Ala Glu Tyr Phe Leu Lys
305                 310

<210>  22
<211>  936
<212>  DNA
<213>  artificial sequence

<220>
<223>  CBD500-500:

<400>  22
attacacatg gcatggatga actatacaaa gagctccaaa acactaatac aaattcaaat      60
cgttacgagg gtaaagtcat tgatagcgca ccactgctac cgaaaatgga ctttaaatca     120
tcaccattcc gcatgtataa ggtaggaact gagttcttag tatatgatca taatcaatat     180
tggtacaaga catacattga tgacaaactt tactacatgt ataaaagctt ttgcgatgtt     240
gtagctaaaa aagacgcaaa aggtcgcatc aaagttcgaa ttaaaagcgc gaaagacttg     300
cgtattccag tctggaataa cataaaattg aattctggga aaattaaatg gtatgcaccc     360
aatgtaaaac tagcgtggta caactatcga agaggatatt tagagctatg gtatccgaac     420
gacggctggt attacacagc agaatacttc ttaaaagagc tccattttga actatgtgat     480
gctgtaagtg gtgagaaaat ccctgctgca acacaaaaca ctaatacaaa ttcaaatcgt     540
tacgagggta aagtcattga tagcgcacca ctgctaccga aaatggactt taaatcatca     600
ccattccgca tgtataaggt aggaactgag ttcttagtat atgatcataa tcaatattgg     660
tacaagacat acattgatga caaactttac tacatgtata aaagcttttg cgatgttgta     720

gctaaaaaag acgcaaaagg tcgcatcaaa gttcgaatta aaagcgcgaa agacttgcgt 780
attccagtct ggaataacat aaaattgaat tctgggaaaa ttaaatggta tgcacccaat 840
gtaaaactag cgtggtacaa ctatcgaaga ggatatttag agctatggta tccgaacgac 900
ggctggtatt acacagcaga atacttctta aaataa 936

<210> 23
<211> 450
<212> PRT
<213> artificial sequence

<220>
<223> EAD_CBD500-500:

<400> 23
Gly Ser Met Ala Leu Thr Glu Ala Trp Leu Ile Glu Lys Ala Asn Arg
1               5                   10                  15
Lys Leu Asn Ala Gly Gly Met Tyr Lys Ile Thr Ser Asp Lys Thr Arg
            20                  25                  30
Asn Val Ile Lys Lys Met Ala Lys Glu Gly Ile Tyr Leu Cys Val Ala
        35                  40                  45
Gln Gly Tyr Arg Ser Thr Ala Glu Gln Asn Ala Leu Tyr Ala Gln Gly
    50                  55                  60
Arg Thr Lys Pro Gly Ala Ile Val Thr Asn Ala Lys Gly Gly Gln Ser
65                  70                  75                  80
Asn His Asn Tyr Gly Val Ala Val Asp Leu Cys Leu Tyr Thr Asn Asp
                85                  90                  95
Gly Lys Asp Val Ile Trp Glu Ser Thr Thr Ser Arg Trp Lys Lys Val
            100                 105                 110
Val Ala Ala Met Lys Ala Glu Gly Phe Lys Trp Gly Gly Asp Trp Lys
        115                 120                 125
Ser Phe Lys Asp Tyr Pro His Phe Glu Leu Cys Asp Ala Val Ser Gly
    130                 135                 140
Glu Lys Ile Pro Ala Ala Thr Gln Asn Thr Asn Thr Asn Ser Asn Arg
145                 150                 155                 160
Tyr Glu Gly Lys Val Ile Asp Ser Ala Pro Leu Leu Pro Lys Met Asp
                165                 170                 175
Phe Lys Ser Ser Pro Phe Arg Met Tyr Lys Val Gly Thr Glu Phe Leu
            180                 185                 190
Val Tyr Asp His Asn Gln Tyr Trp Tyr Lys Thr Tyr Ile Asp Asp Lys
        195                 200                 205
Leu Tyr Tyr Met Tyr Lys Ser Phe Cys Asp Val Val Ala Lys Lys Asp
    210                 215                 220
Ala Lys Gly Arg Ile Lys Val Arg Ile Lys Ser Ala Lys Asp Leu Arg
225                 230                 235                 240
Ile Pro Val Trp Asn Asn Ile Lys Leu Asn Ser Gly Lys Ile Lys Trp
                245                 250                 255
Tyr Ala Pro Asn Val Lys Leu Ala Trp Tyr Asn Tyr Arg Arg Gly Tyr
            260                 265                 270
Leu Glu Leu Trp Tyr Pro Asn Asp Gly Trp Tyr Tyr Thr Ala Glu Tyr
        275                 280                 285
Phe Leu Lys Glu Leu His Phe Glu Leu Cys Asp Ala Val Ser Gly Glu
    290                 295                 300
Lys Ile Pro Ala Ala Thr Gln Asn Thr Asn Thr Asn Ser Asn Arg Tyr
305                 310                 315                 320
Glu Gly Lys Val Ile Asp Ser Ala Pro Leu Leu Pro Lys Met Asp Phe
                325                 330                 335
Lys Ser Ser Pro Phe Arg Met Tyr Lys Val Gly Thr Glu Phe Leu Val
            340                 345                 350
Tyr Asp His Asn Gln Tyr Trp Tyr Lys Thr Tyr Ile Asp Asp Lys Leu
        355                 360                 365
Tyr Tyr Met Tyr Lys Ser Phe Cys Asp Val Val Ala Lys Lys Asp Ala
    370                 375                 380

Lys Gly Arg Ile Lys Val Arg Ile Lys Ser Ala Lys Asp Leu Arg Ile
385                 390                 395                 400
Pro Val Trp Asn Asn Ile Lys Leu Asn Ser Gly Lys Ile Lys Trp Tyr
                405                 410                 415
Ala Pro Asn Val Lys Leu Ala Trp Tyr Asn Tyr Arg Arg Gly Tyr Leu
            420                 425                 430
Glu Leu Trp Tyr Pro Asn Asp Gly Trp Tyr Tyr Thr Ala Glu Tyr Phe
        435                 440                 445
Leu Lys
    450

<210>  24
<211>  1353
<212>  DNA
<213>  artificial sequence

<220>
<223>  EAD_CBD500-500:

<400>  24
ggatccatgg cattaacaga ggcatggcta attgaaaaag caaatcgcaa attgaatgct      60
gggggaatgt ataaaattac atcggataaa acacgaaatg taattaaaaa aatggcaaaa     120
gaaggtattt atctttgtgt tgcgcaaggt taccgctcaa cagcggaaca aaatgcgcta     180
tatgcacaag ggagaaccaa acctggagca attgttacta atgccaaggg cgggcaatct     240
aatcacaact acggggtagc tgttgacttg tgcttgtata caaatgacgg aaaagatgtt     300
atttgggagt caacaacttc ccggtggaaa aaggttgttg ctgctatgaa agcagaaggg     360
tttaaatggg gcggagactg gaaaagtttt aaagactatc cgcattttga actatgtgat     420
gctgtaagtg gtgagaaaat ccctgctgca acacaaaaca ctaatacaaa ttcaaatcgt     480
tacgagggta aagtcattga tagcgcacca ctgctaccga aaatggactt taaatcatca     540
ccattccgca tgtataaggt aggaactgag ttcttagtat atgatcataa tcaatattgg     600
tacaagacat acattgatga caaactttac tacatgtata aaagcttttg cgatgttgta     660
gctaaaaaag acgcaaaagg tcgcatcaaa gttcgaatta aaagcgcgaa agacttgcgt     720
attccagtct ggaataacat aaaattgaat tctgggaaaa ttaaatggta tgcacccaat     780
gtaaaactag cgtggtacaa ctatcgaaga ggatatttag agctatggta tccgaacgac     840
ggctggtatt acacagcaga atacttctta aaagagctcc attttgaact atgtgatgct     900
gtaagtggtg agaaaatccc tgctgcaaca caaaacacta atacaaattc aaatcgttac     960
gagggtaaag tcattgatag cgcaccactg ctaccgaaaa tggactttaa atcatcacca    1020
ttccgcatgt ataaggtagg aactgagttc ttagtatatg atcataatca atattggtac    1080
aagacataca ttgatgacaa actttactac atgtataaaa gcttttgcga tgttgtagct    1140
aaaaaagacg caaaaggtcg catcaaagtt cgaattaaaa gcgcgaaaga cttgcgtatt    1200
ccagtctgga ataacataaa attgaattct gggaaaatta aatggtatgc acccaatgta    1260
aaactagcgt ggtacaacta tcgaagagga tatttagagc tatggtatcc gaacgacggc    1320
tggtattaca cagcagaata cttcttaaaa taa                                 1353

<210>  25
<211>  317
<212>  PRT
<213>  artificial sequence

<220>
<223>  Aminosäuresequenz von Fab25VL

<400>  25
Met Ala Tyr Lys Val Glu Arg Arg Ile Arg Pro Gly Leu Pro Gln Val
1               5                   10                  15
Gly Tyr Ala Pro Tyr Gly Gln Val His Ala His Ser Thr Gly Asn Pro
            20                  25                  30
Arg Ser Thr Ala Gln Asn Glu Ala Asp Tyr Phe Gln Thr Lys Asp Ile
        35                  40                  45
Thr Thr Gly Phe Tyr Thr His Leu Val Gly Asn Gly Arg Val Ile Gln
    50                  55                  60

Leu Ala Glu Val Asn Arg Gly Ala Trp Asp Val Gly Gly Gly Trp Asn
65                  70                  75                  80
Gln Trp Gly Tyr Ala Ser Val Glu Leu Ile Glu Ser His Ser Asn Arg
                85                  90                  95
Asp Glu Phe Met Arg Asp Tyr Lys Ile Tyr Cys Glu Leu Leu His Asp
            100                 105                 110
Leu Ala Lys Gln Ala Gly Leu Pro Thr Thr Val Asp Gln Gly Asn Thr
        115                 120                 125
Gly Ile Ile Thr His Asn Tyr Ala Thr His Asn Gln Pro Asn Asn Gly
    130                 135                 140
Ser Asp His Val Asp Pro Ile Pro Tyr Leu Ala Lys Trp Gly Ile Asn
145                 150                 155                 160
Leu Ala Gln Phe Arg Ser Asp Val Ala Asn Ala Lys Ser Asn Ser Lys
                165                 170                 175
Pro Val Thr Pro Ser Lys Pro Val Ser His Asp Lys Ala Ile Ala Lys
            180                 185                 190
Ser Pro Ala Lys Thr Val Asn Gly Tyr Thr Gly Lys Met Asp Lys Phe
        195                 200                 205
Asn Val Gln Gly Asn Lys Phe Arg Val Ala Gly Trp Met Leu Pro Thr
    210                 215                 220
Ala Gly Gly Gln Pro Tyr Asn Tyr Gly Tyr Val Phe Leu Leu Asp Ala
225                 230                 235                 240
Lys Thr Gly Lys Glu Ile Ala Arg Gln Leu Ala Gly Ala Val Ser Arg
                245                 250                 255
Pro Asp Val Thr Lys Ala Tyr Gly Val Lys Gly Gly Thr Asn Tyr Gly
            260                 265                 270
Leu Asp Val Thr Phe Asp Val Lys Lys Leu Lys Gly Lys Lys Phe Tyr
        275                 280                 285
Ala Met Phe Arg Arg Thr Asn Asp Lys Ala Gly Asn Thr Ala Gly Gly
    290                 295                 300
His Lys Asp Ile Gly Phe Asn Glu Phe Tyr Phe Thr Leu
305                 310                 315

<210>  26
<211>  954
<212>  DNA
<213>  artificial sequence

<220>
<223>  Nukleotidsequenz von Fab25VL

<400>  26
atggcttata aagtagaaag acgaattaga ccagggttgc cccaagttgg gtatgctcca      60
tatggacaag tacacgcaca cagtacaggt aacccacgta gtacagcaca aaatgaggca     120
gattattttc agactaaaga cattaccact ggtttttaca ctcatcttgt aggtaatgga     180
cgagtaatcc aactagctga agttaatcgt ggcgcatggg atgttggtgg aggttggaat     240
caatggggct atgcatcagt tgaattaatt gaatcccact ctaatagaga cgagtttatg     300
cgtgattata agatttattg tgagttactg catgacttag caaaacaagc aggcttacct     360
accactgttg accaaggtaa cactggtatc atcactcata actatgcaac acataatcaa     420
ccaaacaatg gttctgacca tgttgaccct attccatatc ttgctaaatg gggtatcaat     480
ttagctcagt ttagaagtga tgttgctaac gctaagagta acagcaagcc tgtgacacca     540
agcaaaccgg tgtcccatga taaagctatc gctaaaagtc ctgctaaaac agttaatgga     600
tatactggta aaatggataa gttcaatgtt caaggtaata agtttcgtgt ggcaggatgg     660
atgttaccta ccgcaggcgg tcaaccttac aactatggtt acgtattttt acttgatgct     720
aaaacaggta aagaaattgc acgtcaactt gcaggtgcgg tgtctcgtcc tgatgtaact     780
aaagcatatg gcgtaaaagg tggcactaac tatggtctag atgttacctt tgatgttaaa     840
aaactaaaag gtaaaaaatt ctatgcaatg tttagacgta ccaatgataa agcaggtaat     900
actgctggtg gacataaaga cattgggttc aatgaattct acttcacgct ataa           954

<210>  27
<211>  365
<212>  PRT

<213>  artificial sequence

<220>
<223>  Aminosäuresequenz von Fab20VL

<400>  27
Met Lys Leu Lys Gly Ile Leu Phe Gly Ala Leu Ala Thr Ile Gly Leu
1               5                   10                  15
Phe Ala Gly Met Gln Thr Ala Asn Ala Tyr Glu Val Asn Asn Glu Phe
            20                  25                  30
Asn Leu Ser Pro Trp Glu Gly Ser Gly Gln Val Ala Val Pro Asn Lys
        35                  40                  45
Ile Val Leu His Glu Thr Ala Asn Glu Arg Ala Thr Gly Arg Asn Glu
    50                  55                  60
Ala Thr Tyr Met Lys Asn Asn Trp Phe Asn Ala His Thr Thr Ala Ile
65                  70                  75                  80
Ile Gly Asp Gly Gly Ile Val Tyr Lys Ile Ala Pro Glu Gly Asn Ile
                85                  90                  95
Ser Trp Gly Ala Gly Asn Ala Asn Pro Tyr Ala Pro Ile Gln Ile Glu
            100                 105                 110
Leu Gln His Thr His Asp Lys Glu Leu Phe Lys Lys Asn Tyr Lys Ala
        115                 120                 125
Tyr Ile Asp Tyr Thr Arg Asp Met Gly Lys Lys Phe Gly Ile Pro Met
    130                 135                 140
Thr Leu Asp Gln Gly Ser Ser Val Trp Glu Lys Gly Val Ile Ser His
145                 150                 155                 160
Lys Trp Val Ser Asp Tyr Val Trp Gly Asp His Thr Asp Pro Tyr Gly
                165                 170                 175
Tyr Leu Ala Glu Met Gly Ile Ser Lys Ala Gln Leu Thr Lys Asp Leu
            180                 185                 190
Ala Asn Gly Val Ser Gly Glu Ser Val Lys Pro Thr Pro Ser Lys Pro
        195                 200                 205
Lys Thr Phe Lys Lys Gly Gln Asn Val Tyr Ile Tyr Asn Gly His Lys
    210                 215                 220
Ser His Asn Gly Pro Val Val Pro Phe Val Ala Gly Ser Ser Leu Trp
225                 230                 235                 240
Thr Gln Val Gly Thr Ile Thr Glu Val Lys Gln Gly Ser Val Asn Pro
                245                 250                 255
Tyr Lys Ile Glu Asn Ser Gly Lys Phe Val Thr Tyr Ala Asn Ala Gly
            260                 265                 270
Asp Leu Glu Asp Leu Asn Thr Lys Phe Pro Pro Lys Pro Ser Lys Pro
        275                 280                 285
Val Ser Gln Phe Thr Ile Gly Val Asp Ala Ile Val Leu Arg Ser Gly
    290                 295                 300
Arg Pro Ser Val Tyr Ala Pro Val Tyr Gly Thr Trp Lys Gln Gly Ala
305                 310                 315                 320
Val Phe Lys Tyr Asp Glu Ile Thr Val Gly Asp Gly Tyr Val Trp Ile
                325                 330                 335
Gly Gly Thr Asp Thr Asn Gly Thr Arg Ile Tyr Leu Pro Ile Gly Pro
            340                 345                 350
Asn Asp Gly Asp Pro Asn Asn Thr Trp Gly Thr Leu Val
        355                 360                 365


<210>  28
<211>  1098
<212>  DNA
<213>  artificial sequence

<220>
<223>  Nukleotidsequenz von Fab20VL

<400>  28

atgaagttaa aaggtatttt atttggtgca ttagcaacca ttggtttgtt tgctggaatg 60
caaacagcta atgcatatga agttaataac gagttcaatt taagtccttg ggaaggttca 120
ggacaggttg cagtacctaa taagattgtc ttacatgaaa ctgctaatga acgtgccaca 180
ggacggaatg aagcaacgta catgaaaaac aactggttta atgctcatac aacagctatc 240
attggtgatg gtggtattgt ttataagatt gcaccagaag gtaacatttc atggggtgct 300
ggtaatgcaa acccctacgc acctattcaa attgagttac aacatacaca tgataaagag 360
ttgttcaaaa agaactataa agcgtacatt gactatacaa gagacatggg taaaaagttt 420
ggtattccta tgacgcttga ccaaggttct tctgtttggg aaaaaggtgt tatctctcat 480
aaatgggtat cagattatgt atggggtgac cacacagacc catatggtta cttagcagaa 540
atgggaatca gtaaagcaca acttactaaa gacttagcca atggggtatc tggtgaatca 600
gtaaaaccaa caccaagcaa accaaagaca ttcaaaaaag gtcaaaatgt ttacatttat 660
aacggtcaca agtcacacaa tggacctgta gtaccatttg tagctggctc aagtctttgg 720
acccaagttg gtacaattac agaagtgaaa caaggttcag tcaatccgta taagattgaa 780
aacagtggta aatttgtaac atatgctaac gctggcgact tagaggacct taacactaag 840
ttcccaccaa aaccaagcaa accagttagt cagtttacaa ttggtgttga cgctattgtt 900
ttacgtagtg gacgacctag cgtatatgca ccagtatacg gaacatggaa acaaggtgca 960
gtattcaagt atgatgaaat cacagttggt gatggctatg tatggattgg tggaacagac 1020
actaatggta cacgtattta cttaccaatt ggaccaaatg acggagaccc taacaacacg 1080
tggggtacat tagtataa 1098

<210> 29
<211> 346
<212> PRT
<213> artificial sequence

<220>
<223> Aminosäuresequenz von Fab20K

<400> 29
Met Gln Thr Ala Asn Ala Tyr Glu Val Asn Asn Glu Phe Asn Leu Ser
1               5                   10                  15
Pro Trp Glu Gly Ser Gly Gln Val Ala Val Pro Asn Lys Ile Val Leu
            20                  25                  30
His Glu Thr Ala Asn Glu Arg Ala Thr Gly Arg Asn Glu Ala Thr Tyr
        35                  40                  45
Met Lys Asn Asn Trp Phe Asn Ala His Thr Thr Ala Ile Ile Gly Asp
    50                  55                  60
Gly Gly Ile Val Tyr Lys Ile Ala Pro Glu Gly Asn Ile Ser Trp Gly
65                  70                  75                  80
Ala Gly Asn Ala Asn Pro Tyr Ala Pro Ile Gln Ile Glu Leu Gln His
                85                  90                  95
Thr His Asp Lys Glu Leu Phe Lys Lys Asn Tyr Lys Ala Tyr Ile Asp
            100                 105                 110
Tyr Thr Arg Asp Met Gly Lys Lys Phe Gly Ile Pro Met Thr Leu Asp
        115                 120                 125
Gln Gly Ser Ser Val Trp Glu Lys Gly Val Ile Ser His Lys Trp Val
    130                 135                 140
Ser Asp Tyr Val Trp Gly Asp His Thr Asp Pro Tyr Gly Tyr Leu Ala
145                 150                 155                 160
Glu Met Gly Ile Ser Lys Ala Gln Leu Thr Lys Asp Leu Ala Asn Gly
                165                 170                 175
Val Ser Gly Glu Ser Val Lys Pro Thr Pro Ser Lys Pro Lys Thr Phe
            180                 185                 190
Lys Lys Gly Gln Asn Val Tyr Ile Tyr Asn Gly His Lys Ser His Asn
        195                 200                 205
Gly Pro Val Val Pro Phe Val Ala Gly Ser Ser Leu Trp Thr Gln Val
    210                 215                 220
Gly Thr Ile Thr Glu Val Lys Gln Gly Ser Val Asn Pro Tyr Lys Ile
225                 230                 235                 240
Glu Asn Ser Gly Lys Phe Val Thr Tyr Ala Asn Ala Gly Asp Leu Glu
                245                 250                 255
Asp Leu Asn Thr Lys Phe Pro Pro Lys Pro Ser Lys Pro Val Ser Gln

260                 265                 270
Phe Thr Ile Gly Val Asp Ala Ile Val Leu Arg Ser Gly Arg Pro Ser
        275                 280                 285
Val Tyr Ala Pro Val Tyr Gly Thr Trp Lys Gln Gly Ala Val Phe Lys
    290                 295                 300
Tyr Asp Glu Ile Thr Val Gly Asp Gly Tyr Val Trp Ile Gly Gly Thr
305                 310                 315                 320
Asp Thr Asn Gly Thr Arg Ile Tyr Leu Pro Ile Gly Pro Asn Asp Gly
                325                 330                 335
Asp Pro Asn Asn Thr Trp Gly Thr Leu Val
            340                 345


<210>  30
<211>  1041
<212>  DNA
<213>  artificial sequence

<220>
<223>  Nukleotidsequenz von Fab20K

<400>  30
atgcaaacag ctaatgcata tgaagttaat aacgagttca atttaagtcc ttgggaaggt      60
tcaggacagg ttgcagtacc taataagatt gtcttacatg aaactgctaa tgaacgtgcc     120
acaggacgga atgaagcaac gtacatgaaa aacaactggt ttaatgctca tacaacagct     180
atcattggtg atggtggtat tgtttataag attgcaccag aaggtaacat ttcatggggt     240
gctggtaatg caaaccccta cgcacctatt caaattgagt tacaacatac acatgataaa     300
gagttgttca aaaagaacta taaagcgtac attgactata caagagacat gggtaaaaag     360
tttggtattc ctatgacgct tgaccaaggt tcttctgttt gggaaaaagg tgttatctct     420
cataaatggg tatcagatta tgtatggggt gaccacacag acccatatgg ttacttagca     480
gaaatgggaa tcagtaaagc acaacttact aaagacttag ccaatggggt atctggtgaa     540
tcagtaaaac caacaccaag caaaccaaag acattcaaaa aaggtcaaaa tgtttacatt     600
tataacggtc acaagtcaca caatggacct gtagtaccat ttgtagctgg ctcaagtctt     660
tggacccaag ttggtacaat tacagaagtg aaacaaggtt cagtcaatcc gtataagatt     720
gaaaacagtg gtaaatttgt aacatatgct aacgctggcg acttagagga ccttaacact     780
aagttcccac caaaaccaag caaaccagtt agtcagttta caattggtgt tgacgctatt     840
gttttacgta gtggacgacc tagcgtatat gcaccagtat acggaacatg gaaacaaggt     900
gcagtattca agtatgatga aatcacagtt ggtgatggct atgtatggat tggtggaaca     960
gacactaatg gtacacgtat ttacttacca attggaccaa atgacggaga ccctaacaac    1020
acgtggggta cattagtata a                                              1041


<210>  31
<211>  485
<212>  PRT
<213>  artificial sequence

<220>
<223>  Aminosäuresequenz von EAD_Fab25_CBD25_CBD20

<400>  31
Met Ala Tyr Lys Val Glu Arg Arg Ile Arg Pro Gly Leu Pro Gln Val
1               5                   10                  15
Gly Tyr Ala Pro Tyr Gly Gln Val His Ala His Ser Thr Gly Asn Pro
            20                  25                  30
Arg Ser Thr Ala Gln Asn Glu Ala Asp Tyr Phe Gln Thr Lys Asp Ile
        35                  40                  45
Thr Thr Gly Phe Tyr Thr His Leu Val Gly Asn Gly Arg Val Ile Gln
    50                  55                  60
Leu Ala Glu Val Asn Arg Gly Ala Trp Asp Val Gly Gly Gly Trp Asn
65                  70                  75                  80
Gln Trp Gly Tyr Ala Ser Val Glu Leu Ile Glu Ser His Ser Asn Arg
                85                  90                  95

Asp Glu Phe Met Arg Asp Tyr Lys Ile Tyr Cys Glu Leu Leu His Asp
            100                 105                 110
Leu Ala Lys Gln Ala Gly Leu Pro Thr Thr Val Asp Gln Gly Asn Thr
        115                 120                 125
Gly Ile Ile Thr His Asn Tyr Ala Thr His Asn Gln Pro Asn Asn Gly
    130                 135                 140
Ser Asp His Val Asp Pro Ile Pro Tyr Leu Ala Lys Trp Gly Ile Asn
145                 150                 155                 160
Leu Ala Gln Phe Arg Ser Asp Val Ala Asn Ala Lys Ser Asn Ser Lys
                165                 170                 175
Pro Val Thr Pro Ser Lys Pro Val Ser His Asp Lys Ala Ile Ala Lys
            180                 185                 190
Ser Pro Ala Lys Thr Val Asn Gly Tyr Thr Gly Lys Met Asp Lys Phe
        195                 200                 205
Asn Val Gln Gly Asn Lys Phe Arg Val Ala Gly Trp Met Leu Pro Thr
    210                 215                 220
Ala Gly Gly Gln Pro Tyr Asn Tyr Gly Tyr Val Phe Leu Leu Asp Ala
225                 230                 235                 240
Lys Thr Gly Lys Glu Ile Ala Arg Gln Leu Ala Gly Ala Val Ser Arg
                245                 250                 255
Pro Asp Val Thr Lys Ala Tyr Gly Val Lys Gly Gly Thr Asn Tyr Gly
            260                 265                 270
Leu Asp Val Thr Phe Asp Val Lys Lys Leu Lys Gly Lys Lys Phe Tyr
        275                 280                 285
Ala Met Phe Arg Arg Thr Asn Asp Lys Ala Gly Asn Thr Ala Gly Gly
    290                 295                 300
His Lys Asp Ile Gly Phe Asn Glu Phe Tyr Phe Thr Leu Glu Leu Met
305                 310                 315                 320
Val Lys Pro Thr Pro Ser Lys Pro Lys Thr Phe Lys Lys Gly Gln Asn
                325                 330                 335
Val Tyr Ile Tyr Asn Gly His Lys Ser His Asn Gly Pro Val Val Pro
            340                 345                 350
Phe Val Ala Gly Ser Ser Leu Trp Thr Gln Val Gly Thr Ile Thr Glu
        355                 360                 365
Val Lys Gln Gly Ser Val Asn Pro Tyr Lys Ile Glu Asn Ser Gly Lys
    370                 375                 380
Phe Val Thr Tyr Ala Asn Ala Gly Asp Leu Glu Asp Leu Asn Thr Lys
385                 390                 395                 400
Phe Pro Pro Lys Pro Ser Lys Pro Val Ser Gln Phe Thr Ile Gly Val
                405                 410                 415
Asp Ala Ile Val Leu Arg Ser Gly Arg Pro Ser Val Tyr Ala Pro Val
            420                 425                 430
Tyr Gly Thr Trp Lys Gln Gly Ala Val Phe Lys Tyr Asp Glu Ile Thr
        435                 440                 445
Val Gly Asp Gly Tyr Val Trp Ile Gly Gly Thr Asp Thr Asn Gly Thr
    450                 455                 460
Arg Ile Tyr Leu Pro Ile Gly Pro Asn Asp Gly Asp Pro Asn Asn Thr
465                 470                 475                 480
Trp Gly Thr Leu Val
                485


<210>  32
<211>  1458
<212>  DNA
<213>  artificial sequence

<220>
<223>  Nukleotidsequenz von EAD_Fab25_CBD25_CBD20

<400>  32
atggcttata aagtagaaag acgaattaga ccagggttgc cccaagttgg gtatgctcca      60
tatggacaag tacacgcaca cagtacaggt aacccacgta gtacagcaca aaatgaggca     120

gattattttc agactaaaga cattaccact ggtttttaca ctcatcttgt aggtaatgga 180
cgagtaatcc aactagctga agttaatcgt ggcgcatggg atgttggtgg aggttggaat 240
caatggggct atgcatcagt tgaattaatt gaatcccact ctaatagaga cgagtttatg 300
cgtgattata agatttattg tgagttactg catgacttag caaaacaagc aggcttacct 360
accactgttg accaaggtaa cactggtatc atcactcata actatgcaac acataatcaa 420
ccaaacaatg gttctgacca tgttgaccct attccatatc ttgctaaatg gggtatcaat 480
ttagctcagt ttagaagtga tgttgctaac gctaagagta acagcaagcc tgtgacacca 540
agcaaaccgg tgtcccatga taaagctatc gctaaaagtc ctgctaaaac agttaatgga 600
tatactggta aaatggataa gttcaatgtt caaggtaata agtttcgtgt ggcaggatgg 660
atgttaccta ccgcaggcgg tcaaccttac aactatggtt acgtattttt acttgatgct 720
aaaacaggta aagaaattgc acgtcaactt gcaggtgcgg tgtctcgtcc tgatgtaact 780
aaagcatatg gcgtaaaagg tggcactaac tatggtctag atgttacctt tgatgttaaa 840
aaactaaaag gtaaaaaatt ctatgcaatg tttagacgta ccaatgataa agcaggtaat 900
actgctggtg gacataaaga cattgggttc aatgaattct acttcacgct agagctcatg 960
gtaaaaccaa caccaagcaa accaaagaca ttcaaaaaag gtcaaaatgt ttacatttat 1020
aacggtcaca agtcacacaa tggacctgta gtaccatttg tagctggctc aagtctttgg 1080
acccaagttg gtacaattac agaagtgaaa caaggttcag tcaatccgta taagattgaa 1140
aacagtggta aatttgtaac atatgctaac gctggcgact tagaggacct taacactaag 1200
ttcccaccaa aaccaagcaa accagttagt cagtttacaa ttggtgttga cgctattgtt 1260
ttacgtagtg gacgacctag cgtatatgca ccagtatacg gaacatggaa acaaggtgca 1320
gtattcaagt atgatgaaat cacagttggt gatggctatg tatggattgg tggaacagac 1380
actaatggta cacgtattta cttaccaatt ggaccaaatg acggagaccc taacaacacg 1440
tggggtacat tagtataa 1458

<210> 33
<211> 603
<212> PRT
<213> artificial sequence

<220>
<223> HGFP_CBD118-500:

<400> 33
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240

Ile Thr His Gly Met Asp Glu Leu Tyr Lys Glu Leu Asp Lys Gly Lys
                245                 250                 255
Lys Phe Val Ala Lys Ala Lys Ser Leu Gly Phe Glu Trp Gly Gly Asp
            260                 265                 270
Trp Ser Gly Phe Val Asp Asn Pro His Leu Gln Phe Asn Tyr Lys Gly
        275                 280                 285
Tyr Gly Thr Asp Thr Phe Gly Lys Gly Ala Ser Thr Ser Asn Ser Ser
    290                 295                 300
Lys Pro Ser Ala Asp Thr Asn Thr Asn Ser Leu Gly Leu Val Asp Tyr
305                 310                 315                 320
Met Asn Leu Asn Lys Leu Asp Ser Ser Phe Ala Asn Arg Lys Lys Leu
                325                 330                 335
Ala Thr Ser Tyr Gly Ile Lys Asn Tyr Ser Gly Thr Ala Thr Gln Asn
            340                 345                 350
Thr Thr Leu Leu Ala Lys Leu Lys Ala Gly Lys Pro His Thr Pro Ala
        355                 360                 365
Ser Lys Asn Thr Tyr Tyr Thr Glu Asn Pro Arg Lys Val Lys Thr Leu
    370                 375                 380
Val Gln Cys Asp Leu Tyr Lys Ser Val Asp Phe Thr Thr Lys Asn Gln
385                 390                 395                 400
Thr Gly Gly Thr Phe Pro Pro Gly Thr Val Phe Thr Ile Ser Gly Met
                405                 410                 415
Gly Lys Thr Lys Gly Gly Thr Pro Arg Leu Lys Thr Lys Ser Gly Tyr
            420                 425                 430
Tyr Leu Thr Ala Asn Thr Lys Phe Val Lys Lys Ile Glu Leu His Phe
        435                 440                 445
Glu Leu Cys Asp Ala Val Ser Gly Glu Lys Ile Pro Ala Ala Thr Gln
    450                 455                 460
Asn Thr Asn Thr Asn Ser Asn Arg Tyr Glu Gly Lys Val Ile Asp Ser
465                 470                 475                 480
Ala Pro Leu Leu Pro Lys Met Asp Phe Lys Ser Ser Pro Phe Arg Met
                485                 490                 495
Tyr Lys Val Gly Thr Glu Phe Leu Val Tyr Asp His Asn Gln Tyr Trp
            500                 505                 510
Tyr Lys Thr Tyr Ile Asp Asp Lys Leu Tyr Tyr Met Tyr Lys Ser Phe
        515                 520                 525
Cys Asp Val Val Ala Lys Lys Asp Ala Lys Gly Arg Ile Lys Val Arg
    530                 535                 540
Ile Lys Ser Ala Lys Asp Leu Arg Ile Pro Val Trp Asn Asn Ile Lys
545                 550                 555                 560
Leu Asn Ser Gly Lys Ile Lys Trp Tyr Ala Pro Asn Val Lys Leu Ala
                565                 570                 575
Trp Tyr Asn Tyr Arg Arg Gly Tyr Leu Glu Leu Trp Tyr Pro Asn Asp
            580                 585                 590
Gly Trp Tyr Tyr Thr Ala Glu Tyr Phe Leu Lys
        595                 600


<210>  34
<211>  1812
<212>  DNA
<213>  artificial sequence

<220>
<223>  HGFP_CBD118-500:

<400>  34
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180
actactggaa aactacctgt tccatggcca acacttgtca ctactttcgc gtatggtctt     240
caatgctttg cgagataccc agatcatatg aaacggcatg actttttcaa gagtgccatg     300
cccgaaggtt atgtacagga aagaactata tttttcaaag atgacgggaa ctacaagaca     360

cgtgctgaag tcaagtttga aggtgatacc cttgttaata gaatcgagtt aaaaggtatt 420
gattttaaag aagatggaaa cattcttgga cacaaattgg aatacaacta taactcacac 480
aatgtataca tcatggcaga caaacaaaag aatggaatca aagttaactt caaaattaga 540
cacaacattg aagatggaag cgttcaacta gcagaccatt atcaacaaaa tactccaatt 600
ggcgatggcc ctgtcctttt accagacaac cattacctgt ccacacaatc tgccctttcg 660
aaagatccca acgaaaagag agaccacatg gtccttcttg agtttgtaac agctgctggg 720
attacacatg gcatggatga actatacaaa gagctcgaca aaggcaagaa atttgtggca 780
aaggcaaaat ctttaggttt tgagtggggt ggtgattggt ctggatttgt agacaatccg 840
caccttcaat ttaattataa aggctatggg actgatactt ttggaaaagg agctagtact 900
agtaattcat ctaaaccgag cgcagacaca aacacaaaca gtctaggatt agtagattat 960
atgaatttaa ataaactaga ttcaagcttt gcgaatcgca aaaaactagc gacaagttac 1020
ggaattaaaa attacagtgg aacagcaacg cagaacacaa cattattagc gaagttaaaa 1080
gcaggaaaac cacacacacc agcaagcaaa aacacatact acacagaaaa tccgcgaaaa 1140
gttaaaacac tagtacaatg tgatctatac aaatcagtag actttacaac aaaaaaccaa 1200
acaggtggaa catttccgcc aggcacagtc ttcacgattt cagggatggg gaaaacgaaa 1260
ggcggaacac ctcgcttgaa gacgaagagc ggttactatc tcactgctaa cacgaagttt 1320
gttaaaaaga ttgaattgca ttttgaacta tgtgatgctg taagtggtga gaaaatccct 1380
gctgcaacac aaaacactaa tacaaattca aatcgttacg agggtaaagt cattgatagc 1440
gcaccactgc taccgaaaat ggactttaaa tcatcaccat tccgcatgta taaggtagga 1500
actgagttct tagtatatga tcataatcaa tattggtaca agacatacat tgatgacaaa 1560
ctttactaca tgtataaaag cttttgcgat gttgtagcta aaaaagacgc aaaaggtcgc 1620
atcaaagttc gaattaaaag cgcgaaagac ttgcgtattc cagtctggaa taacataaaa 1680
ttgaattctg ggaaaattaa atggtatgca cccaatgtaa aactagcgtg gtacaactat 1740
cgaagaggat atttagagct atggtatccg aacgacggct ggtattacac agcagaatac 1800
ttcttaaaat aa 1812

<210> 35
<211> 603
<212> PRT
<213> artificial sequence

<220>
<223> HGFP_CBD500-118:

<400> 35
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn

210                 215                 220
Glu Lys Arg Asp His Met Val Leu Leu Glu Phe Val Thr Ala Ala Gly
225                 230                 235                 240
Ile Thr His Gly Met Asp Glu Leu Tyr Lys Glu Leu His Phe Glu Leu
                245                 250                 255
Cys Asp Ala Val Ser Gly Glu Lys Ile Pro Ala Ala Thr Gln Asn Thr
            260                 265                 270
Asn Thr Asn Ser Asn Arg Tyr Glu Gly Lys Val Ile Asp Ser Ala Pro
        275                 280                 285
Leu Leu Pro Lys Met Asp Phe Lys Ser Ser Pro Phe Arg Met Tyr Lys
    290                 295                 300
Val Gly Thr Glu Phe Leu Val Tyr Asp His Asn Gln Tyr Trp Tyr Lys
305                 310                 315                 320
Thr Tyr Ile Asp Asp Lys Leu Tyr Tyr Met Tyr Lys Ser Phe Cys Asp
                325                 330                 335
Val Val Ala Lys Lys Asp Ala Lys Gly Arg Ile Lys Val Arg Ile Lys
            340                 345                 350
Ser Ala Lys Asp Leu Arg Ile Pro Val Trp Asn Asn Ile Lys Leu Asn
        355                 360                 365
Ser Gly Lys Ile Lys Trp Tyr Ala Pro Asn Val Lys Leu Ala Trp Tyr
    370                 375                 380
Asn Tyr Arg Arg Gly Tyr Leu Glu Leu Trp Tyr Pro Asn Asp Gly Trp
385                 390                 395                 400
Tyr Tyr Thr Ala Glu Tyr Phe Leu Lys Gln Phe Asp Lys Gly Lys Lys
                405                 410                 415
Phe Val Ala Lys Ala Lys Ser Leu Gly Phe Glu Trp Gly Gly Asp Trp
            420                 425                 430
Ser Gly Phe Val Asp Asn Pro His Leu Gln Phe Asn Tyr Lys Gly Tyr
        435                 440                 445
Gly Thr Asp Thr Phe Gly Lys Gly Ala Ser Thr Ser Asn Ser Ser Lys
    450                 455                 460
Pro Ser Ala Asp Thr Asn Thr Asn Ser Leu Gly Leu Val Asp Tyr Met
465                 470                 475                 480
Asn Leu Asn Lys Leu Asp Ser Ser Phe Ala Asn Arg Lys Lys Leu Ala
                485                 490                 495
Thr Ser Tyr Gly Ile Lys Asn Tyr Ser Gly Thr Ala Thr Gln Asn Thr
            500                 505                 510
Thr Leu Leu Ala Lys Leu Lys Ala Gly Lys Pro His Thr Pro Ala Ser
        515                 520                 525
Lys Asn Thr Tyr Tyr Thr Glu Asn Pro Arg Lys Val Lys Thr Leu Val
    530                 535                 540
Gln Cys Asp Leu Tyr Lys Ser Val Asp Phe Thr Thr Lys Asn Gln Thr
545                 550                 555                 560
Gly Gly Thr Phe Pro Pro Gly Thr Val Phe Thr Ile Ser Gly Met Gly
                565                 570                 575
Lys Thr Lys Gly Gly Thr Pro Arg Leu Lys Thr Lys Ser Gly Tyr Tyr
            580                 585                 590
Leu Thr Ala Asn Thr Lys Phe Val Lys Lys Ile
        595                 600

<210>  36
<211>  1812
<212>  DNA
<213>  artificial sequence

<220>
<223>  HGFP_CBD500-118:

<400>  36
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180

actactggaa aactacctgt tccatggcca acacttgtca ctactttcgc gtatggtctt 240
caatgctttg cgagataccc agatcatatg aaacggcatg actttttcaa gagtgccatg 300
cccgaaggtt atgtacagga aagaactata tttttcaaag atgacgggaa ctacaagaca 360
cgtgctgaag tcaagtttga aggtgatacc cttgttaata gaatcgagtt aaaaggtatt 420
gattttaaag aagatggaaa cattcttgga cacaaattgg aatacaacta taactcacac 480
aatgtataca tcatggcaga caaacaaaag aatggaatca aagttaactt caaaattaga 540
cacaacattg aagatggaag cgttcaacta gcagaccatt atcaacaaaa tactccaatt 600
ggcgatggcc ctgtcctttt accagacaac cattacctgt ccacacaatc tgccctttcg 660
aaagatccca acgaaaagag agaccacatg gtccttcttg agtttgtaac agctgctggg 720
attacacatg gcatggatga actatacaaa gagctccatt ttgaactatg tgatgctgta 780
agtggtgaga aaatccctgc tgcaacacaa aacactaata caaattcaaa tcgttacgag 840
ggtaaagtca ttgatagcgc accactgcta ccgaaaatgg actttaaatc atcaccattc 900
cgcatgtata aggtaggaac tgagttctta gtatatgatc ataatcaata ttggtacaag 960
acatacattg atgacaaact ttactacatg tataaaagct tttgcgatgt tgtagctaaa 1020
aaagacgcaa aaggtcgcat caaagttcga attaaaagcg cgaaagactt gcgtattcca 1080
gtctggaata acataaaatt gaattctggg aaaattaaat ggtatgcacc caatgtaaaa 1140
ctagcgtggt acaactatcg aagaggatat ttagagctat ggtatccgaa cgacggctgg 1200
tattacacag cagaatactt cttaaaacaa ttcgacaaag gcaagaaatt tgtggcaaag 1260
gcaaaatctt taggttttga gtggggtggt gattggtctg gatttgtaga caatccgcac 1320
cttcaattta attataaagg ctatgggact gatacttttg gaaaaggagc tagtactagt 1380
aattcatcta aaccgagcgc agacacaaac acaaacagtc taggattagt agattatatg 1440
aatttaaata aactagattc aagctttgcg aatcgcaaaa aactagcgac aagttacgga 1500
attaaaaatt acagtggaac agcaacgcag aacacaacat tattagcgaa gttaaaagca 1560
ggaaaaccac acacaccagc aagcaaaaac acatactaca cagaaaatcc gcgaaaagtt 1620
aaaacactag tacaatgtga tctatacaaa tcagtagact ttacaacaaa aaaccaaaca 1680
ggtggaacat ttccgccagg cacagtcttc acgatttcag ggatggggaa aacgaaaggc 1740
ggaacacctc gcttgaagac gaagagcggt tactatctca ctgctaacac gaagtttgtt 1800
aaaaagattt aa 1812

<210> 37
<211> 588
<212> PRT
<213> artificial sequence

<220>
<223> HGFP_CBD118L500:

<400> 37
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190

His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Ile Thr His Gly Met Asp Glu Leu Tyr Lys Glu Leu Lys Ser Leu Gly
                245                 250                 255
Phe Glu Trp Gly Gly Asp Trp Ser Gly Phe Val Asp Asn Pro His Leu
            260                 265                 270
Gln Phe Asn Tyr Lys Gly Tyr Gly Thr Asp Thr Phe Gly Lys Gly Ala
        275                 280                 285
Ser Thr Ser Asn Ser Ser Lys Pro Ser Ala Asp Thr Asn Thr Asn Ser
    290                 295                 300
Leu Gly Leu Val Asp Tyr Met Asn Leu Asn Lys Leu Asp Ser Ser Phe
305                 310                 315                 320
Ala Asn Arg Lys Lys Leu Ala Thr Ser Tyr Gly Ile Lys Asn Tyr Ser
                325                 330                 335
Gly Thr Ala Thr Gln Asn Thr Thr Leu Leu Ala Lys Leu Lys Ala Gly
            340                 345                 350
Lys Pro His Thr Pro Ala Ser Lys Asn Thr Tyr Tyr Thr Glu Asn Pro
        355                 360                 365
Arg Lys Val Lys Thr Leu Val Gln Cys Asp Leu Tyr Lys Ser Val Asp
    370                 375                 380
Phe Thr Thr Lys Asn Gln Thr Gly Gly Thr Phe Pro Pro Gly Thr Val
385                 390                 395                 400
Phe Thr Ile Ser Gly Met Gly Lys Thr Lys Gly Gly Thr Pro Arg Leu
                405                 410                 415
Lys Thr Lys Ser Gly Tyr Tyr Leu Thr Ala Asn Thr Lys Phe Val Lys
            420                 425                 430
Lys Ile Thr Gly Lys Thr Val Ala Ala Lys Asn Pro Asn Arg His Ser
        435                 440                 445
Gln Asn Thr Asn Thr Asn Ser Asn Arg Tyr Glu Gly Lys Val Ile Asp
    450                 455                 460
Ser Ala Pro Leu Leu Pro Lys Met Asp Phe Lys Ser Ser Pro Phe Arg
465                 470                 475                 480
Met Tyr Lys Val Gly Thr Glu Phe Leu Val Tyr Asp His Asn Gln Tyr
                485                 490                 495
Trp Tyr Lys Thr Tyr Ile Asp Asp Lys Leu Tyr Tyr Met Tyr Lys Ser
            500                 505                 510
Phe Cys Asp Val Val Ala Lys Lys Asp Ala Lys Gly Arg Ile Lys Val
        515                 520                 525
Arg Ile Lys Ser Ala Lys Asp Leu Arg Ile Pro Val Trp Asn Asn Ile
    530                 535                 540
Lys Leu Asn Ser Gly Lys Ile Lys Trp Tyr Ala Pro Asn Val Lys Leu
545                 550                 555                 560
Ala Trp Tyr Asn Tyr Arg Arg Gly Tyr Leu Glu Leu Trp Tyr Pro Asn
                565                 570                 575
Asp Gly Trp Tyr Tyr Thr Ala Glu Tyr Phe Leu Lys
            580                 585

<210>  38
<211>  1767
<212>  DNA
<213>  artificial sequence

<220>
<223>  HGFP_CBD118L500:

<400>  38
atgagaggat cgcatcacca tcaccatcac ggatccatga gtaaaggaga agaacttttc      60
actggagttg tcccaattct tgttgaatta gatggtgatg ttaatgggca caaattttct     120

gtcagtggag agggtgaagg tgatgcaaca tacggaaaac ttacccttaa atttatttgc 180
actactggaa aactacctgt tccatggcca acacttgtca ctactttcgc gtatggtctt 240
caatgctttg cgagataccc agatcatatg aaacggcatg actttttcaa gagtgccatg 300
cccgaaggtt atgtacagga aagaactata tttttcaaag atgacgggaa ctacaagaca 360
cgtgctgaag tcaagtttga aggtgatacc cttgttaata gaatcgagtt aaaaggtatt 420
gattttaaag aagatggaaa cattcttgga cacaaattgg aatacaacta taactcacac 480
aatgtataca tcatggcaga caaacaaaag aatggaatca aagttaactt caaaattaga 540
cacaacattg aagatggaag cgttcaacta gcagaccatt atcaacaaaa tactccaatt 600
ggcgatggcc ctgtcctttt accagacaac cattacctgt ccacacaatc tgccctttcg 660
aaagatccca acgaaaagag agaccacatg gtccttcttg agtttgtaac agctgctggg 720
attacacatg gcatggatga actatacaaa gagctcaaat ctttaggttt tgagtggggt 780
ggtgattggt ctggatttgt agacaatccg caccttcaat ttaattataa aggctatggg 840
actgatactt ttggaaaagg agctagtact agtaattcat ctaaaccgag cgcagacaca 900
aacacaaaca gtctaggatt agtagattat atgaatttaa ataaactaga ttcaagcttt 960
gcgaatcgca aaaaactagc gacaagttac ggaattaaaa attacagtgg aacagcaacg 1020
cagaacacaa cattattagc gaagttaaaa gcaggaaaac cacacacacc agcaagcaaa 1080
aacacatact acacagaaaa tccgcgaaaa gttaaaacac tagtacaatg tgatctatac 1140
aaatcagtag actttacaac aaaaaaccaa acaggtggaa catttccgcc aggcacagtc 1200
ttcacgattt cagggatggg gaaaacgaaa ggcggaacac ctcgcttgaa gacgaagagc 1260
ggttactatc tcactgctaa cacgaagttt gttaaaaaga ttactggtaa aacagtagcc 1320
gcaaaaaatc caaaccgcca ttctcaaaac actaatacaa attcaaatcg ttacgagggt 1380
aaagtcattg atagcgcacc actgctaccg aaaatggact ttaaatcatc accattccgc 1440
atgtataagg taggaactga gttcttagta tatgatcata atcaatattg gtacaagaca 1500
tacattgatg acaaacttta ctacatgtat aaaagctttt gcgatgttgt agctaaaaaa 1560
gacgcaaaag gtcgcatcaa agttcgaatt aaaagcgcga aagacttgcg tattccagtc 1620
tggaataaca taaaattgaa ttctgggaaa attaaatggt atgcacccaa tgtaaaacta 1680
gcgtggtaca actatcgaag aggatattta gagctatggt atccgaacga cggctggtat 1740
tacacagcag aatacttctt aaaataa 1767

<210> 39
<211> 588
<212> PRT
<213> artificial sequence

<220>
<223> HGFP_CBD500L118:

<400> 39
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190

His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Ile Thr His Gly Met Asp Glu Leu Tyr Lys Glu Leu Gln Asn Thr Asn
                245                 250                 255
Thr Asn Ser Asn Arg Tyr Glu Gly Lys Val Ile Asp Ser Ala Pro Leu
            260                 265                 270
Leu Pro Lys Met Asp Phe Lys Ser Ser Pro Phe Arg Met Tyr Lys Val
        275                 280                 285
Gly Thr Glu Phe Leu Val Tyr Asp His Asn Gln Tyr Trp Tyr Lys Thr
    290                 295                 300
Tyr Ile Asp Asp Lys Leu Tyr Tyr Met Tyr Lys Ser Phe Cys Asp Val
305                 310                 315                 320
Val Ala Lys Lys Asp Ala Lys Gly Arg Ile Lys Val Arg Ile Lys Ser
                325                 330                 335
Ala Lys Asp Leu Arg Ile Pro Val Trp Asn Asn Ile Lys Leu Asn Ser
            340                 345                 350
Gly Lys Ile Lys Trp Tyr Ala Pro Asn Val Lys Leu Ala Trp Tyr Asn
        355                 360                 365
Tyr Arg Arg Gly Tyr Leu Glu Leu Trp Tyr Pro Asn Asp Gly Trp Tyr
    370                 375                 380
Tyr Thr Ala Glu Tyr Phe Leu Lys Thr Gly Lys Thr Val Ala Ala Lys
385                 390                 395                 400
Asn Pro Asn Arg His Ser Lys Ser Leu Gly Phe Glu Trp Gly Gly Asp
                405                 410                 415
Trp Ser Gly Phe Val Asp Asn Pro His Leu Gln Phe Asn Tyr Lys Gly
            420                 425                 430
Tyr Gly Thr Asp Thr Phe Gly Lys Gly Ala Ser Thr Ser Asn Ser Ser
        435                 440                 445
Lys Pro Ser Ala Asp Thr Asn Thr Asn Ser Leu Gly Leu Val Asp Tyr
    450                 455                 460
Met Asn Leu Asn Lys Leu Asp Ser Ser Phe Ala Asn Arg Lys Lys Leu
465                 470                 475                 480
Ala Thr Ser Tyr Gly Ile Lys Asn Tyr Ser Gly Thr Ala Thr Gln Asn
                485                 490                 495
Thr Thr Leu Leu Ala Lys Leu Lys Ala Gly Lys Pro His Thr Pro Ala
            500                 505                 510
Ser Lys Asn Thr Tyr Tyr Thr Glu Asn Pro Arg Lys Val Lys Thr Leu
        515                 520                 525
Val Gln Cys Asp Leu Tyr Lys Ser Val Asp Phe Thr Thr Lys Asn Gln
    530                 535                 540
Thr Gly Gly Thr Phe Pro Pro Gly Thr Val Phe Thr Ile Ser Gly Met
545                 550                 555                 560
Gly Lys Thr Lys Gly Gly Thr Pro Arg Leu Lys Thr Lys Ser Gly Tyr
                565                 570                 575
Tyr Leu Thr Ala Asn Thr Lys Phe Val Lys Lys Ile
            580                 585


<210>  40
<211>  1767
<212>  DNA
<213>  artificial sequence

<220>
<223>  HGFP_CBD500L118:

<400>  40
atgagaggat cgcatcacca tcaccatcac ggatccatga gtaaaggaga agaacttttc      60
actggagttg tcccaattct tgttgaatta gatggtgatg ttaatgggca caaattttct     120

gtcagtggag agggtgaagg tgatgcaaca tacggaaaac ttacccttaa atttatttgc 180
actactggaa aactacctgt tccatggcca acacttgtca ctactttcgc gtatggtctt 240
caatgctttg cgagataccc agatcatatg aaacggcatg actttttcaa gagtgccatg 300
cccgaaggtt atgtacagga aagaactata tttttcaaag atgacgggaa ctacaagaca 360
cgtgctgaag tcaagtttga aggtgatacc cttgttaata gaatcgagtt aaaaggtatt 420
gattttaaag aagatggaaa cattcttgga cacaaattgg aatacaacta taactcacac 480
aatgtataca tcatggcaga caaacaaaag aatggaatca aagttaactt caaaattaga 540
cacaacattg aagatggaag cgttcaacta gcagaccatt atcaacaaaa tactccaatt 600
ggcgatggcc ctgtcctttt accagacaac cattacctgt ccacacaatc tgccctttcg 660
aaagatccca acgaaaagag agaccacatg gtccttcttg agtttgtaac agctgctggg 720
attacacatg gcatggatga actatacaaa gagctccaaa acactaatac aaattcaaat 780
cgttacgagg gtaaagtcat tgatagcgca ccactgctac cgaaaatgga ctttaaatca 840
tcaccattcc gcatgtataa ggtaggaact gagttcttag tatatgatca taatcaatat 900
tggtacaaga catacattga tgacaaactt tactacatgt ataaaagctt ttgcgatgtt 960
gtagctaaaa aagacgcaaa aggtcgcatc aaagttcgaa ttaaaagcgc gaaagacttg 1020
cgtattccag tctggaataa cataaaattg aattctggga aaattaaatg gtatgcaccc 1080
aatgtaaaac tagcgtggta caactatcga agaggatatt tagagctatg gtatccgaac 1140
gacggctggt attacacagc agaatacttc ttaaaaactg gtaaaacagt agccgcaaaa 1200
aatccaaacc gccattctaa atctttaggt tttgagtggg gtggtgattg gtctggattt 1260
gtagacaatc cgcaccttca atttaattat aaaggctatg ggactgatac ttttggaaaa 1320
ggagctagta ctagtaattc atctaaaccg agcgcagaca caaacacaaa cagtctagga 1380
ttagtagatt atatgaattt aaataaacta gattcaagct ttgcgaatcg caaaaaacta 1440
gcgacaagtt acggaattaa aaattacagt ggaacagcaa cgcagaacac aacattatta 1500
gcgaagttaa aagcaggaaa accacacaca ccagcaagca aaaacacata ctacacagaa 1560
aatccgcgaa aagttaaaac actagtacaa tgtgatctat acaaatcagt agactttaca 1620
acaaaaaacc aaacaggtgg aacatttccg ccaggcacag tcttcacgat ttcagggatg 1680
gggaaaacga aaggcggaac acctcgcttg aagacgaaga gcggttacta tctcactgct 1740
aacacgaagt ttgttaaaaa gatttaa 1767

<210> 41
<211> 556
<212> PRT
<213> artificial sequence

<220>
<223> HGFP_CBD500-P35:

<400> 41
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190

His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Ile Thr His Gly Met Asp Glu Leu Tyr Lys Glu Leu Gln Asn Thr Asn
                245                 250                 255
Thr Asn Ser Asn Arg Tyr Glu Gly Lys Val Ile Asp Ser Ala Pro Leu
            260                 265                 270
Leu Pro Lys Met Asp Phe Lys Ser Ser Pro Phe Arg Met Tyr Lys Val
        275                 280                 285
Gly Thr Glu Phe Leu Val Tyr Asp His Asn Gln Tyr Trp Tyr Lys Thr
    290                 295                 300
Tyr Ile Asp Asp Lys Leu Tyr Tyr Met Tyr Lys Ser Phe Cys Asp Val
305                 310                 315                 320
Val Ala Lys Lys Asp Ala Lys Gly Arg Ile Lys Val Arg Ile Lys Ser
                325                 330                 335
Ala Lys Asp Leu Arg Ile Pro Val Trp Asn Asn Ile Lys Leu Asn Ser
            340                 345                 350
Gly Lys Ile Lys Trp Tyr Ala Pro Asn Val Lys Leu Ala Trp Tyr Asn
        355                 360                 365
Tyr Arg Arg Gly Tyr Leu Glu Leu Trp Tyr Pro Asn Asp Gly Trp Tyr
    370                 375                 380
Tyr Thr Ala Glu Tyr Phe Leu Lys Gln Phe Pro His Phe Glu Ala Cys
385                 390                 395                 400
Asp Trp Tyr Arg Gly Glu Arg Lys Tyr Lys Val Asp Thr Ser Glu Trp
                405                 410                 415
Lys Lys Lys Glu Asn Ile Asn Ile Val Ile Lys Asp Val Gly Tyr Phe
            420                 425                 430
Gln Asp Lys Pro Gln Phe Leu Asn Ser Lys Ser Val Arg Gln Trp Lys
        435                 440                 445
His Gly Thr Lys Val Lys Leu Thr Lys His Asn Ser His Trp Tyr Thr
    450                 455                 460
Gly Val Val Lys Asp Gly Asn Lys Ser Val Arg Gly Tyr Ile Tyr His
465                 470                 475                 480
Ser Met Ala Lys Val Thr Ser Lys Asn Ser Asp Gly Ser Val Asn Ala
                485                 490                 495
Thr Ile Asn Ala His Ala Phe Cys Trp Asp Asn Lys Lys Leu Asn Gly
            500                 505                 510
Gly Asp Phe Ile Asn Leu Lys Arg Gly Phe Lys Gly Ile Thr His Pro
        515                 520                 525
Ala Ser Asp Gly Phe Tyr Pro Leu Tyr Phe Ala Ser Arg Lys Lys Thr
    530                 535                 540
Phe Tyr Ile Pro Arg Tyr Met Phe Asp Ile Lys Lys
545                 550                 555


<210>  42
<211>  1671
<212>  DNA
<213>  artificial sequence

<220>
<223>  HGFP_CBD500-P35:

<400>  42
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180
actactggaa aactacctgt tccatggcca acacttgtca ctactttcgc gtatggtctt     240
caatgctttg cgagataccc agatcatatg aaacggcatg actttttcaa gagtgccatg     300
cccgaaggtt atgtacagga aagaactata tttttcaaag atgacgggaa ctacaagaca     360

cgtgctgaag tcaagtttga aggtgatacc cttgttaata gaatcgagtt aaaaggtatt 420
gattttaaag aagatggaaa cattcttgga cacaaattgg aatacaacta taactcacac 480
aatgtataca tcatggcaga caaacaaaag aatggaatca aagttaactt caaaattaga 540
cacaacattg aagatggaag cgttcaacta gcagaccatt atcaacaaaa tactccaatt 600
ggcgatggcc ctgtcctttt accagacaac cattacctgt ccacacaatc tgccctttcg 660
aaagatccca acgaaaagag agaccacatg gtccttcttg agtttgtaac agctgctggg 720
attacacatg gcatggatga actatacaaa gagctccaaa acactaatac aaattcaaat 780
cgttacgagg gtaaagtcat tgatagcgca ccactgctac cgaaaatgga ctttaaatca 840
tcaccattcc gcatgtataa ggtaggaact gagttcttag tatatgatca taatcaatat 900
tggtacaaga catacattga tgacaaactt tactacatgt ataaaagctt ttgcgatgtt 960
gtagctaaaa aagacgcaaa aggtcgcatc aaagttcgaa ttaaaagcgc gaaagacttg 1020
cgtattccag tctggaataa cataaaattg aattctggga aaattaaatg gtatgcaccc 1080
aatgtaaaac tagcgtggta caactatcga agaggatatt tagagctatg gtatccgaac 1140
gacggctggt attacacagc agaatacttc ttaaaacaat tcccacattt tgaagcttgt 1200
gactggtatc gcggggaacg caagtataaa gtggacacat ctgaatggaa aaagaaagag 1260
aatatcaata tcgttattaa agatgttggt tacttccaag acaaacctca attcttaaac 1320
tccaaatcgg ttcgtcagtg gaagcatggc acgaaagtga agcttactaa acataactca 1380
cattggtaca ctggtgtggt caaggatggt aacaaatcag tcaggggata tatttatcat 1440
tcgatggcta aggtcacaag caagaatagc gacggttcgg ttaacgcaac gattaacgcc 1500
cacgcatttt gttgggacaa taaaaaactt aatggtggcg actttatcaa cttgaagcgt 1560
ggttttaaag gtatcaccca tcccgctagt gacggtttct atccactgta tttcgcttct 1620
aggaaaaaaa ctttctacat tccgcgttac atgtttgaca tcaagaaata a 1671

<210> 43
<211> 556
<212> PRT
<213> artificial sequence

<220>
<223> HGFP_CBDP35-500:

<400> 43

Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly

225                 230                 235                 240
Ile Thr His Gly Met Asp Glu Leu Tyr Lys Glu Leu Pro His Phe Glu
                245                 250                 255
Ala Cys Asp Trp Tyr Arg Gly Glu Arg Lys Tyr Lys Val Asp Thr Ser
            260                 265                 270
Glu Trp Lys Lys Lys Glu Asn Ile Asn Ile Val Ile Lys Asp Val Gly
        275                 280                 285
Tyr Phe Gln Asp Lys Pro Gln Phe Leu Asn Ser Lys Ser Val Arg Gln
    290                 295                 300
Trp Lys His Gly Thr Lys Val Lys Leu Thr Lys His Asn Ser His Trp
305                 310                 315                 320
Tyr Thr Gly Val Val Lys Asp Gly Asn Lys Ser Val Arg Gly Tyr Ile
                325                 330                 335
Tyr His Ser Met Ala Lys Val Thr Ser Lys Asn Ser Asp Gly Ser Val
            340                 345                 350
Asn Ala Thr Ile Asn Ala His Ala Phe Cys Trp Asp Asn Lys Lys Leu
        355                 360                 365
Asn Gly Gly Asp Phe Ile Asn Leu Lys Arg Gly Phe Lys Gly Ile Thr
    370                 375                 380
His Pro Ala Ser Asp Gly Phe Tyr Pro Leu Tyr Phe Ala Ser Arg Lys
385                 390                 395                 400
Lys Thr Phe Tyr Ile Pro Arg Tyr Met Phe Asp Ile Lys Lys Gln Phe
                405                 410                 415
Gln Asn Thr Asn Thr Asn Ser Asn Arg Tyr Glu Gly Lys Val Ile Asp
            420                 425                 430
Ser Ala Pro Leu Leu Pro Lys Met Asp Phe Lys Ser Ser Pro Phe Arg
        435                 440                 445
Met Tyr Lys Val Gly Thr Glu Phe Leu Val Tyr Asp His Asn Gln Tyr
    450                 455                 460
Trp Tyr Lys Thr Tyr Ile Asp Asp Lys Leu Tyr Tyr Met Tyr Lys Ser
465                 470                 475                 480
Phe Cys Asp Val Val Ala Lys Lys Asp Ala Lys Gly Arg Ile Lys Val
                485                 490                 495
Arg Ile Lys Ser Ala Lys Asp Leu Arg Ile Pro Val Trp Asn Asn Ile
            500                 505                 510
Lys Leu Asn Ser Gly Lys Ile Lys Trp Tyr Ala Pro Asn Val Lys Leu
        515                 520                 525
Ala Trp Tyr Asn Tyr Arg Arg Gly Tyr Leu Glu Leu Trp Tyr Pro Asn
    530                 535                 540
Asp Gly Trp Tyr Tyr Thr Ala Glu Tyr Phe Leu Lys
545                 550                 555


<210>  44
<211>  1671
<212>  DNA
<213>  artificial sequence

<220>
<223>  HGFP_CBDP35-500:

<400>  44
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180
actactggaa aactacctgt tccatggcca acacttgtca ctactttcgc gtatggtctt     240
caatgctttg cgagataccc agatcatatg aaacggcatg actttttcaa gagtgccatg     300
cccgaaggtt atgtacagga aagaactata tttttcaaag atgacgggaa ctacaagaca     360
cgtgctgaag tcaagtttga aggtgatacc cttgttaata gaatcgagtt aaaaggtatt     420
gattttaaag aagatggaaa cattcttgga cacaaattgg aatacaacta taactcacac     480
aatgtataca tcatggcaga caaacaaaag aatggaatca aagttaactt caaaattaga     540
cacaacattg aagatggaag cgttcaacta gcagaccatt atcaacaaaa tactccaatt     600
ggcgatggcc ctgtcctttt accagacaac cattacctgt ccacacaatc tgccctttcg     660

aaagatccca acgaaaagag agaccacatg gtccttcttg agtttgtaac agctgctggg 720
attacacatg gcatggatga actatacaaa gagctcccac attttgaagc ttgtgactgg 780
tatcgcgggg aacgcaagta taaagtggac acatctgaat ggaaaaagaa agagaatatc 840
aatatcgtta ttaaagatgt tggttacttc caagacaaac ctcaattctt aaactccaaa 900
tcggttcgtc agtggaagca tggcacgaaa gtgaagctta ctaaacataa ctcacattgg 960
tacactggtg tggtcaagga tggtaacaaa tcagtcaggg gatatattta tcattcgatg 1020
gctaaggtca caagcaagaa tagcgacggt tcggttaacg caacgattaa cgcccacgca 1080
ttttgttggg acaataaaaa acttaatggt ggcgacttta tcaacttgaa gcgtggtttt 1140
aaaggtatca cccatcccgc tagtgacggt ttctatccac tgtatttcgc ttctaggaaa 1200
aaaactttct acattccgcg ttacatgttt gacatcaaga aacaattcca aaacactaat 1260
acaaattcaa atcgttacga gggtaaagtc attgatagcg caccactgct accgaaaatg 1320
gactttaaat catcaccatt ccgcatgtat aaggtaggaa ctgagttctt agtatatgat 1380
cataatcaat attggtacaa gacatacatt gatgacaaac tttactacat gtataaaagc 1440
ttttgcgatg ttgtagctaa aaaagacgca aaaggtcgca tcaaagttcg aattaaaagc 1500
gcgaaagact tgcgtattcc agtctggaat aacataaaat tgaattctgg gaaaattaaa 1560
tggtatgcac ccaatgtaaa actagcgtgg tacaactatc gaagaggata tttagagcta 1620
tggtatccga acgacggctg gtattacaca gcagaatact tcttaaaata a 1671

<210> 45
<211> 586
<212> PRT
<213> artificial sequence

<220>
<223> HCBD500_GFP_CBD118:

<400> 45
Met Arg Gly Ser His His His His His His Gly Ser Gln Asn Thr Asn
1               5                   10                  15
Thr Asn Ser Asn Arg Tyr Glu Gly Lys Val Ile Asp Ser Ala Pro Leu
            20                  25                  30
Leu Pro Lys Met Asp Phe Lys Ser Ser Pro Phe Arg Met Tyr Lys Val
        35                  40                  45
Gly Thr Glu Phe Leu Val Tyr Asp His Asn Gln Tyr Trp Tyr Lys Thr
    50                  55                  60
Tyr Ile Asp Asp Lys Leu Tyr Tyr Met Tyr Lys Ser Phe Cys Asp Val
65                  70                  75                  80
Val Ala Lys Lys Asp Ala Lys Gly Arg Ile Lys Val Arg Ile Lys Ser
                85                  90                  95
Ala Lys Asp Leu Arg Ile Pro Val Trp Asn Asn Ile Lys Leu Asn Ser
            100                 105                 110
Gly Lys Ile Lys Trp Tyr Ala Pro Asn Val Lys Leu Ala Trp Tyr Asn
        115                 120                 125
Tyr Arg Arg Gly Tyr Leu Glu Leu Trp Tyr Pro Asn Asp Gly Trp Tyr
    130                 135                 140
Tyr Thr Ala Glu Tyr Phe Leu Lys Gly Thr Met Ser Lys Gly Glu Glu
145                 150                 155                 160
Leu Phe Thr Gly Val Val Pro Ile Leu Val Glu Leu Asp Gly Asp Val
                165                 170                 175
Asn Gly His Lys Phe Ser Val Ser Gly Glu Gly Glu Gly Asp Ala Thr
            180                 185                 190
Tyr Gly Lys Leu Thr Leu Lys Phe Ile Cys Thr Thr Gly Lys Leu Pro
        195                 200                 205
Val Pro Trp Pro Thr Leu Val Thr Thr Phe Ala Tyr Gly Leu Gln Cys
    210                 215                 220
Phe Ala Arg Tyr Pro Asp His Met Lys Arg His Asp Phe Phe Lys Ser
225                 230                 235                 240
Ala Met Pro Glu Gly Tyr Val Gln Glu Arg Thr Ile Phe Phe Lys Asp
                245                 250                 255

Asp Gly Asn Tyr Lys Thr Arg Ala Glu Val Lys Phe Glu Gly Asp Thr

260                 265                 270
Leu Val Asn Arg Ile Glu Leu Lys Gly Ile Asp Phe Lys Glu Asp Gly
        275                 280                 285
Asn Ile Leu Gly His Lys Leu Glu Tyr Asn Tyr Asn Ser His Asn Val
    290                 295                 300
Tyr Ile Met Ala Asp Lys Gln Lys Asn Gly Ile Lys Val Asn Phe Lys
305                 310                 315                 320
Ile Arg His Asn Ile Glu Asp Gly Ser Val Gln Leu Ala Asp His Tyr
                325                 330                 335
Gln Gln Asn Thr Pro Ile Gly Asp Gly Pro Val Leu Leu Pro Asp Asn
            340                 345                 350
His Tyr Leu Ser Thr Gln Ser Ala Leu Ser Lys Asp Pro Asn Glu Lys
        355                 360                 365
Arg Asp His Met Val Leu Leu Glu Phe Val Thr Ala Ala Gly Ile Thr
    370                 375                 380
His Gly Met Asp Glu Leu Tyr Lys Glu Leu Asp Lys Gly Lys Lys Phe
385                 390                 395                 400
Val Ala Lys Ala Lys Ser Leu Gly Phe Glu Trp Gly Gly Asp Trp Ser
                405                 410                 415
Gly Phe Val Asp Asn Pro His Leu Gln Phe Asn Tyr Lys Gly Tyr Gly
            420                 425                 430
Thr Asp Thr Phe Gly Lys Gly Ala Ser Thr Ser Asn Ser Ser Lys Pro
        435                 440                 445
Ser Ala Asp Thr Asn Thr Asn Ser Leu Gly Leu Val Asp Tyr Met Asn
    450                 455                 460
Leu Asn Lys Leu Asp Ser Ser Phe Ala Asn Arg Lys Lys Leu Ala Thr
465                 470                 475                 480
Ser Tyr Gly Ile Lys Asn Tyr Ser Gly Thr Ala Thr Gln Asn Thr Thr
                485                 490                 495
Leu Leu Ala Lys Leu Lys Ala Gly Lys Pro His Thr Pro Ala Ser Lys
            500                 505                 510
Asn Thr Tyr Tyr Thr Glu Asn Pro Arg Lys Val Lys Thr Leu Val Gln
        515                 520                 525
Cys Asp Leu Tyr Lys Ser Val Asp Phe Thr Thr Lys Asn Gln Thr Gly
    530                 535                 540
Gly Thr Phe Pro Pro Gly Thr Val Phe Thr Ile Ser Gly Met Gly Lys
545                 550                 555                 560
Thr Lys Gly Gly Thr Pro Arg Leu Lys Thr Lys Ser Gly Tyr Tyr Leu
                565                 570                 575
Thr Ala Asn Thr Lys Phe Val Lys Lys Ile
            580                 585


<210>  46
<211>  1761
<212>  DNA
<213>  artificial sequence

<220>
<223>  HCBD500_GFP_CBD118:

<400>  46
atgagaggat cgcatcacca tcaccatcac ggatcccaaa acactaatac aaattcaaat      60
cgttacgagg gtaaagtcat tgatagcgca ccactgctac cgaaaatgga ctttaaatca     120
tcaccattcc gcatgtataa ggtaggaact gagttcttag tatatgatca taatcaatat     180
tggtacaaga catacattga tgacaaactt tactacatgt ataaaagctt ttgcgatgtt     240
gtagctaaaa aagacgcaaa aggtcgcatc aaagttcgaa ttaaaagcgc gaaagacttg     300
cgtattccag tctggaataa cataaaattg aattctggga aaattaaatg gtatgcaccc     360
aatgtaaaac tagcgtggta caactatcga agaggatatt tagagctatg gtatccgaac     420
gacggctggt attacacagc agaatacttc ttaaaaggta ccatgagtaa aggagaagaa     480
cttttcactg gagttgtccc aattcttgtt gaattagatg gtgatgttaa tgggcacaaa     540
ttttctgtca gtggagaggg tgaaggtgat gcaacatacg gaaaacttac ccttaaattt     600
atttgcacta ctggaaaact acctgttcca tggccaacac ttgtcactac tttcgcgtat     660

ggtcttcaat gctttgcgag atacccagat catatgaaac ggcatgactt tttcaagagt 720
gccatgcccg aaggttatgt acaggaaaga actatatttt tcaaagatga cgggaactac 780
aagacacgtg ctgaagtcaa gtttgaaggt gatacccttg ttaatagaat cgagttaaaa 840
ggtattgatt ttaaagaaga tggaaacatt cttggacaca aattggaata caactataac 900
tcacacaatg tatacatcat ggcagacaaa caaaagaatg gaatcaaagt taacttcaaa 960
attagacaca acattgaaga tggaagcgtt caactagcag accattatca acaaaatact 1020
ccaattggcg atggccctgt ccttttacca gacaaccatt acctgtccac acaatctgcc 1080
ctttcgaaag atcccaacga aaagagagac cacatggtcc ttcttgagtt tgtaacagct 1140
gctgggatta cacatggcat ggatgaacta tacaaagagc tcgacaaagg caagaaattt 1200
gtggcaaagg caaaatcttt aggttttgag tggggtggtg attggtctgg atttgtagac 1260
aatccgcacc ttcaatttaa ttataaaggc tatggaactg atacttttgg aaaaggagct 1320
agtactagta attcatctaa accgagcgca gacacaaaca caaacagtct aggattagta 1380
gattatatga atttaaataa actagattca agctttgcga atcgcaaaaa actagcgaca 1440
agttacggaa ttaaaaatta cagtggaaca gcaacgcaga acacaacatt attagcgaag 1500
ttaaaagcag gaaaaccaca cacaccagca agcaaaaaca catactacac agaaaatccg 1560
cgaaaagtta aaacactagt acaatgtgat ctatacaaat cagtagactt tacaacaaaa 1620
aaccaaacag gtggaacatt tccgccaggc acagtcttca cgatttcagg gatggggaaa 1680
acgaaaggcg gaacacctcg cttgaagacg aagagcggtt actatctcac tgctaacacg 1740
aagtttgtta aaaagattta a 1761

<210> 47
<211> 601
<212> PRT
<213> artificial sequence

<220>
<223> HCBD118_GFP_CBD500:

<400> 47
Met Arg Gly Ser His His His His His His Gly Ser Asp Lys Gly Lys
1               5                   10                  15
Lys Phe Val Ala Lys Ala Lys Ser Leu Gly Phe Glu Trp Gly Gly Asp
            20                  25                  30
Trp Ser Gly Phe Val Asp Asn Pro His Leu Gln Phe Asn Tyr Lys Gly
        35                  40                  45
Tyr Gly Thr Asp Thr Phe Gly Lys Gly Ala Ser Thr Ser Asn Ser Ser
    50                  55                  60
Lys Pro Ser Ala Asp Thr Asn Thr Asn Ser Leu Gly Leu Val Asp Tyr
65                  70                  75                  80
Met Asn Leu Asn Lys Leu Asp Ser Ser Phe Ala Asn Arg Lys Lys Leu
                85                  90                  95
Ala Thr Ser Tyr Gly Ile Lys Asn Tyr Ser Gly Thr Ala Thr Gln Asn
            100                 105                 110
Thr Thr Leu Leu Ala Lys Leu Lys Ala Gly Lys Pro His Thr Pro Ala
        115                 120                 125
Ser Lys Asn Thr Tyr Tyr Thr Glu Asn Pro Arg Lys Val Lys Thr Leu
    130                 135                 140
Val Gln Cys Asp Leu Tyr Lys Ser Val Asp Phe Thr Thr Lys Asn Gln
145                 150                 155                 160
Thr Gly Gly Thr Phe Pro Pro Gly Thr Val Phe Thr Ile Ser Gly Met
                165                 170                 175
Gly Lys Thr Lys Gly Gly Thr Pro Arg Leu Lys Thr Lys Ser Gly Tyr
            180                 185                 190
Tyr Leu Thr Ala Asn Thr Lys Phe Val Lys Lys Ile Met Ser Lys Gly
        195                 200                 205
Glu Glu Leu Phe Thr Gly Val Val Pro Ile Leu Val Glu Leu Asp Gly
    210                 215                 220
Asp Val Asn Gly His Lys Phe Ser Val Ser Gly Glu Gly Glu Gly Asp
225                 230                 235                 240
Ala Thr Tyr Gly Lys Leu Thr Leu Lys Phe Ile Cys Thr Thr Gly Lys
                245                 250                 255
Leu Pro Val Pro Trp Pro Thr Leu Val Thr Thr Phe Ala Tyr Gly Leu

            260                 265                 270
Gln Cys Phe Ala Arg Tyr Pro Asp His Met Lys Arg His Asp Phe Phe
        275                 280                 285
Lys Ser Ala Met Pro Glu Gly Tyr Val Gln Glu Arg Thr Ile Phe Phe
    290                 295                 300
Lys Asp Asp Gly Asn Tyr Lys Thr Arg Ala Glu Val Lys Phe Glu Gly
305                 310                 315                 320
Asp Thr Leu Val Asn Arg Ile Glu Leu Lys Gly Ile Asp Phe Lys Glu
                325                 330                 335
Asp Gly Asn Ile Leu Gly His Lys Leu Glu Tyr Asn Tyr Asn Ser His
            340                 345                 350
Asn Val Tyr Ile Met Ala Asp Lys Gln Lys Asn Gly Ile Lys Val Asn
        355                 360                 365
Phe Lys Ile Arg His Asn Ile Glu Asp Gly Ser Val Gln Leu Ala Asp
    370                 375                 380
His Tyr Gln Gln Asn Thr Pro Ile Gly Asp Gly Pro Val Leu Leu Pro
385                 390                 395                 400
Asp Asn His Tyr Leu Ser Thr Gln Ser Ala Leu Ser Lys Asp Pro Asn
                405                 410                 415
Glu Lys Arg Asp His Met Val Leu Leu Glu Phe Val Thr Ala Ala Gly
            420                 425                 430
Ile Thr His Gly Met Asp Glu Leu Tyr Lys Glu Leu His Phe Glu Leu
        435                 440                 445
Cys Asp Ala Val Ser Gly Glu Lys Ile Pro Ala Ala Thr Gln Asn Thr
    450                 455                 460
Asn Thr Asn Ser Asn Arg Tyr Glu Gly Lys Val Ile Asp Ser Ala Pro
465                 470                 475                 480
Leu Leu Pro Lys Met Asp Phe Lys Ser Ser Pro Phe Arg Met Tyr Lys
                485                 490                 495
Val Gly Thr Glu Phe Leu Val Tyr Asp His Asn Gln Tyr Trp Tyr Lys
            500                 505                 510
Thr Tyr Ile Asp Asp Lys Leu Tyr Tyr Met Tyr Lys Ser Phe Cys Asp
        515                 520                 525
Val Val Ala Lys Lys Asp Ala Lys Gly Arg Ile Lys Val Arg Ile Lys
    530                 535                 540
Ser Ala Lys Asp Leu Arg Ile Pro Val Trp Asn Asn Ile Lys Leu Asn
545                 550                 555                 560
Ser Gly Lys Ile Lys Trp Tyr Ala Pro Asn Val Lys Leu Ala Trp Tyr
                565                 570                 575
Asn Tyr Arg Arg Gly Tyr Leu Glu Leu Trp Tyr Pro Asn Asp Gly Trp
            580                 585                 590
Tyr Tyr Thr Ala Glu Tyr Phe Leu Lys
        595                 600


<210>  48
<211>  1806
<212>  DNA
<213>  artificial sequence

<220>
<223>  HCBD118_GFP_CBD500:

<400>  48
atgagaggat cgcatcacca tcaccatcac ggatccgaca aaggcaagaa atttgtggca      60
aaggcaaaat ctttaggttt tgagtggggt ggtgattggt ctggatttgt agacaatccg     120
caccttcaat ttaattataa aggctatggg actgatactt ttggaaaagg agctagtact     180
agtaattcat ctaaaccgag cgcagacaca aacacaaaca gtctaggatt agtagattat     240
atgaatttaa ataaactaga ttcaagcttt gcgaatcgca aaaaactagc gacaagttac     300
ggaattaaaa attacagtgg aacagcaacg cagaacacaa cattattagc gaagttaaaa     360
gcaggaaaac cacacacacc agcaagcaaa aacacatact acacagaaaa tccgcgaaaa     420
gttaaaacac tagtacaatg tgatctatac aaatcagtag actttacaac aaaaaaccaa     480
acaggtggaa catttccgcc aggcacagtc ttcacgattt cagggatggg gaaaacgaaa     540

ggcggaacac ctcgcttgaa gacgaagagc ggttactatc tcactgctaa cacgaagttt 600
gttaaaaaga ttatgagtaa aggagaagaa cttttcactg gagttgtccc aattcttgtt 660
gaattagatg gtgatgttaa tgggcacaaa ttttctgtca gtggagaggg tgaaggtgat 720
gcaacatacg gaaaacttac ccttaaattt atttgcacta ctggaaaact acctgttcca 780
tggccaacac ttgtcactac tttcgcgtat ggtcttcaat gctttgcgag atacccagat 840
catatgaaac ggcatgactt tttcaagagt gccatgcccg aaggttatgt acaggaaaga 900
actatatttt tcaaagatga cgggaactac aagacacgtg ctgaagtcaa gtttgaaggt 960
gatacccttg ttaatagaat cgagttaaaa ggtattgatt ttaaagaaga tggaaacatt 1020
cttggacaca aattggaata caactataac tcacacaatg tatacatcat ggcagacaaa 1080
caaaagaatg gaatcaaagt taacttcaaa attagacaca acattgaaga tggaagcgtt 1140
caactagcag accattatca acaaaatact ccaattggcg atggccctgt ccttttacca 1200
gacaaccatt acctgtccac acaatctgcc ctttcgaaag atcccaacga aaagagagac 1260
cacatggtcc ttcttgagtt tgtaacagct gctgggatta cacatggcat ggatgaacta 1320
tacaaagagc tccattttga actatgtgat gctgtaagtg gtgagaaaat ccctgctgca 1380
acacaaaaca ctaatacaaa ttcaaatcgt tacgagggta aagtcattga tagcgcacca 1440
ctgctaccga aaatggactt taaatcatca ccattccgca tgtataaggt aggaactgag 1500
ttcttagtat atgatcataa tcaatattgg tacaagacat acattgatga caaactttac 1560
tacatgtata aagcttttgc gatgttgta gctaaaaaag acgcaaaagg tcgcatcaaa 1620
gttcgaatta aaagcgcgaa agacttgcgt attccagtct ggaataacat aaaattgaat 1680
tctgggaaaa ttaaatggta tgcacccaat gtaaaactag cgtggtacaa ctatcgaaga 1740
ggatatttag agctatggta tccgaacgac ggctggtatt acacagcaga atacttctta 1800
aaataa 1806

<210> 49
<211> 551
<212> PRT
<213> artificial sequence

<220>
<223> HGFP_CBD500-500:

<400> 49
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240

Ile Thr His Gly Met Asp Glu Leu Tyr Lys Glu Leu Gln Asn Thr Asn
                245                 250                 255
Thr Asn Ser Asn Arg Tyr Glu Gly Lys Val Ile Asp Ser Ala Pro Leu
            260                 265                 270
Leu Pro Lys Met Asp Phe Lys Ser Ser Pro Phe Arg Met Tyr Lys Val
        275                 280                 285
Gly Thr Glu Phe Leu Val Tyr Asp His Asn Gln Tyr Trp Tyr Lys Thr
    290                 295                 300
Tyr Ile Asp Asp Lys Leu Tyr Tyr Met Tyr Lys Ser Phe Cys Asp Val
305                 310                 315                 320
Val Ala Lys Lys Asp Ala Lys Gly Arg Ile Lys Val Arg Ile Lys Ser
                325                 330                 335
Ala Lys Asp Leu Arg Ile Pro Val Trp Asn Asn Ile Lys Leu Asn Ser
            340                 345                 350
Gly Lys Ile Lys Trp Tyr Ala Pro Asn Val Lys Leu Ala Trp Tyr Asn
        355                 360                 365
Tyr Arg Arg Gly Tyr Leu Glu Leu Trp Tyr Pro Asn Asp Gly Trp Tyr
    370                 375                 380
Tyr Thr Ala Glu Tyr Phe Leu Lys Glu Leu His Phe Glu Leu Cys Asp
385                 390                 395                 400
Ala Val Ser Gly Glu Lys Ile Pro Ala Ala Thr Gln Asn Thr Asn Thr
                405                 410                 415
Asn Ser Asn Arg Tyr Glu Gly Lys Val Ile Asp Ser Ala Pro Leu Leu
            420                 425                 430
Pro Lys Met Asp Phe Lys Ser Ser Pro Phe Arg Met Tyr Lys Val Gly
        435                 440                 445
Thr Glu Phe Leu Val Tyr Asp His Asn Gln Tyr Trp Tyr Lys Thr Tyr
    450                 455                 460
Ile Asp Asp Lys Leu Tyr Tyr Met Tyr Lys Ser Phe Cys Asp Val Val
465                 470                 475                 480
Ala Lys Lys Asp Ala Lys Gly Arg Ile Lys Val Arg Ile Lys Ser Ala
                485                 490                 495
Lys Asp Leu Arg Ile Pro Val Trp Asn Asn Ile Lys Leu Asn Ser Gly
            500                 505                 510
Lys Ile Lys Trp Tyr Ala Pro Asn Val Lys Leu Ala Trp Tyr Asn Tyr
        515                 520                 525
Arg Arg Gly Tyr Leu Glu Leu Trp Tyr Pro Asn Asp Gly Trp Tyr Tyr
    530                 535                 540
Thr Ala Glu Tyr Phe Leu Lys
545                 550

<210>  50
<211>  1656
<212>  DNA
<213>  artificial sequence

<220>
<223>  HGFP_CBD500-500:

<400>  50
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180
actactggaa aactacctgt tccatggcca acacttgtca ctactttcgc gtatggtctt     240
caatgctttg cgagataccc agatcatatg aaacggcatg actttttcaa gagtgccatg     300
cccgaaggtt atgtacagga aagaactata tttttcaaag atgacgggaa ctacaagaca     360
cgtgctgaag tcaagtttga aggtgatacc cttgttaata gaatcgagtt aaaaggtatt     420
gattttaaag aagatggaaa cattcttgga cacaaattgg aatacaacta taactcacac     480
aatgtataca tcatggcaga caaacaaaag aatggaatca aagttaactt caaaattaga     540
cacaacattg aagatggaag cgttcaacta gcagaccatt atcaacaaaa tactccaatt     600
ggcgatggcc ctgtcctttt accagacaac cattacctgt ccacacaatc tgccctttcg     660
aaagatccca acgaaaagag agaccacatg gtccttcttg agtttgtaac agctgctggg     720

attacacatg gcatggatga actatacaaa gagctccaaa acactaatac aaattcaaat 780
cgttacgagg gtaaagtcat tgatagcgca ccactgctac cgaaaatgga ctttaaatca 840
tcaccattcc gcatgtataa ggtaggaact gagttcttag tatatgatca taatcaatat 900
tggtacaaga catacattga tgacaaactt tactacatgt ataaaagctt ttgcgatgtt 960
gtagctaaaa aagacgcaaa aggtcgcatc aaagttcgaa ttaaaagcgc gaaagacttg 1020
cgtattccag tctggaataa cataaaattg aattctggga aaattaaatg gtatgcaccc 1080
aatgtaaaac tagcgtggta caactatcga agaggatatt tagagctatg gtatccgaac 1140
gacggctggt attacacagc agaatacttc ttaaaagagc tccattttga actatgtgat 1200
gctgtaagtg gtgagaaaat ccctgctgca acacaaaaca ctaatacaaa ttcaaatcgt 1260
tacgagggta aagtcattga tagcgcacca ctgctaccga aaatggactt taaatcatca 1320
ccattccgca tgtataaggt aggaactgag ttcttagtat atgatcataa tcaatattgg 1380
tacaagacat acattgatga caaactttac tacatgtata aaagcttttg cgatgttgta 1440
gctaaaaaag acgcaaaagg tcgcatcaaa gttcgaatta aaagcgcgaa agacttgcgt 1500
attccagtct ggaataacat aaaattgaat tctgggaaaa ttaaatggta tgcacccaat 1560
gtaaaactag cgtggtacaa ctatcgaaga ggatatttag agctatggta tccgaacgac 1620
ggctggtatt acacagcaga atacttctta aaataa 1656

<210> 51
<211> 460
<212> PRT
<213> artificial sequence

<220>
<223> HEAD_CBD500-500:

<400> 51
Met Arg Gly Ser His His His His His His Gly Ser Met Ala Leu Thr
1               5                   10                  15
Glu Ala Trp Leu Ile Glu Lys Ala Asn Arg Lys Leu Asn Ala Gly Gly
            20                  25                  30
Met Tyr Lys Ile Thr Ser Asp Lys Thr Arg Asn Val Ile Lys Lys Met
        35                  40                  45
Ala Lys Glu Gly Ile Tyr Leu Cys Val Ala Gln Gly Tyr Arg Ser Thr
    50                  55                  60
Ala Glu Gln Asn Ala Leu Tyr Ala Gln Gly Arg Thr Lys Pro Gly Ala
65                  70                  75                  80
Ile Val Thr Asn Ala Lys Gly Gly Gln Ser Asn His Asn Tyr Gly Val
                85                  90                  95
Ala Val Asp Leu Cys Leu Tyr Thr Asn Asp Gly Lys Asp Val Ile Trp
            100                 105                 110
Glu Ser Thr Thr Ser Arg Trp Lys Lys Val Val Ala Ala Met Lys Ala
        115                 120                 125
Glu Gly Phe Lys Trp Gly Gly Asp Trp Lys Ser Phe Lys Asp Tyr Pro
    130                 135                 140
His Phe Glu Leu Cys Asp Ala Val Ser Gly Glu Lys Ile Pro Ala Ala
145                 150                 155                 160
Thr Gln Asn Thr Asn Thr Asn Ser Asn Arg Tyr Glu Gly Lys Val Ile
                165                 170                 175
Asp Ser Ala Pro Leu Leu Pro Lys Met Asp Phe Lys Ser Ser Pro Phe
            180                 185                 190
Arg Met Tyr Lys Val Gly Thr Glu Phe Leu Val Tyr Asp His Asn Gln
        195                 200                 205
Tyr Trp Tyr Lys Thr Tyr Ile Asp Asp Lys Leu Tyr Tyr Met Tyr Lys
    210                 215                 220
Ser Phe Cys Asp Val Val Ala Lys Lys Asp Ala Lys Gly Arg Ile Lys
225                 230                 235                 240
Val Arg Ile Lys Ser Ala Lys Asp Leu Arg Ile Pro Val Trp Asn Asn
                245                 250                 255
Ile Lys Leu Asn Ser Gly Lys Ile Lys Trp Tyr Ala Pro Asn Val Lys
            260                 265                 270
Leu Ala Trp Tyr Asn Tyr Arg Arg Gly Tyr Leu Glu Leu Trp Tyr Pro
        275                 280                 285

Asn Asp Gly Trp Tyr Tyr Thr Ala Glu Tyr Phe Leu Lys Glu Leu His
    290                 295                 300
Phe Glu Leu Cys Asp Ala Val Ser Gly Glu Lys Ile Pro Ala Ala Thr
305                 310                 315                 320
Gln Asn Thr Asn Thr Asn Ser Asn Arg Tyr Glu Gly Lys Val Ile Asp
                325                 330                 335
Ser Ala Pro Leu Leu Pro Lys Met Asp Phe Lys Ser Ser Pro Phe Arg
            340                 345                 350
Met Tyr Lys Val Gly Thr Glu Phe Leu Val Tyr Asp His Asn Gln Tyr
    355                 360                 365
Trp Tyr Lys Thr Tyr Ile Asp Asp Lys Leu Tyr Tyr Met Tyr Lys Ser
    370                 375                 380
Phe Cys Asp Val Val Ala Lys Lys Asp Ala Lys Gly Arg Ile Lys Val
385                 390                 395                 400
Arg Ile Lys Ser Ala Lys Asp Leu Arg Ile Pro Val Trp Asn Asn Ile
                405                 410                 415
Lys Leu Asn Ser Gly Lys Ile Lys Trp Tyr Ala Pro Asn Val Lys Leu
            420                 425                 430
Ala Trp Tyr Asn Tyr Arg Arg Gly Tyr Leu Glu Leu Trp Tyr Pro Asn
        435                 440                 445
Asp Gly Trp Tyr Tyr Thr Ala Glu Tyr Phe Leu Lys
    450                 455                 460

<210>  52
<211>  1383
<212>  DNA
<213>  artificial sequence

<220>
<223>  HEAD_CBD500-500:

<400>  52
atgagaggat cgcatcacca tcaccatcac ggatccatgg cattaacaga ggcatggcta      60
attgaaaaag caaatcgcaa attgaatgct gggggaatgt ataaaattac atcggataaa     120
acacgaaatg taattaaaaa aatggcaaaa gaaggtattt atctttgtgt tgcgcaaggt     180
taccgctcaa cagcggaaca aaatgcgcta tatgcacaag ggagaaccaa acctggagca     240
attgttacta atgccaaggg cgggcaatct aatcacaact acggggtagc tgttgacttg     300
tgcttgtata caaatgacgg aaaagatgtt atttgggagt caacaacttc ccggtggaaa     360
aaggttgttg ctgctatgaa agcagaaggg tttaaatggg gcggagactg gaaaagtttt     420
aaagactatc cgcattttga actatgtgat gctgtaagtg gtgagaaaat ccctgctgca     480
acacaaaaca ctaatacaaa ttcaaatcgt tacgagggta aagtcattga tagcgcacca     540
ctgctaccga aaatggactt taaatcatca ccattccgca tgtataaggt aggaactgag     600
ttcttagtat atgatcataa tcaatattgg tacaagacat acattgatga caaactttac     660
tacatgtata aaagcttttg cgatgttgta gctaaaaaag acgcaaaagg tcgcatcaaa     720
gttcgaatta aaagcgcgaa agacttgcgt attccagtct ggaataacat aaaattgaat     780
tctgggaaaa ttaaatggta tgcacccaat gtaaaactag cgtggtacaa ctatcgaaga     840
ggatatttag agctatggta tccgaacgac ggctggtatt acacagcaga atacttctta     900
aaagagctcc attttgaact atgtgatgct gtaagtggtg agaaaatccc tgctgcaaca     960
caaaacacta atacaaattc aaatcgttac gagggtaaag tcattgatag cgcaccactg    1020
ctaccgaaaa tggactttaa atcatcacca ttccgcatgt ataaggtagg aactgagttc    1080
ttagtatatg atcataatca atattggtac aagacataca ttgatgacaa actttactac    1140
atgtataaaa gcttttgcga tgttgtagct aaaaaagacg caaaaggtcg catcaaagtt    1200
cgaattaaaa gcgcgaaaga cttgcgtatt ccagtctgga ataacataaa attgaattct    1260
gggaaaatta aatggtatgc acccaatgta aaactagcgt ggtacaacta tcgaagagga    1320
tatttagagc tatggtatcc gaacgacggc tggtattaca cagcagaata cttcttaaaa    1380
taa                                                                  1383

<210>  53
<211>  10
<212>  PRT
<213>  artificial sequence

<220>
<223>  His Tag:

<400>  53
Met Arg Gly Ser His His His His His His
1               5                   10


<210>  54
<211>  30
<212>  DNA
<213>  artificial sequence

<220>
<223>  His Tag:

<400>  54
atgagaggat cgcatcacca tcaccatcac                                        30


<210>  55
<211>  228
<212>  PRT
<213>  artificial sequence

<220>
<223>  GFP

<400>  55
Met Ser Lys Gly Glu Glu Leu Phe Thr Gly Val Val Pro Ile Leu Val
1               5                   10                  15
Glu Leu Asp Gly Asp Val Asn Gly His Lys Phe Ser Val Ser Gly Glu
            20                  25                  30
Gly Glu Gly Asp Ala Thr Tyr Gly Lys Leu Thr Leu Lys Phe Ile Cys
        35                  40                  45
Thr Thr Gly Lys Leu Pro Val Pro Trp Pro Thr Leu Val Thr Thr Phe
    50                  55                  60
Ala Tyr Gly Leu Gln Cys Phe Ala Arg Tyr Pro Asp His Met Lys Arg
65                  70                  75                  80
His Asp Phe Phe Lys Ser Ala Met Pro Glu Gly Tyr Val Gln Glu Arg
                85                  90                  95
Thr Ile Phe Phe Lys Asp Asp Gly Asn Tyr Lys Thr Arg Ala Glu Val
            100                 105                 110
Lys Phe Glu Gly Asp Thr Leu Val Asn Arg Ile Glu Leu Lys Gly Ile
        115                 120                 125
Asp Phe Lys Glu Asp Gly Asn Ile Leu Gly His Lys Leu Glu Tyr Asn
    130                 135                 140
Tyr Asn Ser His Asn Val Tyr Ile Met Ala Asp Lys Gln Lys Asn Gly
145                 150                 155                 160
Ile Lys Val Asn Phe Lys Ile Arg His Asn Ile Glu Asp Gly Ser Val
                165                 170                 175
Gln Leu Ala Asp His Tyr Gln Gln Asn Thr Pro Ile Gly Asp Gly Pro
            180                 185                 190
Val Leu Leu Pro Asp Asn His Tyr Leu Ser Thr Gln Ser Ala Leu Ser
        195                 200                 205
Lys Asp Pro Asn Glu Lys Arg Asp His Met Val Leu Leu Glu Phe Val
    210                 215                 220
Thr Ala Ala Gly
225


<210>  56

<211>  684
<212>  DNA
<213>  artificial sequence

<220>
<223>  GFP

<400>  56
atgagtaaag gagaagaact tttcactgga gttgtcccaa ttcttgttga attagatggt      60
gatgttaatg ggcacaaatt ttctgtcagt ggagagggtg aaggtgatgc aacatacgga     120
aaacttaccc ttaaatttat ttgcactact ggaaaactac ctgttccatg gccaacactt     180
gtcactactt tcgcgtatgg tcttcaatgc tttgcgagat acccagatca tatgaaacgg     240
catgactttt tcaagagtgc catgcccgaa ggttatgtac aggaaagaac tatatttttc     300
aaagatgacg ggaactacaa gacacgtgct gaagtcaagt ttgaaggtga tacccttgtt     360
aatagaatcg agttaaaagg tattgatttt aaagaagatg gaaacattct tggacacaaa     420
ttggaataca actataactc acacaatgta tacatcatgg cagacaaaca aaagaatgga     480
atcaaagtta acttcaaaat tagacacaac attgaagatg gaagcgttca actagcagac     540
cattatcaac aaaatactcc aattggcgat ggccctgtcc ttttaccaga caaccattac     600
ctgtccacac aatctgccct ttcgaaagat cccaacgaaa agagagacca catggtcctt     660
cttgagtttg taacagctgc tggg                                            684


<210>  57
<211>  615
<212>  DNA
<213>  artificial sequence

<220>
<223>  CBD118:

<400>  57
attacacatg gcatggatga actatacaaa gagctcgaca aaggcaagaa atttgtggca      60
aaggcaaaat ctttaggttt tgagtggggt ggtgattggt ctggatttgt agacaatccg     120
caccttcaat ttaattataa aggctatggg actgatactt ttggaaaagg agctagtact     180
agtaattcat ctaaaccgag cgcagacaca aacacaaaca gtctaggatt agtagattat     240
atgaatttaa ataaactaga ttcaagcttt gcgaatcgca aaaaactagc gacaagttac     300
ggaattaaaa attacagtgg aacagcaacg cagaacacaa cattattagc gaagttaaaa     360
gcaggaaaac cacacacacc agcaagcaaa aacacatact acacagaaaa tccgcgaaaa     420
gttaaaacac tagtacaatg tgatctatac aaatcagtag actttacaac aaaaaaccaa     480
acaggtggaa catttccgcc aggcacagtc ttcacgattt cagggatggg gaaaacgaaa     540
ggcggaacac ctcgcttgaa gacgaagagc ggttactatc tcactgctaa cacgaagttt     600
gttaaaaaga tttaa                                                      615


<210>  58
<211>  510
<212>  DNA
<213>  artificial sequence

<220>
<223>  CBD500:

<400>  58
attacacatg gcatggatga actatacaaa gagctccatt ttgaactatg tgatgctgta      60
agtggtgaga aaatccctgc tgcaacacaa aacactaata caaattcaaa tcgttacgag     120
ggtaaagtca ttgatagcgc accactgcta ccgaaaatgg actttaaatc atcaccattc     180
cgcatgtata aggtaggaac tgagttctta gtatatgatc ataatcaata ttggtacaag     240
acatacattg atgacaaact ttactacatg tataaaagct tttgcgatgt tgtagctaaa     300
aaagacgcaa aaggtcgcat caaagttcga attaaaagcg cgaaagactt gcgtattcca     360
gtctggaata acataaaatt gaattctggg aaaattaaat ggtatgcacc caatgtaaaa     420
ctagcgtggt acaactatcg aagaggatat ttagagctat ggtatccgaa cgacggctgg     480
tattacacag cagaatactt cttaaaataa                                      510

<210>  59
<211>  525
<212>  DNA
<213>  artificial sequence

<220>
<223>  CBDP35:

<400>  59
attacacatg gcatggatga actatacaaa gagctcccac attttgaagc ttgtgactgg      60
tatcgcgggg aacgcaagta taaagtggac acatctgaat ggaaaaagaa agagaatatc     120
aatatcgtta ttaaagatgt tggttacttc caagacaaac ctcaattctt aaactccaaa     180
tcggttcgtc agtggaagca tggcacgaaa gtgaagctta ctaaacataa ctcacattgg     240
tacactggtg tggtcaagga tggtaacaaa tcagtcaggg gatatattta tcattcgatg     300
gctaaggtca caagcaagaa tagcgacggt tcggttaacg caacgattaa cgcccacgca     360
ttttgttggg acaataaaaa acttaatggt ggcgacttta tcaacttgaa gcgtggtttt     420
aaaggtatca cccatcccgc tagtgacggt ttctatccac tgtatttcgc ttctaggaaa     480
aaaactttct acattccgcg ttacatgttt gacatcaaga aataa                     525


<210>  60
<211>  409
<212>  PRT
<213>  artificial sequence

<220>
<223>  HGFP_CBD500:

<400>  60
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Ile Thr His Gly Met Asp Glu Leu Tyr Lys Glu Leu His Phe Glu Leu
                245                 250                 255
Cys Asp Ala Val Ser Gly Glu Lys Ile Pro Ala Ala Thr Gln Asn Thr

260                 265                 270
Asn Thr Asn Ser Asn Arg Tyr Glu Gly Lys Val Ile Asp Ser Ala Pro
        275                 280                 285
Leu Leu Pro Lys Met Asp Phe Lys Ser Ser Pro Phe Arg Met Tyr Lys
    290                 295                 300
Val Gly Thr Glu Phe Leu Val Tyr Asp His Asn Gln Tyr Trp Tyr Lys
305                 310                 315                 320
Thr Tyr Ile Asp Asp Lys Leu Tyr Tyr Met Tyr Lys Ser Phe Cys Asp
                325                 330                 335
Val Val Ala Lys Lys Asp Ala Lys Gly Arg Ile Lys Val Arg Ile Lys
            340                 345                 350
Ser Ala Lys Asp Leu Arg Ile Pro Val Trp Asn Asn Ile Lys Leu Asn
        355                 360                 365
Ser Gly Lys Ile Lys Trp Tyr Ala Pro Asn Val Lys Leu Ala Trp Tyr
    370                 375                 380
Asn Tyr Arg Arg Gly Tyr Leu Glu Leu Trp Tyr Pro Asn Asp Gly Trp
385                 390                 395                 400
Tyr Tyr Thr Ala Glu Tyr Phe Leu Lys
                405

<210>  61
<211>  6
<212>  PRT
<213>  artificial sequence

<220>
<223>  PlyPSA linker

<400>  61
Ala Ala Lys Asn Pro Asn
1               5

<210>  62
<211>  18
<212>  DNA
<213>  artificial sequence

<220>
<223>  PlyPSA linker

<400>  62
gccgcaaaaa atccaaac                                                    18

<210>  63
<211>  9
<212>  PRT
<213>  artificial sequence

<220>
<223>  Polypeptidelinker

<400>  63

Ala Gly Ala Gly Ala Gly Ala Gly Ser
1               5

<210>  64
<211>  11
<212>  PRT

<213>  artificial sequence

<220>
<223>  Polypeptidelinker

<400>  64
Ala Gly Ala Gly Ala Gly Ala Gly Ser Glu Leu
1               5                   10


<210>  65
<211>  14
<212>  PRT
<213>  artificial sequence

<220>
<223>  Polypeptidelinker

<400>  65

Thr Pro Thr Pro Pro Asn Pro Gly Pro Lys Asn Phe Thr Thr
1               5                   10


<210>  66
<211>  9
<212>  PRT
<213>  artificial sequence

<220>
<223>  Polypeptidelinker

<400>  66
Ala Gly Ala Gly Ala Gly Ala Gly Leu
1               5


<210>  67
<211>  1341
<212>  DNA
<213>  artificial sequence

<220>
<223>  HGFP_L_CBD118: (mit PlyPSA linker)

<400>  67
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180
actactggaa aactacctgt tccatggcca acacttgtca ctactttcgc gtatggtctt     240
caatgctttg cgagataccc agatcatatg aaacggcatg actttttcaa gagtgccatg     300
cccgaaggtt atgtacagga aagaactata tttttcaaag atgacgggaa ctacaagaca     360
cgtgctgaag tcaagtttga aggtgatacc cttgttaata gaatcgagtt aaaaggtatt     420
gattttaaag aagatggaaa cattcttgga cacaaattgg aatacaacta taactcacac     480
aatgtataca tcatggcaga caaacaaaag aatggaatca aagttaactt caaaattaga     540
cacaacattg aagatggaag cgttcaacta gcagaccatt atcaacaaaa tactccaatt     600
ggcgatggcc ctgtcctttt accagacaac cattacctgt ccacacaatc tgccctttcg     660
aaagatccca acgaaaagag agaccacatg gtccttcttg agtttgtaac agctgctggg     720
actggtaaaa cagtagccgc aaaaaatcca aaccgccatt ctgacaaagg caagaaattt     780
gtggcaaagg caaaatcttt aggttttgag tggggtggtg attggtctgg atttgtagac     840
aatccgcacc ttcaatttaa ttataaaggc tatggactgg atacttttgg aaaaggagct     900
agtactagta attcatctaa accgagcgca gacacaaaca caaacagtct aggattagta     960
gattatatga atttaaataa actagattca agctttgcga atcgcaaaaa actagcgaca    1020

agttacggaa ttaaaaatta cagtggaaca gcaacgcaga acacaacatt attagcgaag   1080
ttaaaagcag gaaaaccaca cacaccagca agcaaaaaca catactacac agaaaatccg   1140
cgaaaagtta aaacactagt acaatgtgat ctatacaaat cagtagactt tacaacaaaa   1200
aaccaaacag gtggaacatt tccgccaggc acagtcttca cgatttcagg gatggggaaa   1260
acgaaaggcg gaacacctcg cttgaagacg aagagcggtt actatctcac tgctaacacg   1320
aagtttgtta aaaagattta a                                             1341

<210>  68
<211>  446
<212>  PRT
<213>  artificial sequence

<220>
<223>  HGFP_L_CBD118: (mit PlyPSA linker)

<400>  68
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Thr Gly Lys Thr Val Ala Ala Lys Asn Pro Asn Arg His Ser Asp Lys
                245                 250                 255
Gly Lys Lys Phe Val Ala Lys Ala Lys Ser Leu Gly Phe Glu Trp Gly
            260                 265                 270
Gly Asp Trp Ser Gly Phe Val Asp Asn Pro His Leu Gln Phe Asn Tyr
        275                 280                 285
Lys Gly Tyr Gly Thr Asp Thr Phe Gly Lys Gly Ala Ser Thr Ser Asn
    290                 295                 300
Ser Ser Lys Pro Ser Ala Asp Thr Asn Thr Asn Ser Leu Gly Leu Val
305                 310                 315                 320
Asp Tyr Met Asn Leu Asn Lys Leu Asp Ser Ser Phe Ala Asn Arg Lys
                325                 330                 335
Lys Leu Ala Thr Ser Tyr Gly Ile Lys Asn Tyr Ser Gly Thr Ala Thr
            340                 345                 350
Gln Asn Thr Thr Leu Leu Ala Lys Leu Lys Ala Gly Lys Pro His Thr
        355                 360                 365

Pro Ala Ser Lys Asn Thr Tyr Tyr Thr Glu Asn Pro Arg Lys Val Lys
    370                 375                 380
Thr Leu Val Gln Cys Asp Leu Tyr Lys Ser Val Asp Phe Thr Thr Lys
385                 390                 395                 400
Asn Gln Thr Gly Gly Thr Phe Pro Pro Gly Thr Val Phe Thr Ile Ser
                405                 410                 415
Gly Met Gly Lys Thr Lys Gly Gly Thr Pro Arg Leu Lys Thr Lys Ser
            420                 425                 430
Gly Tyr Tyr Leu Thr Ala Asn Thr Lys Phe Val Lys Lys Ile
        435                 440                 445

<210>  69
<211>  1251
<212>  DNA
<213>  artificial sequence

<220>
<223>  HGFP_ L_CBDP35: (mit PlyPSA linker)

<400>  69
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180
actactggaa aactacctgt tccatggcca acacttgtca ctactttcgc gtatggtctt     240
caatgctttg cgagataccc agatcatatg aaacggcatg actttttcaa gagtgccatg     300
cccgaaggtt atgtacagga aagaactata tttttcaaag atgacgggaa ctacaagaca     360
cgtgctgaag tcaagtttga aggtgatacc cttgttaata gaatcgagtt aaaaggtatt     420
gattttaaag aagatggaaa cattcttgga cacaaattgg aatacaacta taactcacac     480
aatgtataca tcatggcaga caaacaaaag aatggaatca aagttaactt caaaattaga     540
cacaacattg aagatggaag cgttcaacta gcagaccatt atcaacaaaa tactccaatt     600
ggcgatggcc ctgtcctttt accagacaac cattacctgt ccacacaatc tgccctttcg     660
aaagatccca acgaaaagag agaccacatg gtccttcttg agtttgtaac agctgctggg     720
actggtaaaa cagtagccgc aaaaaatcca aaccgccatt ctccacattt tgaagcttgt     780
gactggtatc gcggggaacg caagtataaa gtggacacat ctgaatggaa aaagaaagag     840
aatatcaata tcgttattaa agatgttggt tacttccaag acaaacctca attcttaaac     900
tccaaatcgg ttcgtcagtg gaagcatggc acgaaagtga agcttactaa acataactca     960
cattggtaca ctggtgtggt caaggatggt aacaaatcag tcaggggata tatttatcat    1020
tcgatggcta aggtcacaag caagaatagc gacggttcgg ttaacgcaac gattaacgcc    1080
cacgcatttt gttgggacaa taaaaaactt aatggtggcg actttatcaa cttgaagcgt    1140
ggttttaaag gtatcaccca tcccgctagt gacggtttct atccactgta tttcgcttct    1200
aggaaaaaaa ctttctacat tccgcgttac atgtttgaca tcaagaaata a             1251

<210>  70
<211>  416
<212>  PRT
<213>  artificial sequence

<220>
<223>  HGFP_ L_CBDP35: (mit PlyPSA linker)

<400>  70
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80

Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Thr Gly Lys Thr Val Ala Ala Lys Asn Pro Asn Arg His Ser Pro His
                245                 250                 255
Phe Glu Ala Cys Asp Trp Tyr Arg Gly Glu Arg Lys Tyr Lys Val Asp
            260                 265                 270
Thr Ser Glu Trp Lys Lys Lys Glu Asn Ile Asn Ile Val Ile Lys Asp
        275                 280                 285
Val Gly Tyr Phe Gln Asp Lys Pro Gln Phe Leu Asn Ser Lys Ser Val
    290                 295                 300
Arg Gln Trp Lys His Gly Thr Lys Val Lys Leu Thr Lys His Asn Ser
305                 310                 315                 320
His Trp Tyr Thr Gly Val Val Lys Asp Gly Asn Lys Ser Val Arg Gly
                325                 330                 335
Tyr Ile Tyr His Ser Met Ala Lys Val Thr Ser Lys Asn Ser Asp Gly
            340                 345                 350
Ser Val Asn Ala Thr Ile Asn Ala His Ala Phe Cys Trp Asp Asn Lys
        355                 360                 365
Lys Leu Asn Gly Gly Asp Phe Ile Asn Leu Lys Arg Gly Phe Lys Gly
    370                 375                 380
Ile Thr His Pro Ala Ser Asp Gly Phe Tyr Pro Leu Tyr Phe Ala Ser
385                 390                 395                 400
Arg Lys Lys Thr Phe Tyr Ile Pro Arg Tyr Met Phe Asp Ile Lys Lys
                405                 410                 415


<210>  71
<211>  605
<212>  PRT
<213>  artificial sequence

<220>
<223>  HGFP_ L_CBD500-118: (mit PlyPSA linker)

<400>  71
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80

Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Thr Gly Lys Thr Val Ala Ala Lys Asn Pro Asn Arg His Ser His Phe
                245                 250                 255
Glu Leu Cys Asp Ala Val Ser Gly Glu Lys Ile Pro Ala Ala Thr Gln
            260                 265                 270
Asn Thr Asn Thr Asn Ser Asn Arg Tyr Glu Gly Lys Val Ile Asp Ser
        275                 280                 285
Ala Pro Leu Leu Pro Lys Met Asp Phe Lys Ser Ser Pro Phe Arg Met
    290                 295                 300
Tyr Lys Val Gly Thr Glu Phe Leu Val Tyr Asp His Asn Gln Tyr Trp
305                 310                 315                 320
Tyr Lys Thr Tyr Ile Asp Asp Lys Leu Tyr Tyr Met Tyr Lys Ser Phe
                325                 330                 335
Cys Asp Val Val Ala Lys Lys Asp Ala Lys Gly Arg Ile Lys Val Arg
            340                 345                 350
Ile Lys Ser Ala Lys Asp Leu Arg Ile Pro Val Trp Asn Asn Ile Lys
        355                 360                 365
Leu Asn Ser Gly Lys Ile Lys Trp Tyr Ala Pro Asn Val Lys Leu Ala
    370                 375                 380
Trp Tyr Asn Tyr Arg Arg Gly Tyr Leu Glu Leu Trp Tyr Pro Asn Asp
385                 390                 395                 400
Gly Trp Tyr Tyr Thr Ala Glu Tyr Phe Leu Lys Gln Phe Asp Lys Gly
                405                 410                 415
Lys Lys Phe Val Ala Lys Ala Lys Ser Leu Gly Phe Glu Trp Gly Gly
            420                 425                 430
Asp Trp Ser Gly Phe Val Asp Asn Pro His Leu Gln Phe Asn Tyr Lys
        435                 440                 445
Gly Tyr Gly Thr Asp Thr Phe Gly Lys Gly Ala Ser Thr Ser Asn Ser
    450                 455                 460
Ser Lys Pro Ser Ala Asp Thr Asn Thr Asn Ser Leu Gly Leu Val Asp
465                 470                 475                 480
Tyr Met Asn Leu Asn Lys Leu Asp Ser Ser Phe Ala Asn Arg Lys Lys
                485                 490                 495
Leu Ala Thr Ser Tyr Gly Ile Lys Asn Tyr Ser Gly Thr Ala Thr Gln
            500                 505                 510
Asn Thr Thr Leu Leu Ala Lys Leu Lys Ala Gly Lys Pro His Thr Pro
        515                 520                 525
Ala Ser Lys Asn Thr Tyr Tyr Thr Glu Asn Pro Arg Lys Val Lys Thr
    530                 535                 540
Leu Val Gln Cys Asp Leu Tyr Lys Ser Val Asp Phe Thr Thr Lys Asn
545                 550                 555                 560
Gln Thr Gly Gly Thr Phe Pro Pro Gly Thr Val Phe Thr Ile Ser Gly
                565                 570                 575
Met Gly Lys Thr Lys Gly Gly Thr Pro Arg Leu Lys Thr Lys Ser Gly

580                 585                 590
Tyr Tyr Leu Thr Ala Asn Thr Lys Phe Val Lys Lys Ile
        595                 600                 605

<210>  72
<211>  1818
<212>  DNA
<213>  artificial sequence

<220>
<223>  HGFP_ L_CBD500-118: (mit PlyPSA linker)

<400>  72
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180
actactggaa aactacctgt tccatggcca acacttgtca ctactttcgc gtatggtctt     240
caatgctttg cgagataccc agatcatatg aaacggcatg actttttcaa gagtgccatg     300
cccgaaggtt atgtacagga aagaactata tttttcaaag atgacgggaa ctacaagaca     360
cgtgctgaag tcaagtttga aggtgatacc cttgttaata gaatcgagtt aaaaggtatt     420
gattttaaag aagatggaaa cattcttgga cacaaattgg aatacaacta taactcacac     480
aatgtataca tcatggcaga caaacaaaag aatggaatca aagttaactt caaaattaga     540
cacaacattg aagatggaag cgttcaacta gcagaccatt atcaacaaaa tactccaatt     600
ggcgatggcc ctgtcctttt accagacaac cattacctgt ccacacaatc tgccctttcg     660
aaagatccca acgaaaagag agaccacatg gtccttcttg agtttgtaac agctgctggg     720
actggtaaaa cagtagccgc aaaaaatcca aaccgccatt ctcattttga actatgtgat     780
gctgtaagtg gtgagaaaat ccctgctgca acacaaaaca ctaatacaaa ttcaaatcgt     840
tacgagggta aagtcattga tagcgcacca ctgctaccga aaatggactt taaatcatca     900
ccattccgca tgtataaggt aggaactgag ttcttagtat atgatcataa tcaatattgg     960
tacaagacat acattgatga caaactttac tacatgtata aaagcttttg cgatgttgta    1020
gctaaaaaag acgcaaaagg tcgcatcaaa gttcgaatta aaagcgcgaa agacttgcgt    1080
attccagtct ggaataacat aaaattgaat tctgggaaaa ttaaatggta tgcacccaat    1140
gtaaaactag cgtggtacaa ctatcgaaga ggatatttag agctatggta tccgaacgac    1200
ggctggtatt acacagcaga atacttctta aaacaattcg acaaaggcaa gaaatttgtg    1260
gcaaaggcaa aatctttagg ttttgagtgg ggtggtgatt ggtctggatt tgtagacaat    1320
ccgcaccttc aatttaatta taaaggctat gggactgata cttttggaaa aggagctagt    1380
actagtaatt catctaaacc gagcgcagac acaaacacaa acagtctagg attagtagat    1440
tatatgaatt taaataaact agattcaagc tttgcgaatc gcaaaaaact agcgacaagt    1500
tacggaatta aaaattacag tggaacagca acgcagaaca caacattatt agcgaagtta    1560
aaagcaggaa aaccacacac accagcaagc aaaaacacat actacacaga aaatccgcga    1620
aaagttaaaa cactagtaca atgtgatcta tacaaatcag tagactttac aacaaaaaac    1680
caaacaggtg gaacatttcc gccaggcaca gtcttcacga tttcagggat ggggaaaacg    1740
aaaggcggaa cacctcgctt gaagacgaag agcggttact atctcactgc taacacgaag    1800
tttgttaaaa agatttaa                                                  1818

<210>  73
<211>  352
<212>  PRT
<213>  artificial sequence

<220>
<223>  CBD500-118:

<400>  73
Met His Phe Glu Leu Cys Asp Ala Val Ser Gly Glu Lys Ile Pro Ala
1               5                   10                  15
Ala Thr Gln Asn Thr Asn Thr Asn Ser Asn Arg Tyr Glu Gly Lys Val
            20                  25                  30
Ile Asp Ser Ala Pro Leu Leu Pro Lys Met Asp Phe Lys Ser Ser Pro
        35                  40                  45
Phe Arg Met Tyr Lys Val Gly Thr Glu Phe Leu Val Tyr Asp His Asn

50                  55                  60
Gln Tyr Trp Tyr Lys Thr Tyr Ile Asp Asp Lys Leu Tyr Tyr Met Tyr
65                  70                  75                  80
Lys Ser Phe Cys Asp Val Val Ala Lys Lys Asp Ala Lys Gly Arg Ile
                85                  90                  95
Lys Val Arg Ile Lys Ser Ala Lys Asp Leu Arg Ile Pro Val Trp Asn
            100                 105                 110
Asn Ile Lys Leu Asn Ser Gly Lys Ile Lys Trp Tyr Ala Pro Asn Val
        115                 120                 125
Lys Leu Ala Trp Tyr Asn Tyr Arg Arg Gly Tyr Leu Glu Leu Trp Tyr
    130                 135                 140
Pro Asn Asp Gly Trp Tyr Tyr Thr Ala Glu Tyr Phe Leu Lys Gln Phe
145                 150                 155                 160
Asp Lys Gly Lys Lys Phe Val Ala Lys Ala Lys Ser Leu Gly Phe Glu
                165                 170                 175
Trp Gly Gly Asp Trp Ser Gly Phe Val Asp Asn Pro His Leu Gln Phe
            180                 185                 190
Asn Tyr Lys Gly Tyr Gly Thr Asp Thr Phe Gly Lys Gly Ala Ser Thr
        195                 200                 205
Ser Asn Ser Ser Lys Pro Ser Ala Asp Thr Asn Thr Asn Ser Leu Gly
    210                 215                 220
Leu Val Asp Tyr Met Asn Leu Asn Lys Leu Asp Ser Ser Phe Ala Asn
225                 230                 235                 240
Arg Lys Lys Leu Ala Thr Ser Tyr Gly Ile Lys Asn Tyr Ser Gly Thr
                245                 250                 255
Ala Thr Gln Asn Thr Thr Leu Leu Ala Lys Leu Lys Ala Gly Lys Pro
            260                 265                 270
His Thr Pro Ala Ser Lys Asn Thr Tyr Tyr Thr Glu Asn Pro Arg Lys
        275                 280                 285
Val Lys Thr Leu Val Gln Cys Asp Leu Tyr Lys Ser Val Asp Phe Thr
    290                 295                 300
Thr Lys Asn Gln Thr Gly Gly Thr Phe Pro Pro Gly Thr Val Phe Thr
305                 310                 315                 320
Ile Ser Gly Met Gly Lys Thr Lys Gly Gly Thr Pro Arg Leu Lys Thr
                325                 330                 335
Lys Ser Gly Tyr Tyr Leu Thr Ala Asn Thr Lys Phe Val Lys Lys Ile
            340                 345                 350

<210>  74
<211>  1059
<212>  DNA
<213>  artificial sequence

<220>
<223>  CBD500-118:

<400>  74
atgcattttg aactatgtga tgctgtaagt ggtgagaaaa tccctgctgc aacacaaaac      60
actaatacaa attcaaatcg ttacgagggt aaagtcattg atagcgcacc actgctaccg     120
aaaatggact ttaaatcatc accattccgc atgtataagg taggaactga gttcttagta     180
tatgatcata atcaatattg gtacaagaca tacattgatg acaaacttta ctacatgtat     240
aaaagctttt gcgatgttgt agctaaaaaa gacgcaaaag gtcgcatcaa agttcgaatt     300
aaaagcgcga aagacttgcg tattccagtc tggaataaca taaaattgaa ttctgggaaa     360
attaaatggt atgcacccaa tgtaaaacta gcgtggtaca actatcgaag aggatattta     420
gagctatggt atccgaacga cggctggtat tacacagcag aatacttctt aaaacaattc     480
gacaaaggca agaaatttgt ggcaaaggca aaatctttag gttttgagtg gggtggtgat     540
tggtctggat ttgtagacaa tccgcacctt caatttaatt ataaaggcta tgggactgat     600
acttttggaa aaggagctag tactagtaat tcatctaaac cgagcgcaga cacaaacaca     660
aacagtctag gattagtaga ttatatgaat ttaaataaac tagattcaag ctttgcgaat     720
cgcaaaaaac tagcgacaag ttacggaatt aaaaattaca gtggaacagc aacgcagaac     780
acaacattat tagcgaagtt aaaagcagga aaaccacaca caccagcaag caaaaacaca     840
tactacacag aaaatccgcg aaaagttaaa acactagtac aatgtgatct atacaaatca     900

gtagacttta caacaaaaaa ccaaacaggt ggaacatttc cgccaggcac agtcttcacg 960
atttcaggga tggggaaaac gaaaggcgga acacctcgct tgaagacgaa gagcggttac 1020
tatctcactg ctaacacgaa gtttgttaaa aagatttaa 1059

<210> 75
<211> 605
<212> PRT
<213> artificial sequence

<220>
<223> HGFP_ L_CBD118-500: (mit PlyPSA linker)

<400> 75
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Thr Gly Lys Thr Val Ala Ala Lys Asn Pro Asn Arg His Ser Asp Lys
                245                 250                 255
Gly Lys Lys Phe Val Ala Lys Ala Lys Ser Leu Gly Phe Glu Trp Gly
            260                 265                 270
Gly Asp Trp Ser Gly Phe Val Asp Asn Pro His Leu Gln Phe Asn Tyr
        275                 280                 285
Lys Gly Tyr Gly Thr Asp Thr Phe Gly Lys Gly Ala Ser Thr Ser Asn
    290                 295                 300
Ser Ser Lys Pro Ser Ala Asp Thr Asn Thr Asn Ser Leu Gly Leu Val
305                 310                 315                 320
Asp Tyr Met Asn Leu Asn Lys Leu Asp Ser Ser Phe Ala Asn Arg Lys
                325                 330                 335
Lys Leu Ala Thr Ser Tyr Gly Ile Lys Asn Tyr Ser Gly Thr Ala Thr
            340                 345                 350
Gln Asn Thr Thr Leu Leu Ala Lys Leu Lys Ala Gly Lys Pro His Thr
        355                 360                 365
Pro Ala Ser Lys Asn Thr Tyr Tyr Thr Glu Asn Pro Arg Lys Val Lys
    370                 375                 380
Thr Leu Val Gln Cys Asp Leu Tyr Lys Ser Val Asp Phe Thr Thr Lys

385                 390                 395                 400
Asn Gln Thr Gly Gly Thr Phe Pro Pro Gly Thr Val Phe Thr Ile Ser
                405                 410                 415
Gly Met Gly Lys Thr Lys Gly Gly Thr Pro Arg Leu Lys Thr Lys Ser
            420                 425                 430
Gly Tyr Tyr Leu Thr Ala Asn Thr Lys Phe Val Lys Lys Ile Glu Leu
        435                 440                 445
His Phe Glu Leu Cys Asp Ala Val Ser Gly Glu Lys Ile Pro Ala Ala
    450                 455                 460
Thr Gln Asn Thr Asn Thr Asn Ser Asn Arg Tyr Glu Gly Lys Val Ile
465                 470                 475                 480
Asp Ser Ala Pro Leu Leu Pro Lys Met Asp Phe Lys Ser Ser Pro Phe
                485                 490                 495
Arg Met Tyr Lys Val Gly Thr Glu Phe Leu Val Tyr Asp His Asn Gln
            500                 505                 510
Tyr Trp Tyr Lys Thr Tyr Ile Asp Asp Lys Leu Tyr Tyr Met Tyr Lys
        515                 520                 525
Ser Phe Cys Asp Val Val Ala Lys Lys Asp Ala Lys Gly Arg Ile Lys
    530                 535                 540
Val Arg Ile Lys Ser Ala Lys Asp Leu Arg Ile Pro Val Trp Asn Asn
545                 550                 555                 560
Ile Lys Leu Asn Ser Gly Lys Ile Lys Trp Tyr Ala Pro Asn Val Lys
                565                 570                 575
Leu Ala Trp Tyr Asn Tyr Arg Arg Gly Tyr Leu Glu Leu Trp Tyr Pro
            580                 585                 590
Asn Asp Gly Trp Tyr Tyr Thr Ala Glu Tyr Phe Leu Lys
        595                 600                 605


<210>  76
<211>  1818
<212>  DNA
<213>  artificial sequence

<220>
<223>  HGFP_ L_CBD118-500: (mit PlyPSA linker)

<400>  76
   atgagaggat cgcatcacca tcaccatcac ggatccatga gtaaaggaga agaacttttc      60
   actggagttg tcccaattct tgttgaatta gatggtgatg ttaatgggca caaattttct     120
   gtcagtggag agggtgaagg tgatgcaaca tacggaaaac ttacccttaa atttatttgc     180
   actactggaa aactacctgt tccatggcca acacttgtca ctactttcgc gtatggtctt     240
   caatgctttg cgagataccc agatcatatg aaacggcatg actttttcaa gagtgccatg     300
   cccgaaggtt atgtacagga aagaactata tttttcaaag atgacgggaa ctacaagaca     360
   cgtgctgaag tcaagtttga aggtgatacc cttgttaata gaatcgagtt aaaaggtatt     420
   gattttaaag aagatggaaa cattcttgga cacaaattgg aatacaacta taactcacac     480
   aatgtataca tcatggcaga caaacaaaag aatggaatca aagttaactt caaaattaga     540
   cacaacattg aagatggaag cgttcaacta gcagaccatt atcaacaaaa tactccaatt     600
   ggcgatggcc ctgtcctttt accagacaac cattacctgt ccacacaatc tgccctttcg     660
   aaagatccca acgaaaagag agaccacatg gtccttcttg agtttgtaac agctgctggg     720
   actggtaaaa cagtagccgc aaaaaatcca aaccgccatt ctgacaaagg caagaaattt     780
   gtggcaaagg caaaatcttt aggttttgag tggggtggtg attggtctgg atttgtagac     840
   aatccgcacc ttcaatttaa ttataaaggc tatgggactg atacttttgg aaaaggagct     900
   agtactagta attcatctaa accgagcgca gacacaaaca caaacagtct aggattagta     960
   gattatatga atttaaataa actagattca agctttgcga atcgcaaaaa actagcgaca    1020
   agttacggaa ttaaaaatta cagtggaaca gcaacgcaga acacaacatt attagcgaag    1080
   ttaaaagcag gaaaaccaca cacaccagca agcaaaaaca catactacac agaaaatccg    1140
   cgaaaagtta aaacactagt acaatgtgat ctatacaaat cagtagactt tacaacaaaa    1200
   aaccaaacag gtggaacatt tccgccaggc acagtcttca cgatttcagg gatgggaaaa    1260
   acgaaaggcg gaacacctcg cttgaagacg aagagcggtt actatctcac tgctaacacg    1320
   aagtttgtta aaaagattga attgcatttt gaactatgtg atgctgtaag tggtgagaaa    1380
   atccctgctg caacacaaaa cactaataca aattcaaatc gttacgaggg taaagtcatt    1440
   gatagcgcac cactgctacc gaaaatggac tttaaatcat caccattccg catgtataag    1500
   gtaggaactg agttcttagt atatgatcat aatcaatatt ggtacaagac atacattgat 1560
   gacaaacttt actacatgta taaaagcttt tgcgatgttg tagctaaaaa agacgcaaaa 1620
   ggtcgcatca aagttcgaat taaaagcgcg aaagacttgc gtattccagt ctggaataac 1680
   ataaaattga attctgggaa aattaaatgg tatgcaccca atgtaaaact agcgtggtac 1740
   aactatcgaa gaggatattt agagctatgg tatccgaacg acggctggta ttacacagca 1800
   gaatacttct taaaataa 1818

<210> 77
<211> 352
<212> PRT
<213> artificial sequence

<220>
<223> CBD118-500:

<400> 77
Met Asp Lys Gly Lys Lys Phe Val Ala Lys Ala Lys Ser Leu Gly Phe
1               5                   10                  15
Glu Trp Gly Gly Asp Trp Ser Gly Phe Val Asp Asn Pro His Leu Gln
            20                  25                  30
Phe Asn Tyr Lys Gly Tyr Gly Thr Asp Thr Phe Gly Lys Gly Ala Ser
        35                  40                  45
Thr Ser Asn Ser Ser Lys Pro Ser Ala Asp Thr Asn Thr Asn Ser Leu
    50                  55                  60
Gly Leu Val Asp Tyr Met Asn Leu Asn Lys Leu Asp Ser Ser Phe Ala
65                  70                  75                  80
Asn Arg Lys Lys Leu Ala Thr Ser Tyr Gly Ile Lys Asn Tyr Ser Gly
                85                  90                  95
Thr Ala Thr Gln Asn Thr Thr Leu Leu Ala Lys Leu Lys Ala Gly Lys
            100                 105                 110
Pro His Thr Pro Ala Ser Lys Asn Thr Tyr Tyr Thr Glu Asn Pro Arg
        115                 120                 125
Lys Val Lys Thr Leu Val Gln Cys Asp Leu Tyr Lys Ser Val Asp Phe
    130                 135                 140
Thr Thr Lys Asn Gln Thr Gly Gly Thr Phe Pro Pro Gly Thr Val Phe
145                 150                 155                 160
Thr Ile Ser Gly Met Gly Lys Thr Lys Gly Gly Thr Pro Arg Leu Lys
                165                 170                 175
Thr Lys Ser Gly Tyr Tyr Leu Thr Ala Asn Thr Lys Phe Val Lys Lys
            180                 185                 190
Ile Glu Leu His Phe Glu Leu Cys Asp Ala Val Ser Gly Glu Lys Ile
        195                 200                 205
Pro Ala Ala Thr Gln Asn Thr Asn Thr Asn Ser Asn Arg Tyr Glu Gly
    210                 215                 220
Lys Val Ile Asp Ser Ala Pro Leu Leu Pro Lys Met Asp Phe Lys Ser
225                 230                 235                 240
Ser Pro Phe Arg Met Tyr Lys Val Gly Thr Glu Phe Leu Val Tyr Asp
                245                 250                 255
His Asn Gln Tyr Trp Tyr Lys Thr Tyr Ile Asp Asp Lys Leu Tyr Tyr
            260                 265                 270
Met Tyr Lys Ser Phe Cys Asp Val Val Ala Lys Lys Asp Ala Lys Gly
        275                 280                 285
Arg Ile Lys Val Arg Ile Lys Ser Ala Lys Asp Leu Arg Ile Pro Val
    290                 295                 300
Trp Asn Asn Ile Lys Leu Asn Ser Gly Lys Ile Lys Trp Tyr Ala Pro
305                 310                 315                 320
Asn Val Lys Leu Ala Trp Tyr Asn Tyr Arg Arg Gly Tyr Leu Glu Leu
                325                 330                 335
Trp Tyr Pro Asn Asp Gly Trp Tyr Tyr Thr Ala Glu Tyr Phe Leu Lys
            340                 345                 350

<210>  78
<211>  1059
<212>  DNA
<213>  artificial sequence

<220>
<223>  CBD118-500:

<400>  78
atggacaaag gcaagaaatt tgtggcaaag gcaaaatctt taggttttga gtggggtggt      60
gattggtctg gatttgtaga caatccgcac cttcaattta attataaagg ctatgggact     120
gatacttttg gaaaaggagc tagtactagt aattcatcta aaccgagcgc agacacaaac     180
acaaacagtc taggattagt agattatatg aatttaaata aactagattc aagctttgcg     240
aatcgcaaaa aactagcgac aagttacgga attaaaaatt acagtggaac agcaacgcag     300
aacacaacat tattagcgaa gttaaaagca ggaaaaccac acacaccagc aagcaaaaac     360
acatactaca cagaaaatcc gcgaaaagtt aaaacactag tacaatgtga tctatacaaa     420
tcagtagact ttacaacaaa aaaccaaaca ggtggaacat ttccgccagg cacagtcttc     480
acgatttcag ggatggggaa aacgaaaggc ggaacacctc gcttgaagac gaagagcggt     540
tactatctca ctgctaacac gaagtttgtt aaaaagattg aattgcattt tgaactatgt     600
gatgctgtaa gtggtgagaa aatccctgct gcaacacaaa acactaatac aaattcaaat     660
cgttacgagg gtaaagtcat tgatagcgca ccactgctac cgaaaatgga ctttaaatca     720
tcaccattcc gcatgtataa ggtaggaact gagttcttag tatatgatca taatcaatat     780
tggtacaaga catacattga tgacaaactt tactacatgt ataaaagctt ttgcgatgtt     840
gtagctaaaa aagacgcaaa aggtcgcatc aaagttcgaa ttaaaagcgc gaaagacttg     900
cgtattccag tctggaataa cataaaattg aattctggga aaattaaatg gtatgcaccc     960
aatgtaaaac tagcgtggta caactatcga agaggatatt tagagctatg gtatccgaac    1020
gacggctggt attacacagc agaatacttc ttaaaataa                           1059


<210>  79
<211>  590
<212>  PRT
<213>  artificial sequence

<220>
<223>  HGFP_ L_CBD500L118: (mit PlyPSA linker)

<400>  79
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro

195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Thr Gly Lys Thr Val Ala Ala Lys Asn Pro Asn Arg His Ser Gln Asn
                245                 250                 255
Thr Asn Thr Asn Ser Asn Arg Tyr Glu Gly Lys Val Ile Asp Ser Ala
            260                 265                 270
Pro Leu Leu Pro Lys Met Asp Phe Lys Ser Ser Pro Phe Arg Met Tyr
        275                 280                 285
Lys Val Gly Thr Glu Phe Leu Val Tyr Asp His Asn Gln Tyr Trp Tyr
    290                 295                 300
Lys Thr Tyr Ile Asp Asp Lys Leu Tyr Tyr Met Tyr Lys Ser Phe Cys
305                 310                 315                 320
Asp Val Val Ala Lys Lys Asp Ala Lys Gly Arg Ile Lys Val Arg Ile
                325                 330                 335
Lys Ser Ala Lys Asp Leu Arg Ile Pro Val Trp Asn Asn Ile Lys Leu
            340                 345                 350
Asn Ser Gly Lys Ile Lys Trp Tyr Ala Pro Asn Val Lys Leu Ala Trp
        355                 360                 365
Tyr Asn Tyr Arg Arg Gly Tyr Leu Glu Leu Trp Tyr Pro Asn Asp Gly
    370                 375                 380
Trp Tyr Tyr Thr Ala Glu Tyr Phe Leu Lys Thr Gly Lys Thr Val Ala
385                 390                 395                 400
Ala Lys Asn Pro Asn Arg His Ser Lys Ser Leu Gly Phe Glu Trp Gly
                405                 410                 415
Gly Asp Trp Ser Gly Phe Val Asp Asn Pro His Leu Gln Phe Asn Tyr
            420                 425                 430
Lys Gly Tyr Gly Thr Asp Thr Phe Gly Lys Gly Ala Ser Thr Ser Asn
        435                 440                 445
Ser Ser Lys Pro Ser Ala Asp Thr Asn Thr Asn Ser Leu Gly Leu Val
    450                 455                 460
Asp Tyr Met Asn Leu Asn Lys Leu Asp Ser Ser Phe Ala Asn Arg Lys
465                 470                 475                 480
Lys Leu Ala Thr Ser Tyr Gly Ile Lys Asn Tyr Ser Gly Thr Ala Thr
                485                 490                 495
Gln Asn Thr Thr Leu Leu Ala Lys Leu Lys Ala Gly Lys Pro His Thr
            500                 505                 510
Pro Ala Ser Lys Asn Thr Tyr Tyr Thr Glu Asn Pro Arg Lys Val Lys
        515                 520                 525
Thr Leu Val Gln Cys Asp Leu Tyr Lys Ser Val Asp Phe Thr Thr Lys
    530                 535                 540
Asn Gln Thr Gly Gly Thr Phe Pro Pro Gly Thr Val Phe Thr Ile Ser
545                 550                 555                 560
Gly Met Gly Lys Thr Lys Gly Gly Thr Pro Arg Leu Lys Thr Lys Ser
                565                 570                 575
Gly Tyr Tyr Leu Thr Ala Asn Thr Lys Phe Val Lys Lys Ile
            580                 585                 590


<210>  80
<211>  1773
<212>  DNA
<213>  artificial sequence

<220>
<223>  HGFP_ L_CBD500L118: (mit PlyPSA linker)

<400>  80
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180

actactggaa aactacctgt tccatggcca acacttgtca ctactttcgc gtatggtctt 240
caatgctttg cgagataccc agatcatatg aaacggcatg actttttcaa gagtgccatg 300
cccgaaggtt atgtacagga aagaactata tttttcaaag atgacgggaa ctacaagaca 360
cgtgctgaag tcaagtttga aggtgatacc cttgttaata gaatcgagtt aaaaggtatt 420
gattttaaag aagatggaaa cattcttgga cacaaattgg aatacaacta taactcacac 480
aatgtataca tcatggcaga caaacaaaag aatggaatca aagttaactt caaaattaga 540
cacaacattg aagatggaag cgttcaacta gcagaccatt atcaacaaaa tactccaatt 600
ggcgatggcc ctgtcctttt accagacaac cattacctgt ccacacaatc tgccctttcg 660
aaagatccca acgaaaagag agaccacatg gtccttcttg agtttgtaac agctgctggg 720
actggtaaaa cagtagccgc aaaaaatcca aaccgccatt ctcaaaacac taatacaaat 780
tcaaatcgtt acgagggtaa agtcattgat agcgcaccac tgctaccgaa aatggacttt 840
aaatcatcac cattccgcat gtataaggta ggaactgagt tcttagtata tgatcataat 900
caatattggt acaagacata cattgatgac aaactttact acatgtataa aagcttttgc 960
gatgttgtag ctaaaaaaga cgcaaaaggt cgcatcaaag ttcgaattaa aagcgcgaaa 1020
gacttgcgta ttccagtctg gaataacata aaattgaatt ctgggaaaat taaatggtat 1080
gcacccaatg taaaactagc gtggtacaac tatcgaagag gatatttaga gctatggtat 1140
ccgaacgacg gctggtatta cacagcagaa tacttcttaa aaactggtaa aacagtagcc 1200
gcaaaaaatc caaaccgcca ttctaaatct ttaggttttg agtggggtgg tgattggtct 1260
ggatttgtag acaatccgca ccttcaattt aattataaag gctatgggac tgatactttt 1320
ggaaaaggag ctagtactag taattcatct aaaccgagcg cagacacaaa cacaaacagt 1380
ctaggattag tagattatat gaatttaaat aaactagatt caagctttgc gaatcgcaaa 1440
aaactagcga caagttacgg aattaaaaat tacagtggaa cagcaacgca gaacacaaca 1500
ttattagcga agttaaaagc aggaaaacca cacacaccag caagcaaaaa cacatactac 1560
acagaaaatc cgcgaaaagt taaaacacta gtacaatgtg atctatacaa atcagtagac 1620
tttacaacaa aaaaccaaac aggtggaaca tttccgccag gcacagtctt cacgatttca 1680
gggatgggga aaacgaaagg cggaacacct cgcttgaaga cgaagagcgg ttactatctc 1740
actgctaaca cgaagtttgt taaaaagatt taa 1773

<210> 81
<211> 337
<212> PRT
<213> artificial sequence

<220>
<223> CBD500L118:

<400> 81
Met Gln Asn Thr Asn Thr Asn Ser Asn Arg Tyr Glu Gly Lys Val Ile
1               5                   10                  15
Asp Ser Ala Pro Leu Leu Pro Lys Met Asp Phe Lys Ser Ser Pro Phe
            20                  25                  30
Arg Met Tyr Lys Val Gly Thr Glu Phe Leu Val Tyr Asp His Asn Gln
        35                  40                  45
Tyr Trp Tyr Lys Thr Tyr Ile Asp Asp Lys Leu Tyr Tyr Met Tyr Lys
    50                  55                  60
Ser Phe Cys Asp Val Val Ala Lys Lys Asp Ala Lys Gly Arg Ile Lys
65                  70                  75                  80
Val Arg Ile Lys Ser Ala Lys Asp Leu Arg Ile Pro Val Trp Asn Asn
                85                  90                  95
Ile Lys Leu Asn Ser Gly Lys Ile Lys Trp Tyr Ala Pro Asn Val Lys
            100                 105                 110
Leu Ala Trp Tyr Asn Tyr Arg Arg Gly Tyr Leu Glu Leu Trp Tyr Pro
        115                 120                 125
Asn Asp Gly Trp Tyr Tyr Thr Ala Glu Tyr Phe Leu Lys Thr Gly Lys
    130                 135                 140
Thr Val Ala Ala Lys Asn Pro Asn Arg His Ser Lys Ser Leu Gly Phe
145                 150                 155                 160
Glu Trp Gly Gly Asp Trp Ser Gly Phe Val Asp Asn Pro His Leu Gln
                165                 170                 175
Phe Asn Tyr Lys Gly Tyr Gly Thr Asp Thr Phe Gly Lys Gly Ala Ser
            180                 185                 190
Thr Ser Asn Ser Ser Lys Pro Ser Ala Asp Thr Asn Thr Asn Ser Leu

195                 200                 205
Gly Leu Val Asp Tyr Met Asn Leu Asn Lys Leu Asp Ser Ser Phe Ala
    210                 215                 220
Asn Arg Lys Lys Leu Ala Thr Ser Tyr Gly Ile Lys Asn Tyr Ser Gly
225                 230                 235                 240
Thr Ala Thr Gln Asn Thr Thr Leu Leu Ala Lys Leu Lys Ala Gly Lys
                245                 250                 255
Pro His Thr Pro Ala Ser Lys Asn Thr Tyr Tyr Thr Glu Asn Pro Arg
            260                 265                 270
Lys Val Lys Thr Leu Val Gln Cys Asp Leu Tyr Lys Ser Val Asp Phe
        275                 280                 285
Thr Thr Lys Asn Gln Thr Gly Gly Thr Phe Pro Pro Gly Thr Val Phe
    290                 295                 300
Thr Ile Ser Gly Met Gly Lys Thr Lys Gly Gly Thr Pro Arg Leu Lys
305                 310                 315                 320
Thr Lys Ser Gly Tyr Tyr Leu Thr Ala Asn Thr Lys Phe Val Lys Lys
                325                 330                 335
Ile

<210>  82
<211>  1014
<212>  DNA
<213>  artificial sequence

<220>
<223>  CBD500L118:

<400>  82
atgcaaaaca ctaatacaaa ttcaaatcgt tacgagggta aagtcattga tagcgcacca      60
ctgctaccga aaatggactt taaatcatca ccattccgca tgtataaggt aggaactgag     120
ttcttagtat atgatcataa tcaatattgg tacaagacat acattgatga caaactttac     180
tacatgtata aaagcttttg cgatgttgta gctaaaaaag acgcaaaagg tcgcatcaaa     240
gttcgaatta aaagcgcgaa agacttgcgt attccagtct ggaataacat aaaattgaat     300
tctgggaaaa ttaaatggta tgcacccaat gtaaaactag cgtggtacaa ctatcgaaga     360
ggatatttag agctatggta tccgaacgac ggctggtatt acacagcaga atacttctta     420
aaaactggta aaacagtagc cgcaaaaaat ccaaaccgcc attctaaatc tttaggtttt     480
gagtggggtg gtgattggtc tggatttgta gacaatccgc accttcaatt taattataaa     540
ggctatggga ctgatacttt tggaaaagga gctagtacta gtaattcatc taaaccgagc     600
gcagacacaa acacaaacag tctaggatta gtagattata tgaatttaaa taaactagat     660
tcaagctttg cgaatcgcaa aaaactagcg acaagttacg gaattaaaaa ttacagtgga     720
acagcaacgc agaacacaac attattagcg aagttaaaag caggaaaacc acacacacca     780
gcaagcaaaa acacatacta cacagaaaat ccgcgaaaag ttaaaacact agtacaatgt     840
gatctataca aatcagtaga ctttacaaca aaaaaccaaa caggtggaac atttccgcca     900
ggcacagtct tcacgatttc agggatgggg aaaacgaaag gcggaacacc tcgcttgaag     960
acgaagagcg gttactatct cactgctaac acgaagtttg ttaaaaagat ttaa          1014

<210>  83
<211>  590
<212>  PRT
<213>  artificial sequence

<220>
<223>  HGFP_ L_CBD118L500: (mit PlyPSA linker)

<400>  83

Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30

Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Thr Gly Lys Thr Val Ala Ala Lys Asn Pro Asn Arg His Ser Lys Ser
                245                 250                 255
Leu Gly Phe Glu Trp Gly Gly Asp Trp Ser Gly Phe Val Asp Asn Pro
            260                 265                 270
His Leu Gln Phe Asn Tyr Lys Gly Tyr Gly Thr Asp Thr Phe Gly Lys
        275                 280                 285
Gly Ala Ser Thr Ser Asn Ser Ser Lys Pro Ser Ala Asp Thr Asn Thr
    290                 295                 300
Asn Ser Leu Gly Leu Val Asp Tyr Met Asn Leu Asn Lys Leu Asp Ser
305                 310                 315                 320
Ser Phe Ala Asn Arg Lys Lys Leu Ala Thr Ser Tyr Gly Ile Lys Asn
                325                 330                 335
Tyr Ser Gly Thr Ala Thr Gln Asn Thr Thr Leu Leu Ala Lys Leu Lys
            340                 345                 350
Ala Gly Lys Pro His Thr Pro Ala Ser Lys Asn Thr Tyr Tyr Thr Glu
        355                 360                 365
Asn Pro Arg Lys Val Lys Thr Leu Val Gln Cys Asp Leu Tyr Lys Ser
    370                 375                 380
Val Asp Phe Thr Thr Lys Asn Gln Thr Gly Gly Thr Phe Pro Pro Gly
385                 390                 395                 400
Thr Val Phe Thr Ile Ser Gly Met Gly Lys Thr Lys Gly Gly Thr Pro
                405                 410                 415
Arg Leu Lys Thr Lys Ser Gly Tyr Tyr Leu Thr Ala Asn Thr Lys Phe
            420                 425                 430
Val Lys Lys Ile Thr Gly Lys Thr Val Ala Ala Lys Asn Pro Asn Arg
        435                 440                 445
His Ser Gln Asn Thr Asn Thr Asn Ser Asn Arg Tyr Glu Gly Lys Val
    450                 455                 460
Ile Asp Ser Ala Pro Leu Leu Pro Lys Met Asp Phe Lys Ser Ser Pro
465                 470                 475                 480
Phe Arg Met Tyr Lys Val Gly Thr Glu Phe Leu Val Tyr Asp His Asn
                485                 490                 495
Gln Tyr Trp Tyr Lys Thr Tyr Ile Asp Asp Lys Leu Tyr Tyr Met Tyr
            500                 505                 510
Lys Ser Phe Cys Asp Val Val Ala Lys Lys Asp Ala Lys Gly Arg Ile
        515                 520                 525
Lys Val Arg Ile Lys Ser Ala Lys Asp Leu Arg Ile Pro Val Trp Asn

530                 535                 540
Asn Ile Lys Leu Asn Ser Gly Lys Ile Lys Trp Tyr Ala Pro Asn Val
545                 550                 555                 560
Lys Leu Ala Trp Tyr Asn Tyr Arg Arg Gly Tyr Leu Glu Leu Trp Tyr
                565                 570                 575
Pro Asn Asp Gly Trp Tyr Tyr Thr Ala Glu Tyr Phe Leu Lys
            580                 585                 590

<210>  84
<211>  1773
<212>  DNA
<213>  artificial sequence

<220>
<223>  HGFP_ L_CBD118L500: (mit PlyPSA linker)

<400>  84
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180
actactggaa aactacctgt tccatggcca acacttgtca ctactttcgc gtatggtctt     240
caatgctttg cgagataccc agatcatatg aaacggcatg actttttcaa gagtgccatg     300
cccgaaggtt atgtacagga aagaactata tttttcaaag atgacgggaa ctacaagaca     360
cgtgctgaag tcaagtttga aggtgatacc cttgttaata gaatcgagtt aaaaggtatt     420
gattttaaag aagatggaaa cattcttgga cacaaattgg aatacaacta taactcacac     480
aatgtataca tcatggcaga caaacaaaag aatggaatca aagttaactt caaaattaga     540
cacaacattg aagatggaag cgttcaacta gcagaccatt atcaacaaaa tactccaatt     600
ggcgatggcc ctgtcctttt accagacaac cattacctgt ccacacaatc tgccctttcg     660
aaagatccca acgaaaagag agaccacatg gtccttcttg agtttgtaac agctgctggg     720
actggtaaaa cagtagccgc aaaaaatcca aaccgccatt ctaaatcttt aggttttgag     780
tggggtggtg attggtctgg atttgtagac aatccgcacc ttcaatttaa ttataaaggc     840
tatggactgg atacttttgg aaaaggagct agtactagta attcatctaa accgagcgca     900
gacacaaaca caaacagtct aggattagta gattatatga atttaaataa actagattca     960
agctttgcga atcgcaaaaa actagcgaca agttacggaa ttaaaaatta cagtggaaca    1020
gcaacgcaga acacaacatt attagcgaag ttaaaagcag gaaaaccaca cacaccagca    1080
agcaaaaaca catactacac agaaaatccg cgaaaagtta aaacactagt acaatgtgat    1140
ctatacaaat cagtagactt tacaacaaaa aaccaaacag gtggaacatt tccgccaggc    1200
acagtcttca cgatttcagg gatggggaaa acgaaaggcg gaacacctcg cttgaagacg    1260
aagagcggtt actatctcac tgctaacacg aagtttgtta aaaagattac tggtaaaaca    1320
gtagccgcaa aaaatccaaa ccgccattct caaaacacta atacaaattc aaatcgttac    1380
gagggtaaag tcattgatag cgcaccactg ctaccgaaaa tggactttaa atcatcacca    1440
ttccgcatgt ataaggtagg aactgagttc ttagtatatg atcataatca atattggtac    1500
aagacataca ttgatgacaa actttactac atgtataaaa gcttttgcga tgttgtagct    1560
aaaaaagacg caaaaggtcg catcaaagtt cgaattaaaa gcgcgaaaga cttgcgtatt    1620
ccagtctgga ataacataaa attgaattct gggaaaatta aatggtatgc acccaatgta    1680
aaactagcgt ggtacaacta tcgaagagga tatttagagc tatggtatcc gaacgacggc    1740
tggtattaca cagcagaata cttcttaaaa taa                                 1773

<210>  85
<211>  337
<212>  PRT
<213>  artificial sequence

<220>
<223>  CBD118L500:

<400>  85
Met Lys Ser Leu Gly Phe Glu Trp Gly Gly Asp Trp Ser Gly Phe Val
1               5                   10                  15
Asp Asn Pro His Leu Gln Phe Asn Tyr Lys Gly Tyr Gly Thr Asp Thr
            20                  25                  30

Phe Gly Lys Gly Ala Ser Thr Ser Asn Ser Ser Lys Pro Ser Ala Asp
        35                  40                  45
Thr Asn Thr Asn Ser Leu Gly Leu Val Asp Tyr Met Asn Leu Asn Lys
    50                  55                  60
Leu Asp Ser Ser Phe Ala Asn Arg Lys Lys Leu Ala Thr Ser Tyr Gly
65                  70                  75                  80
Ile Lys Asn Tyr Ser Gly Thr Ala Thr Gln Asn Thr Thr Leu Leu Ala
                85                  90                  95
Lys Leu Lys Ala Gly Lys Pro His Thr Pro Ala Ser Lys Asn Thr Tyr
            100                 105                 110
Tyr Thr Glu Asn Pro Arg Lys Val Lys Thr Leu Val Gln Cys Asp Leu
        115                 120                 125
Tyr Lys Ser Val Asp Phe Thr Thr Lys Asn Gln Thr Gly Gly Thr Phe
    130                 135                 140
Pro Pro Gly Thr Val Phe Thr Ile Ser Gly Met Gly Lys Thr Lys Gly
145                 150                 155                 160
Gly Thr Pro Arg Leu Lys Thr Lys Ser Gly Tyr Tyr Leu Thr Ala Asn
                165                 170                 175
Thr Lys Phe Val Lys Lys Ile Thr Gly Lys Thr Val Ala Ala Lys Asn
            180                 185                 190
Pro Asn Arg His Ser Gln Asn Thr Asn Thr Asn Ser Asn Arg Tyr Glu
        195                 200                 205
Gly Lys Val Ile Asp Ser Ala Pro Leu Leu Pro Lys Met Asp Phe Lys
    210                 215                 220
Ser Ser Pro Phe Arg Met Tyr Lys Val Gly Thr Glu Phe Leu Val Tyr
225                 230                 235                 240
Asp His Asn Gln Tyr Trp Tyr Lys Thr Tyr Ile Asp Asp Lys Leu Tyr
                245                 250                 255
Tyr Met Tyr Lys Ser Phe Cys Asp Val Val Ala Lys Lys Asp Ala Lys
            260                 265                 270
Gly Arg Ile Lys Val Arg Ile Lys Ser Ala Lys Asp Leu Arg Ile Pro
        275                 280                 285
Val Trp Asn Asn Ile Lys Leu Asn Ser Gly Lys Ile Lys Trp Tyr Ala
    290                 295                 300
Pro Asn Val Lys Leu Ala Trp Tyr Asn Tyr Arg Arg Gly Tyr Leu Glu
305                 310                 315                 320
Leu Trp Tyr Pro Asn Asp Gly Trp Tyr Tyr Thr Ala Glu Tyr Phe Leu
                325                 330                 335
Lys

<210>  86
<211>  1014
<212>  DNA
<213>  artificial sequence

<220>
<223>  CBD118L500:

<400>  86
atgaaatctt taggttttga gtggggtggt gattggtctg gatttgtaga caatccgcac      60
cttcaattta attataaagg ctatgggact gatacttttg gaaaaggagc tagtactagt     120
aattcatcta aaccgagcgc agacacaaac acaaacagtc taggattagt agattatatg     180
aatttaaata aactagattc aagctttgcg aatcgcaaaa aactagcgac aagttacgga     240
attaaaaatt acagtggaac agcaacgcag aacacaacat tattagcgaa gttaaaagca     300
ggaaaaccac acacaccagc aagcaaaaac acatactaca cagaaaatcc gcgaaaagtt     360
aaaacactag tacaatgtga tctatacaaa tcagtagact ttacaacaaa aaaccaaaca     420
ggtggaacat ttccgccagg cacagtcttc acgatttcag ggatggggaa aacgaaaggc     480
ggaacacctc gcttgaagac gaagagcggt tactatctca ctgctaacac gaagtttgtt     540
aaaaagatta ctggtaaaac agtagccgca aaaaatccaa accgccattc tcaaaacact     600
aatacaaatt caaatcgtta cgagggtaaa gtcattgata gcgcaccact gctaccgaaa     660
atggacttta aatcatcacc attccgcatg tataaggtag gaactgagtt cttagtatat     720

gatcataatc aatattggta caagacatac attgatgaca aactttacta catgtataaa    780
agcttttgcg atgttgtagc taaaaaagac gcaaaaggtc gcatcaaagt tcgaattaaa    840
agcgcgaaag acttgcgtat tccagtctgg aataacataa aattgaattc tgggaaaatt    900
aaatggtatg cacccaatgt aaaactagcg tggtacaact atcgaagagg atatttagag    960
ctatggtatc cgaacgacgg ctggtattac acagcagaat acttcttaaa ataa         1014

<210>  87
<211>  588
<212>  PRT
<213>  artificial sequence

<220>
<223>  HCBD500_GFP_ L_118: (mit PlyPSA linker)

<400>  87
Met Arg Gly Ser His His His His His His Gly Ser Gln Asn Thr Asn
1               5                   10                  15
Thr Asn Ser Asn Arg Tyr Glu Gly Lys Val Ile Asp Ser Ala Pro Leu
            20                  25                  30
Leu Pro Lys Met Asp Phe Lys Ser Ser Pro Phe Arg Met Tyr Lys Val
        35                  40                  45
Gly Thr Glu Phe Leu Val Tyr Asp His Asn Gln Tyr Trp Tyr Lys Thr
    50                  55                  60
Tyr Ile Asp Asp Lys Leu Tyr Tyr Met Tyr Lys Ser Phe Cys Asp Val
65                  70                  75                  80
Val Ala Lys Lys Asp Ala Lys Gly Arg Ile Lys Val Arg Ile Lys Ser
                85                  90                  95
Ala Lys Asp Leu Arg Ile Pro Val Trp Asn Asn Ile Lys Leu Asn Ser
            100                 105                 110
Gly Lys Ile Lys Trp Tyr Ala Pro Asn Val Lys Leu Ala Trp Tyr Asn
        115                 120                 125
Tyr Arg Arg Gly Tyr Leu Glu Leu Trp Tyr Pro Asn Asp Gly Trp Tyr
    130                 135                 140
Tyr Thr Ala Glu Tyr Phe Leu Lys Gly Thr Met Ser Lys Gly Glu Glu
145                 150                 155                 160
Leu Phe Thr Gly Val Val Pro Ile Leu Val Glu Leu Asp Gly Asp Val
                165                 170                 175
Asn Gly His Lys Phe Ser Val Ser Gly Glu Gly Glu Gly Asp Ala Thr
            180                 185                 190
Tyr Gly Lys Leu Thr Leu Lys Phe Ile Cys Thr Thr Gly Lys Leu Pro
        195                 200                 205
Val Pro Trp Pro Thr Leu Val Thr Thr Phe Ala Tyr Gly Leu Gln Cys
    210                 215                 220
Phe Ala Arg Tyr Pro Asp His Met Lys Arg His Asp Phe Phe Lys Ser
225                 230                 235                 240
Ala Met Pro Glu Gly Tyr Val Gln Glu Arg Thr Ile Phe Phe Lys Asp
                245                 250                 255
Asp Gly Asn Tyr Lys Thr Arg Ala Glu Val Lys Phe Glu Gly Asp Thr
            260                 265                 270
Leu Val Asn Arg Ile Glu Leu Lys Gly Ile Asp Phe Lys Glu Asp Gly
        275                 280                 285
Asn Ile Leu Gly His Lys Leu Glu Tyr Asn Tyr Asn Ser His Asn Val
    290                 295                 300
Tyr Ile Met Ala Asp Lys Gln Lys Asn Gly Ile Lys Val Asn Phe Lys
305                 310                 315                 320
Ile Arg His Asn Ile Glu Asp Gly Ser Val Gln Leu Ala Asp His Tyr
                325                 330                 335
Gln Gln Asn Thr Pro Ile Gly Asp Gly Pro Val Leu Leu Pro Asp Asn
            340                 345                 350
His Tyr Leu Ser Thr Gln Ser Ala Leu Ser Lys Asp Pro Asn Glu Lys
        355                 360                 365
Arg Asp His Met Val Leu Leu Glu Phe Val Thr Ala Ala Gly Thr Gly

370                 375                 380
Lys Thr Val Ala Ala Lys Asn Pro Asn Arg His Ser Asp Lys Gly Lys
385                 390                 395                 400
Lys Phe Val Ala Lys Ala Lys Ser Leu Gly Phe Glu Trp Gly Gly Asp
                405                 410                 415
Trp Ser Gly Phe Val Asp Asn Pro His Leu Gln Phe Asn Tyr Lys Gly
            420                 425                 430
Tyr Gly Thr Asp Thr Phe Gly Lys Gly Ala Ser Thr Ser Asn Ser Ser
        435                 440                 445
Lys Pro Ser Ala Asp Thr Asn Thr Asn Ser Leu Gly Leu Val Asp Tyr
    450                 455                 460
Met Asn Leu Asn Lys Leu Asp Ser Ser Phe Ala Asn Arg Lys Lys Leu
465                 470                 475                 480
Ala Thr Ser Tyr Gly Ile Lys Asn Tyr Ser Gly Thr Ala Thr Gln Asn
                485                 490                 495
Thr Thr Leu Leu Ala Lys Leu Lys Ala Gly Lys Pro His Thr Pro Ala
            500                 505                 510
Ser Lys Asn Thr Tyr Tyr Thr Glu Asn Pro Arg Lys Val Lys Thr Leu
        515                 520                 525
Val Gln Cys Asp Leu Tyr Lys Ser Val Asp Phe Thr Thr Lys Asn Gln
    530                 535                 540
Thr Gly Gly Thr Phe Pro Pro Gly Thr Val Phe Thr Ile Ser Gly Met
545                 550                 555                 560
Gly Lys Thr Lys Gly Gly Thr Pro Arg Leu Lys Thr Lys Ser Gly Tyr
                565                 570                 575
Tyr Leu Thr Ala Asn Thr Lys Phe Val Lys Lys Ile
            580                 585


<210>  88
<211>  1767
<212>  DNA
<213>  artificial sequence

<220>
<223>  HCBD500_GFP_ L_118: (mit PlyPSA linker)

<400>  88
atgagaggat cgcatcacca tcaccatcac ggatcccaaa acactaatac aaattcaaat      60
cgttacgagg gtaaagtcat tgatagcgca ccactgctac cgaaaatgga ctttaaatca     120
tcaccattcc gcatgtataa ggtaggaact gagttcttag tatatgatca taatcaatat     180
tggtacaaga catacattga tgacaaactt tactacatgt ataaaagctt ttgcgatgtt     240
gtagctaaaa aagacgcaaa aggtcgcatc aaagttcgaa ttaaaagcgc gaaagacttg     300
cgtattccag tctggaataa cataaaattg aattctggga aaattaaatg gtatgcaccc     360
aatgtaaaac tagcgtggta caactatcga agaggatatt tagagctatg gtatccgaac     420
gacggctggt attacacagc agaatacttc ttaaaaggta ccatgagtaa aggagaagaa     480
cttttcactg gagttgtccc aattcttgtt gaattagatg gtgatgttaa tgggcacaaa     540
ttttctgtca gtggagaggg tgaaggtgat gcaacatacg gaaaacttac ccttaaattt     600
atttgcacta ctggaaaact acctgttcca tggccaacac ttgtcactac tttcgcgtat     660
ggtcttcaat gctttgcgag atacccagat catatgaaac ggcatgactt tttcaagagt     720
gccatgcccg aaggttatgt acaggaaaga actatatttt tcaaagatga cgggaactac     780
aagacacgtg ctgaagtcaa gtttgaaggt gatacccttg ttaatagaat cgagttaaaa     840
ggtattgatt ttaaagaaga tggaaacatt cttggacaca aattggaata caactataac     900
tcacacaatg tatacatcat ggcagacaaa caaaagaatg gaatcaaagt taacttcaaa     960
attagacaca acattgaaga tggaagcgtt caactagcag accattatca acaaaatact    1020
ccaattggcg atggccctgt ccttttacca gacaaccatt acctgtccac acaatctgcc    1080
ctttcgaaag atcccaacga aaagagagac cacatggtcc ttcttgagtt tgtaacagct    1140
gctgggactg gtaaaacagt agccgcaaaa aatccaaacc gccattctga caaaggcaag    1200
aaatttgtgg caaaggcaaa atctttaggt tttgagtggg gtggtgattg gtctggattt    1260
gtagacaatc cgcaccttca atttaattat aaaggctatg ggactgatac ttttggaaaa    1320
ggagctagta ctagtaattc atctaaaccg agcgcagaca caaacacaaa cagtctagga    1380
ttagtagatt atatgaattt aaataaacta gattcaagct ttgcgaatcg caaaaaacta    1440
gcgacaagtt acggaattaa aaattacagt ggaacagcaa cgcagaacac aacattatta    1500

gcgaagttaa aagcaggaaa accacacaca ccagcaagca aaaacacata ctacacagaa   1560
aatccgcgaa aagttaaaac actagtacaa tgtgatctat acaaatcagt agactttaca   1620
acaaaaaacc aaacaggtgg aacatttccg ccaggcacag tcttcacgat ttcagggatg   1680
ggaaaaacga aaggcggaac acctcgcttg aagacgaaga gcggttacta tctcactgct   1740
aacacgaagt ttgttaaaaa gatttaa                                       1767

<210>  89
<211>  603
<212>  PRT
<213>  artificial sequence

<220>
<223>  HCBD118_GFP_ L_500: (mit PlyPSA linker)

<400>  89
Met Arg Gly Ser His His His His His His Gly Ser Asp Lys Gly Lys
1               5                   10                  15
Lys Phe Val Ala Lys Ala Lys Ser Leu Gly Phe Glu Trp Gly Gly Asp
            20                  25                  30
Trp Ser Gly Phe Val Asp Asn Pro His Leu Gln Phe Asn Tyr Lys Gly
        35                  40                  45
Tyr Gly Thr Asp Thr Phe Gly Lys Gly Ala Ser Thr Ser Asn Ser Ser
    50                  55                  60
Lys Pro Ser Ala Asp Thr Asn Thr Asn Ser Leu Gly Leu Val Asp Tyr
65                  70                  75                  80
Met Asn Leu Asn Lys Leu Asp Ser Ser Phe Ala Asn Arg Lys Lys Leu
                85                  90                  95
Ala Thr Ser Tyr Gly Ile Lys Asn Tyr Ser Gly Thr Ala Thr Gln Asn
            100                 105                 110
Thr Thr Leu Leu Ala Lys Leu Lys Ala Gly Lys Pro His Thr Pro Ala
        115                 120                 125
Ser Lys Asn Thr Tyr Tyr Thr Glu Asn Pro Arg Lys Val Lys Thr Leu
    130                 135                 140
Val Gln Cys Asp Leu Tyr Lys Ser Val Asp Phe Thr Thr Lys Asn Gln
145                 150                 155                 160
Thr Gly Gly Thr Phe Pro Pro Gly Thr Val Phe Thr Ile Ser Gly Met
                165                 170                 175
Gly Lys Thr Lys Gly Gly Thr Pro Arg Leu Lys Thr Lys Ser Gly Tyr
            180                 185                 190
Tyr Leu Thr Ala Asn Thr Lys Phe Val Lys Lys Ile Met Ser Lys Gly
        195                 200                 205
Glu Glu Leu Phe Thr Gly Val Val Pro Ile Leu Val Glu Leu Asp Gly
    210                 215                 220
Asp Val Asn Gly His Lys Phe Ser Val Ser Gly Glu Gly Glu Gly Asp
225                 230                 235                 240
Ala Thr Tyr Gly Lys Leu Thr Leu Lys Phe Ile Cys Thr Thr Gly Lys
                245                 250                 255
Leu Pro Val Pro Trp Pro Thr Leu Val Thr Thr Phe Ala Tyr Gly Leu
            260                 265                 270
Gln Cys Phe Ala Arg Tyr Pro Asp His Met Lys Arg His Asp Phe Phe
        275                 280                 285
Lys Ser Ala Met Pro Glu Gly Tyr Val Gln Glu Arg Thr Ile Phe Phe
    290                 295                 300
Lys Asp Asp Gly Asn Tyr Lys Thr Arg Ala Glu Val Lys Phe Glu Gly
305                 310                 315                 320
Asp Thr Leu Val Asn Arg Ile Glu Leu Lys Gly Ile Asp Phe Lys Glu
                325                 330                 335
Asp Gly Asn Ile Leu Gly His Lys Leu Glu Tyr Asn Tyr Asn Ser His
            340                 345                 350
Asn Val Tyr Ile Met Ala Asp Lys Gln Lys Asn Gly Ile Lys Val Asn
        355                 360                 365
Phe Lys Ile Arg His Asn Ile Glu Asp Gly Ser Val Gln Leu Ala Asp

370                 375                 380
His Tyr Gln Gln Asn Thr Pro Ile Gly Asp Gly Pro Val Leu Leu Pro
385                 390                 395                 400
Asp Asn His Tyr Leu Ser Thr Gln Ser Ala Leu Ser Lys Asp Pro Asn
                405                 410                 415
Glu Lys Arg Asp His Met Val Leu Leu Glu Phe Val Thr Ala Ala Gly
            420                 425                 430
Thr Gly Lys Thr Val Ala Ala Lys Asn Pro Asn Arg His Ser His Phe
        435                 440                 445
Glu Leu Cys Asp Ala Val Ser Gly Glu Lys Ile Pro Ala Ala Thr Gln
    450                 455                 460
Asn Thr Asn Thr Asn Ser Asn Arg Tyr Glu Gly Lys Val Ile Asp Ser
465                 470                 475                 480
Ala Pro Leu Leu Pro Lys Met Asp Phe Lys Ser Ser Pro Phe Arg Met
                485                 490                 495
Tyr Lys Val Gly Thr Glu Phe Leu Val Tyr Asp His Asn Gln Tyr Trp
            500                 505                 510
Tyr Lys Thr Tyr Ile Asp Asp Lys Leu Tyr Tyr Met Tyr Lys Ser Phe
        515                 520                 525
Cys Asp Val Val Ala Lys Lys Asp Ala Lys Gly Arg Ile Lys Val Arg
    530                 535                 540
Ile Lys Ser Ala Lys Asp Leu Arg Ile Pro Val Trp Asn Asn Ile Lys
545                 550                 555                 560
Leu Asn Ser Gly Lys Ile Lys Trp Tyr Ala Pro Asn Val Lys Leu Ala
                565                 570                 575
Trp Tyr Asn Tyr Arg Arg Gly Tyr Leu Glu Leu Trp Tyr Pro Asn Asp
            580                 585                 590
Gly Trp Tyr Tyr Thr Ala Glu Tyr Phe Leu Lys
        595                 600


<210>  90
<211>  1812
<212>  DNA
<213>  artificial sequence

<220>
<223>  HCBD118_GFP_ L_500: (mit PlyPSA linker)

<400>  90
atgagaggat cgcatcacca tcaccatcac ggatccgaca aaggcaagaa atttgtggca      60
aaggcaaaat ctttaggttt tgagtggggt ggtgattggt ctggatttgt agacaatccg     120
caccttcaat ttaattataa aggctatggg actgatactt ttggaaaagg agctagtact     180
agtaattcat ctaaaccgag cgcagacaca aacacaaaca gtctaggatt agtagattat     240
atgaatttaa ataaactaga ttcaagcttt gcgaatcgca aaaaactagc gacaagttac     300
ggaattaaaa attacagtgg aacagcaacg cagaacacaa cattattagc gaagttaaaa     360
gcaggaaaac cacacacacc agcaagcaaa aacacatact acacagaaaa tccgcgaaaa     420
gttaaaacac tagtacaatg tgatctatac aaatcagtag actttacaac aaaaaaccaa     480
acaggtggaa catttccgcc aggcacagtc ttcacgattt cagggatggg gaaaacgaaa     540
ggcggaacac ctcgcttgaa gacgaagagc ggttactatc tcactgctaa cacgaagttt     600
gttaaaaaga ttatgagtaa aggagaagaa cttttcactg gagttgtccc aattcttgtt     660
gaattagatg gtgatgttaa tgggcacaaa ttttctgtca gtggagaggg tgaaggtgat     720
gcaacatacg gaaaacttac ccttaaattt atttgcacta ctggaaaact acctgttcca     780
tggccaacac ttgtcactac tttcgcgtat ggtcttcaat gctttgcgag atacccagat     840
catatgaaac ggcatgactt tttcaagagt gccatgcccg aaggttatgt acaggaaaga     900
actatatttt tcaaagatga cgggaactac aagacacgtg ctgaagtcaa gtttgaaggt     960
gatacccttg ttaatagaat cgagttaaaa ggtattgatt ttaaagaaga tggaaacatt    1020
cttggacaca aattggaata caactataac tcacacaatg tatacatcat ggcagacaaa    1080
caaaagaatg gaatcaaagt taacttcaaa attagacaca acattgaaga tggaagcgtt    1140
caactagcag accattatca acaaaatact ccaattggcg atggccctgt ccttttacca    1200
gacaaccatt acctgtccac acaatctgcc ctttcgaaag atcccaacga aaagagagac    1260
cacatggtcc ttcttgagtt tgtaacagct gctgggactg gtaaaacagt agccgcaaaa    1320
aatccaaacc gccattctca ttttgaacta tgtgatgctg taagtggtga gaaaatccct    1380

gctgcaacac aaaacactaa tacaaattca aatcgttacg agggtaaagt cattgatagc 1440
gcaccactgc taccgaaaat ggactttaaa tcatcaccat tccgcatgta taaggtagga 1500
actgagttct tagtatatga tcataatcaa tattggtaca agacatacat tgatgacaaa 1560
ctttactaca tgtataaaag cttttgcgat gttgtagcta aaaaagacgc aaaaggtcgc 1620
atcaaagttc gaattaaaag cgcgaaagac ttgcgtattc cagtctggaa taacataaaa 1680
ttgaattctg ggaaaattaa atggtatgca cccaatgtaa aactagcgtg gtacaactat 1740
cgaagaggat atttagagct atggtatccg aacgacggct ggtattacac agcagaatac 1800
ttcttaaaat aa 1812

<210> 91
<211> 558
<212> PRT
<213> artificial sequence

<220>
<223> HGFP_ L_CBD500-P35: (mit PlyPSA linker)

<400> 91
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Thr Gly Lys Thr Val Ala Ala Lys Asn Pro Asn Arg His Ser Gln Asn
                245                 250                 255
Thr Asn Thr Asn Ser Asn Arg Tyr Glu Gly Lys Val Ile Asp Ser Ala
            260                 265                 270
Pro Leu Leu Pro Lys Met Asp Phe Lys Ser Ser Pro Phe Arg Met Tyr
        275                 280                 285
Lys Val Gly Thr Glu Phe Leu Val Tyr Asp His Asn Gln Tyr Trp Tyr
    290                 295                 300
Lys Thr Tyr Ile Asp Asp Lys Leu Tyr Tyr Met Tyr Lys Ser Phe Cys
305                 310                 315                 320
Asp Val Val Ala Lys Lys Asp Ala Lys Gly Arg Ile Lys Val Arg Ile
                325                 330                 335
Lys Ser Ala Lys Asp Leu Arg Ile Pro Val Trp Asn Asn Ile Lys Leu
            340                 345                 350

Asn Ser Gly Lys Ile Lys Trp Tyr Ala Pro Asn Val Lys Leu Ala Trp
        355                 360                 365
Tyr Asn Tyr Arg Arg Gly Tyr Leu Glu Leu Trp Tyr Pro Asn Asp Gly
    370                 375                 380
Trp Tyr Tyr Thr Ala Glu Tyr Phe Leu Lys Gln Phe Pro His Phe Glu
385                 390                 395                 400
Ala Cys Asp Trp Tyr Arg Gly Glu Arg Lys Tyr Lys Val Asp Thr Ser
                405                 410                 415
Glu Trp Lys Lys Lys Glu Asn Ile Asn Ile Val Ile Lys Asp Val Gly
            420                 425                 430
Tyr Phe Gln Asp Lys Pro Gln Phe Leu Asn Ser Lys Ser Val Arg Gln
        435                 440                 445
Trp Lys His Gly Thr Lys Val Lys Leu Thr Lys His Asn Ser His Trp
    450                 455                 460
Tyr Thr Gly Val Val Lys Asp Gly Asn Lys Ser Val Arg Gly Tyr Ile
465                 470                 475                 480
Tyr His Ser Met Ala Lys Val Thr Ser Lys Asn Ser Asp Gly Ser Val
                485                 490                 495
Asn Ala Thr Ile Asn Ala His Ala Phe Cys Trp Asp Asn Lys Lys Leu
            500                 505                 510
Asn Gly Gly Asp Phe Ile Asn Leu Lys Arg Gly Phe Lys Gly Ile Thr
        515                 520                 525
His Pro Ala Ser Asp Gly Phe Tyr Pro Leu Tyr Phe Ala Ser Arg Lys
    530                 535                 540
Lys Thr Phe Tyr Ile Pro Arg Tyr Met Phe Asp Ile Lys Lys
545                 550                 555


<210>  92
<211>  1677
<212>  DNA
<213>  artificial sequence

<220>
<223>  HGFP_ L_CBD500-P35: (mit PlyPSA linker)

<400>  92
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180
actactggaa aactacctgt tccatggcca acacttgtca ctactttcgc gtatggtctt     240
caatgctttg cgagataccc agatcatatg aaacggcatg actttttcaa gagtgccatg     300
cccgaaggtt atgtacagga aagaactata tttttcaaag atgacgggaa ctacaagaca     360
cgtgctgaag tcaagtttga aggtgatacc cttgttaata gaatcgagtt aaaaggtatt     420
gattttaaag aagatggaaa cattcttgga cacaaattgg aatacaacta taactcacac     480
aatgtataca tcatggcaga caaacaaaag aatggaatca aagttaactt caaaattaga     540
cacaacattg aagatggaag cgttcaacta gcagaccatt atcaacaaaa tactccaatt     600
ggcgatggcc ctgtcctttt accagacaac cattacctgt ccacacaatc tgccctttcg     660
aaagatccca acgaaaagag agaccacatg gtccttcttg agtttgtaac agctgctggg     720
actggtaaaa cagtagccgc aaaaaatcca aaccgccatt ctcaaaacac taatacaaat     780
tcaaatcgtt acgagggtaa agtcattgat agcgcaccac tgctaccgaa aatggacttt     840
aaatcatcac cattccgcat gtataaggta ggaactgagt tcttagtata tgatcataat     900
caatattggt acaagacata cattgatgac aaactttact acatgtataa aagcttttgc     960
gatgttgtag ctaaaaaaga cgcaaaaggt cgcatcaaag ttcgaattaa aagcgcgaaa    1020
gacttgcgta ttccagtctg gaataacata aaattgaatt ctgggaaaat taaatggtat    1080
gcacccaatg taaaactagc gtggtacaac tatcgaagag gatatttaga gctatggtat    1140
ccgaacgacg gctggtatta cacagcagaa tacttcttaa aacaattccc acattttgaa    1200
gcttgtgact ggtatcgcgg ggaacgcaag tataaagtgg acacatctga atggaaaaag    1260
aaagagaata tcaatatcgt tattaaagat gttggttact tccaagacaa acctcaattc    1320
ttaaactcca aatcggttcg tcagtggaag catggcacga aagtgaagct tactaaacat    1380
aactcacatt ggtacactgg tgtggtcaag gatggtaaca aatcagtcag gggatatatt    1440
tatcattcga tggctaaggt cacaagcaag aatagcgacg gttcggttaa cgcaacgatt    1500
aacgcccacg cattttgttg ggacaataaa aaacttaatg gtggcgactt tatcaacttg    1560

aagcgtggtt ttaaaggtat cacccatccc gctagtgacg gtttctatcc actgtatttc    1620
gcttctagga aaaaaacttt ctacattccg cgttacatgt ttgacatcaa gaaataa       1677

<210>  93
<211>  305
<212>  PRT
<213>  artificial sequence

<220>
<223>  CBD500-P35:

<400>  93
Met Gln Asn Thr Asn Thr Asn Ser Asn Arg Tyr Glu Gly Lys Val Ile
1               5                   10                  15
Asp Ser Ala Pro Leu Leu Pro Lys Met Asp Phe Lys Ser Ser Pro Phe
            20                  25                  30
Arg Met Tyr Lys Val Gly Thr Glu Phe Leu Val Tyr Asp His Asn Gln
        35                  40                  45
Tyr Trp Tyr Lys Thr Tyr Ile Asp Asp Lys Leu Tyr Tyr Met Tyr Lys
    50                  55                  60
Ser Phe Cys Asp Val Val Ala Lys Lys Asp Ala Lys Gly Arg Ile Lys
65                  70                  75                  80
Val Arg Ile Lys Ser Ala Lys Asp Leu Arg Ile Pro Val Trp Asn Asn
                85                  90                  95
Ile Lys Leu Asn Ser Gly Lys Ile Lys Trp Tyr Ala Pro Asn Val Lys
            100                 105                 110
Leu Ala Trp Tyr Asn Tyr Arg Arg Gly Tyr Leu Glu Leu Trp Tyr Pro
        115                 120                 125
Asn Asp Gly Trp Tyr Tyr Thr Ala Glu Tyr Phe Leu Lys Gln Phe Pro
    130                 135                 140
His Phe Glu Ala Cys Asp Trp Tyr Arg Gly Glu Arg Lys Tyr Lys Val
145                 150                 155                 160
Asp Thr Ser Glu Trp Lys Lys Lys Glu Asn Ile Asn Ile Val Ile Lys
                165                 170                 175
Asp Val Gly Tyr Phe Gln Asp Lys Pro Gln Phe Leu Asn Ser Lys Ser
            180                 185                 190
Val Arg Gln Trp Lys His Gly Thr Lys Val Lys Leu Thr Lys His Asn
        195                 200                 205
Ser His Trp Tyr Thr Gly Val Val Lys Asp Gly Asn Lys Ser Val Arg
    210                 215                 220
Gly Tyr Ile Tyr His Ser Met Ala Lys Val Thr Ser Lys Asn Ser Asp
225                 230                 235                 240
Gly Ser Val Asn Ala Thr Ile Asn Ala His Ala Phe Cys Trp Asp Asn
                245                 250                 255
Lys Lys Leu Asn Gly Gly Asp Phe Ile Asn Leu Lys Arg Gly Phe Lys
            260                 265                 270
Gly Ile Thr His Pro Ala Ser Asp Gly Phe Tyr Pro Leu Tyr Phe Ala
        275                 280                 285
Ser Arg Lys Lys Thr Phe Tyr Ile Pro Arg Tyr Met Phe Asp Ile Lys
    290                 295                 300
Lys
305

<210>  94
<211>  918
<212>  DNA
<213>  artificial sequence

<220>
<223>  CBD500-P35:

<400> 94
atgcaaaaca ctaatacaaa ttcaaatcgt tacgagggta aagtcattga tagcgcacca 60
ctgctaccga aaatggactt taaatcatca ccattccgca tgtataaggt aggaactgag 120
ttcttagtat atgatcataa tcaatattgg tacaagacat acattgatga caaactttac 180
tacatgtata aaagcttttg cgatgttgta gctaaaaaag acgcaaaagg tcgcatcaaa 240
gttcgaatta aaagcgcgaa agacttgcgt attccagtct ggaataacat aaaattgaat 300
tctgggaaaa ttaaatggta tgcacccaat gtaaaactag cgtggtacaa ctatcgaaga 360
ggatatttag agctatggta tccgaacgac ggctggtatt acacagcaga atacttctta 420
aaacaattcc cacattttga agcttgtgac tggtatcgcg gggaacgcaa gtataaagtg 480
gacacatctg aatggaaaaa gaaagagaat atcaatatcg ttattaaaga tgttggttac 540
ttccaagaca aacctcaatt cttaaactcc aaatcggttc gtcagtggaa gcatggcacg 600
aaagtgaagc ttactaaaca taactcacat tggtacactg gtgtggtcaa ggatggtaac 660
aaatcagtca ggggatatat ttatcattcg atggctaagg tcacaagcaa gaatagcgac 720
ggttcggtta acgcaacgat taacgcccac gcattttgtt gggacaataa aaaacttaat 780
ggtggcgact ttatcaactt gaagcgtggt tttaaaggta tcacccatcc cgctagtgac 840
ggtttctatc cactgtattt cgcttctagg aaaaaaactt tctacattcc gcgttacatg 900
tttgacatca agaaataa 918

<210> 95
<211> 558
<212> PRT
<213> artificial sequence

<220>
<223> HGFP_ L_CBDP35-500: (mit PlyPSA linker)

<400> 95
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205
Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Thr Gly Lys Thr Val Ala Ala Lys Asn Pro Asn Arg His Ser Pro His
                245                 250                 255
Phe Glu Ala Cys Asp Trp Tyr Arg Gly Glu Arg Lys Tyr Lys Val Asp
            260                 265                 270
Thr Ser Glu Trp Lys Lys Lys Glu Asn Ile Asn Ile Val Ile Lys Asp

275                 280                 285
Val Gly Tyr Phe Gln Asp Lys Pro Gln Phe Leu Asn Ser Lys Ser Val
    290                 295                 300
Arg Gln Trp Lys His Gly Thr Lys Val Lys Leu Thr Lys His Asn Ser
305                 310                 315                 320
His Trp Tyr Thr Gly Val Val Lys Asp Gly Asn Lys Ser Val Arg Gly
                325                 330                 335
Tyr Ile Tyr His Ser Met Ala Lys Val Thr Ser Lys Asn Ser Asp Gly
            340                 345                 350
Ser Val Asn Ala Thr Ile Asn Ala His Ala Phe Cys Trp Asp Asn Lys
        355                 360                 365
Lys Leu Asn Gly Gly Asp Phe Ile Asn Leu Lys Arg Gly Phe Lys Gly
    370                 375                 380
Ile Thr His Pro Ala Ser Asp Gly Phe Tyr Pro Leu Tyr Phe Ala Ser
385                 390                 395                 400
Arg Lys Lys Thr Phe Tyr Ile Pro Arg Tyr Met Phe Asp Ile Lys Lys
                405                 410                 415
Gln Phe Gln Asn Thr Asn Thr Asn Ser Asn Arg Tyr Glu Gly Lys Val
            420                 425                 430
Ile Asp Ser Ala Pro Leu Leu Pro Lys Met Asp Phe Lys Ser Ser Pro
        435                 440                 445
Phe Arg Met Tyr Lys Val Gly Thr Glu Phe Leu Val Tyr Asp His Asn
    450                 455                 460
Gln Tyr Trp Tyr Lys Thr Tyr Ile Asp Asp Lys Leu Tyr Tyr Met Tyr
465                 470                 475                 480
Lys Ser Phe Cys Asp Val Val Ala Lys Lys Asp Ala Lys Gly Arg Ile
                485                 490                 495
Lys Val Arg Ile Lys Ser Ala Lys Asp Leu Arg Ile Pro Val Trp Asn
            500                 505                 510
Asn Ile Lys Leu Asn Ser Gly Lys Ile Lys Trp Tyr Ala Pro Asn Val
        515                 520                 525
Lys Leu Ala Trp Tyr Asn Tyr Arg Arg Gly Tyr Leu Glu Leu Trp Tyr
    530                 535                 540
Pro Asn Asp Gly Trp Tyr Tyr Thr Ala Glu Tyr Phe Leu Lys
545                 550                 555


<210>  96
<211>  1677
<212>  DNA
<213>  artificial sequence

<220>
<223>  HGFP_ L_CBDP35-500: (mit PlyPSA linker)

<400>  96
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180
actactggaa aactacctgt tccatggcca acacttgtca ctactttcgc gtatggtctt     240
caatgctttg cgagataccc agatcatatg aaacggcatg actttttcaa gagtgccatg     300
cccgaaggtt atgtacagga aagaactata tttttcaaag atgacgggaa ctacaagaca     360
cgtgctgaag tcaagtttga aggtgatacc cttgttaata gaatcgagtt aaaaggtatt     420
gattttaaag aagatggaaa cattcttgga cacaaattgg aatacaacta taactcacac     480
aatgtataca tcatggcaga caaacaaaag aatggaatca aagttaactt caaaattaga     540
cacaacattg aagatggaag cgttcaacta gcagaccatt atcaacaaaa tactccaatt     600
ggcgatggcc ctgtcctttt accagacaac cattacctgt ccacacaatc tgccctttcg     660
aaagatccca acgaaaagag agaccacatg gtccttcttg agtttgtaac agctgctggg     720
actggtaaaa cagtagccgc aaaaaatcca aaccgccatt ctccacattt tgaagcttgt     780
gactggtatc gcggggaacg caagtataaa gtggacacat ctgaatggaa aaagaaagag     840
aatatcaata tcgttattaa agatgttggt tacttccaag acaaacctca attcttaaac     900
tccaaatcgg ttcgtcagtg gaagcatggc acgaaagtga agcttactaa acataactca     960
cattggtaca ctggtgtggt caaggatggt aacaaatcag tcaggggata tatttatcat    1020

tcgatggcta aggtcacaag caagaatagc gacggttcgg ttaacgcaac gattaacgcc 1080
cacgcatttt gttgggacaa taaaaaactt aatggtggcg actttatcaa cttgaagcgt 1140
ggttttaaag gtatcaccca tcccgctagt gacggtttct atccactgta tttcgcttct 1200
aggaaaaaaa ctttctacat tccgcgttac atgtttgaca tcaagaaaca attccaaaac 1260
actaatacaa attcaaatcg ttacgagggt aaagtcattg atagcgcacc actgctaccg 1320
aaaatggact ttaaatcatc accattccgc atgtataagg taggaactga gttcttagta 1380
tatgatcata atcaatattg gtacaagaca tacattgatg acaaacttta ctacatgtat 1440
aaaagctttt gcgatgttgt agctaaaaaa gacgcaaaag gtcgcatcaa agttcgaatt 1500
aaaagcgcga aagacttgcg tattccagtc tggaataaca taaaattgaa ttctgggaaa 1560
attaaatggt atgcacccaa tgtaaaacta gcgtggtaca actatcgaag aggatattta 1620
gagctatggt atccgaacga cggctggtat tacacagcag aatacttctt aaaataa 1677

<210> 97
<211> 305
<212> PRT
<213> artificial sequence

<220>
<223> CBDP35-500:

<400> 97
Met Pro His Phe Glu Ala Cys Asp Trp Tyr Arg Gly Glu Arg Lys Tyr
1               5                   10                  15
Lys Val Asp Thr Ser Glu Trp Lys Lys Lys Glu Asn Ile Asn Ile Val
            20                  25                  30
Ile Lys Asp Val Gly Tyr Phe Gln Asp Lys Pro Gln Phe Leu Asn Ser
        35                  40                  45
Lys Ser Val Arg Gln Trp Lys His Gly Thr Lys Val Lys Leu Thr Lys
    50                  55                  60
His Asn Ser His Trp Tyr Thr Gly Val Val Lys Asp Gly Asn Lys Ser
65                  70                  75                  80
Val Arg Gly Tyr Ile Tyr His Ser Met Ala Lys Val Thr Ser Lys Asn
                85                  90                  95
Ser Asp Gly Ser Val Asn Ala Thr Ile Asn Ala His Ala Phe Cys Trp
            100                 105                 110
Asp Asn Lys Lys Leu Asn Gly Gly Asp Phe Ile Asn Leu Lys Arg Gly
        115                 120                 125
Phe Lys Gly Ile Thr His Pro Ala Ser Asp Gly Phe Tyr Pro Leu Tyr
    130                 135                 140
Phe Ala Ser Arg Lys Lys Thr Phe Tyr Ile Pro Arg Tyr Met Phe Asp
145                 150                 155                 160
Ile Lys Lys Gln Phe Gln Asn Thr Asn Thr Asn Ser Asn Arg Tyr Glu
                165                 170                 175
Gly Lys Val Ile Asp Ser Ala Pro Leu Leu Pro Lys Met Asp Phe Lys
            180                 185                 190
Ser Ser Pro Phe Arg Met Tyr Lys Val Gly Thr Glu Phe Leu Val Tyr
        195                 200                 205
Asp His Asn Gln Tyr Trp Tyr Lys Thr Tyr Ile Asp Asp Lys Leu Tyr
    210                 215                 220
Tyr Met Tyr Lys Ser Phe Cys Asp Val Val Ala Lys Lys Asp Ala Lys
225                 230                 235                 240
Gly Arg Ile Lys Val Arg Ile Lys Ser Ala Lys Asp Leu Arg Ile Pro
                245                 250                 255
Val Trp Asn Asn Ile Lys Leu Asn Ser Gly Lys Ile Lys Trp Tyr Ala
            260                 265                 270
Pro Asn Val Lys Leu Ala Trp Tyr Asn Tyr Arg Arg Gly Tyr Leu Glu
        275                 280                 285
Leu Trp Tyr Pro Asn Asp Gly Trp Tyr Tyr Thr Ala Glu Tyr Phe Leu
    290                 295                 300
Lys
305

<210>  98
<211>  918
<212>  DNA
<213>  artificial sequence

<220>
<223>  CBDP35-500:

<400>  98
atgccacatt ttgaagcttg tgactggtat cgcggggaac gcaagtataa agtggacaca      60
tctgaatgga aaaagaaaga gaatatcaat atcgttatta aagatgttgg ttacttccaa     120
gacaaacctc aattcttaaa ctccaaatcg gttcgtcagt ggaagcatgg cacgaaagtg     180
aagcttacta aacataactc acattggtac actggtgtgg tcaaggatgg taacaaatca     240
gtcaggggat atatttatca ttcgatggct aaggtcacaa gcaagaatag cgacggttcg     300
gttaacgcaa cgattaacgc ccacgcattt tgttgggaca ataaaaaact taatggtggc     360
gactttatca acttgaagcg tggttttaaa ggtatcaccc atcccgctag tgacggtttc     420
tatccactgt atttcgcttc taggaaaaaa actttctaca ttccgcgtta catgtttgac     480
atcaagaaac aattccaaaa cactaataca aattcaaatc gttacgaggg taaagtcatt     540
gatagcgcac cactgctacc gaaaatggac tttaaatcat caccattccg catgtataag     600
gtaggaactg agttcttagt atatgatcat aatcaatatt ggtacaagac atacattgat     660
gacaaacttt actacatgta taaaagcttt tgcgatgttg tagctaaaaa agacgcaaaa     720
ggtcgcatca aagttcgaat taaaagcgcg aaagacttgc gtattccagt ctggaataac     780
ataaaattga attctgggaa aattaaatgg tatgcaccca atgtaaaact agcgtggtac     840
aactatcgaa gaggatattt agagctatgg tatccgaacg acggctggta ttacacagca     900
gaatacttct taaaataa                                                   918


<210>  99
<211>  553
<212>  PRT
<213>  artificial sequence

<220>
<223>  HGFP_ L_CBD500-500: (mit PlyPSA linker)

<400>  99
Met Arg Gly Ser His His His His His His Gly Ser Met Ser Lys Gly
1               5                   10                  15
Glu Glu Leu Phe Thr Gly Val Val Pro Ile Leu Val Glu Leu Asp Gly
            20                  25                  30
Asp Val Asn Gly His Lys Phe Ser Val Ser Gly Glu Gly Glu Gly Asp
        35                  40                  45
Ala Thr Tyr Gly Lys Leu Thr Leu Lys Phe Ile Cys Thr Thr Gly Lys
    50                  55                  60
Leu Pro Val Pro Trp Pro Thr Leu Val Thr Thr Phe Ala Tyr Gly Leu
65                  70                  75                  80
Gln Cys Phe Ala Arg Tyr Pro Asp His Met Lys Arg His Asp Phe Phe
                85                  90                  95
Lys Ser Ala Met Pro Glu Gly Tyr Val Gln Glu Arg Thr Ile Phe Phe
            100                 105                 110
Lys Asp Asp Gly Asn Tyr Lys Thr Arg Ala Glu Val Lys Phe Glu Gly
        115                 120                 125
Asp Thr Leu Val Asn Arg Ile Glu Leu Lys Gly Ile Asp Phe Lys Glu
    130                 135                 140
Asp Gly Asn Ile Leu Gly His Lys Leu Glu Tyr Asn Tyr Asn Ser His
145                 150                 155                 160
Asn Val Tyr Ile Met Ala Asp Lys Gln Lys Asn Gly Ile Lys Val Asn
                165                 170                 175
Phe Lys Ile Arg His Asn Ile Glu Asp Gly Ser Val Gln Leu Ala Asp
            180                 185                 190
His Tyr Gln Gln Asn Thr Pro Ile Gly Asp Gly Pro Val Leu Leu Pro
        195                 200                 205

Asp Asn His Tyr Leu Ser Thr Gln Ser Ala Leu Ser Lys Asp Pro Asn
    210                 215                 220
Glu Lys Arg Asp His Met Val Leu Leu Glu Phe Val Thr Ala Ala Gly
225                 230                 235                 240
Thr Gly Lys Thr Val Ala Ala Lys Asn Pro Asn Arg His Ser Gln Asn
                245                 250                 255
Thr Asn Thr Asn Ser Asn Arg Tyr Glu Gly Lys Val Ile Asp Ser Ala
            260                 265                 270
Pro Leu Leu Pro Lys Met Asp Phe Lys Ser Ser Pro Phe Arg Met Tyr
        275                 280                 285
Lys Val Gly Thr Glu Phe Leu Val Tyr Asp His Asn Gln Tyr Trp Tyr
    290                 295                 300
Lys Thr Tyr Ile Asp Asp Lys Leu Tyr Tyr Met Tyr Lys Ser Phe Cys
305                 310                 315                 320
Asp Val Val Ala Lys Lys Asp Ala Lys Gly Arg Ile Lys Val Arg Ile
                325                 330                 335
Lys Ser Ala Lys Asp Leu Arg Ile Pro Val Trp Asn Asn Ile Lys Leu
            340                 345                 350
Asn Ser Gly Lys Ile Lys Trp Tyr Ala Pro Asn Val Lys Leu Ala Trp
        355                 360                 365
Tyr Asn Tyr Arg Arg Gly Tyr Leu Glu Leu Trp Tyr Pro Asn Asp Gly
    370                 375                 380
Trp Tyr Tyr Thr Ala Glu Tyr Phe Leu Lys Glu Leu His Phe Glu Leu
385                 390                 395                 400
Cys Asp Ala Val Ser Gly Glu Lys Ile Pro Ala Ala Thr Gln Asn Thr
                405                 410                 415
Asn Thr Asn Ser Asn Arg Tyr Glu Gly Lys Val Ile Asp Ser Ala Pro
            420                 425                 430
Leu Leu Pro Lys Met Asp Phe Lys Ser Ser Pro Phe Arg Met Tyr Lys
        435                 440                 445
Val Gly Thr Glu Phe Leu Val Tyr Asp His Asn Gln Tyr Trp Tyr Lys
    450                 455                 460
Thr Tyr Ile Asp Asp Lys Leu Tyr Tyr Met Tyr Lys Ser Phe Cys Asp
465                 470                 475                 480
Val Val Ala Lys Lys Asp Ala Lys Gly Arg Ile Lys Val Arg Ile Lys
                485                 490                 495
Ser Ala Lys Asp Leu Arg Ile Pro Val Trp Asn Asn Ile Lys Leu Asn
            500                 505                 510
Ser Gly Lys Ile Lys Trp Tyr Ala Pro Asn Val Lys Leu Ala Trp Tyr
        515                 520                 525
Asn Tyr Arg Arg Gly Tyr Leu Glu Leu Trp Tyr Pro Asn Asp Gly Trp
    530                 535                 540
Tyr Tyr Thr Ala Glu Tyr Phe Leu Lys
545                 550


<210>  100
<211>  1662
<212>  DNA
<213>  artificial sequence

<220>
<223>  HGFP_ L_CBD500-500: (mit PlyPSA linker)

<400>  100
atgagaggat cgcatcacca tcaccatcac ggatccatga gtaaaggaga agaacttttc      60
actggagttg tcccaattct tgttgaatta gatggtgatg ttaatgggca caaattttct     120
gtcagtggag agggtgaagg tgatgcaaca tacggaaaac ttacccttaa atttatttgc     180
actactggaa aactacctgt tccatggcca acacttgtca ctactttcgc gtatggtctt     240
caatgctttg cgagataccc agatcatatg aaacggcatg actttttcaa gagtgccatg     300
cccgaaggtt atgtacagga aagaactata tttttcaaag atgacgggaa ctacaagaca     360
cgtgctgaag tcaagtttga aggtgatacc cttgttaata gaatcgagtt aaaaggtatt     420
gattttaaag aagatggaaa cattcttgga cacaaattgg aatacaacta taactcacac     480

aatgtataca tcatggcaga caaacaaaag aatggaatca aagttaactt caaaattaga 540
cacaacattg aagatggaag cgttcaacta gcagaccatt atcaacaaaa tactccaatt 600
ggcgatggcc ctgtcctttt accagacaac cattacctgt ccacacaatc tgccctttcg 660
aaagatccca acgaaaagag agaccacatg gtccttcttg agtttgtaac agctgctggg 720
actggtaaaa cagtagccgc aaaaaatcca aaccgccatt ctcaaaacac taatacaaat 780
tcaaatcgtt acgagggtaa agtcattgat agcgcaccac tgctaccgaa aatggacttt 840
aaatcatcac cattccgcat gtataaggta ggaactgagt tcttagtata tgatcataat 900
caatattggt acaagacata cattgatgac aaactttact acatgtataa aagcttttgc 960
gatgttgtag ctaaaaaaga cgcaaaaggt cgcatcaaag ttcgaattaa aagcgcgaaa 1020
gacttgcgta ttccagtctg gaataacata aaattgaatt ctgggaaaat taaatggtat 1080
gcacccaatg taaaactagc gtggtacaac tatcgaagag gatatttaga gctatggtat 1140
ccgaacgacg gctggtatta cacagcagaa tacttcttaa aagagctcca ttttgaacta 1200
tgtgatgctg taagtggtga gaaaatccct gctgcaacac aaaacactaa tacaaattca 1260
aatcgttacg agggtaaagt cattgatagc gcaccactgc taccgaaaat ggactttaaa 1320
tcatcaccat tccgcatgta taaggtagga actgagttct tagtatatga tcataatcaa 1380
tattggtaca agacatacat tgatgacaaa ctttactaca tgtataaaag cttttgcgat 1440
gttgtagcta aaaaagacgc aaaaggtcgc atcaaagttc gaattaaaag cgcgaaagac 1500
ttgcgtattc cagtctggaa taacataaaa ttgaattctg ggaaaattaa atggtatgca 1560
cccaatgtaa aactagcgtg gtacaactat cgaagaggat atttagagct atggtatccg 1620
aacgacggct ggtattacac agcagaatac ttcttaaaat aa 1662

<210> 101
<211> 300
<212> PRT
<213> artificial sequence

<220>
<223> CBD500-500:

<400> 101
Met Gln Asn Thr Asn Thr Asn Ser Asn Arg Tyr Glu Gly Lys Val Ile
1               5                   10                  15
Asp Ser Ala Pro Leu Leu Pro Lys Met Asp Phe Lys Ser Ser Pro Phe
            20                  25                  30
Arg Met Tyr Lys Val Gly Thr Glu Phe Leu Val Tyr Asp His Asn Gln
        35                  40                  45
Tyr Trp Tyr Lys Thr Tyr Ile Asp Asp Lys Leu Tyr Tyr Met Tyr Lys
    50                  55                  60
Ser Phe Cys Asp Val Val Ala Lys Lys Asp Ala Lys Gly Arg Ile Lys
65                  70                  75                  80
Val Arg Ile Lys Ser Ala Lys Asp Leu Arg Ile Pro Val Trp Asn Asn
                85                  90                  95
Ile Lys Leu Asn Ser Gly Lys Ile Lys Trp Tyr Ala Pro Asn Val Lys
            100                 105                 110
Leu Ala Trp Tyr Asn Tyr Arg Arg Gly Tyr Leu Glu Leu Trp Tyr Pro
        115                 120                 125
Asn Asp Gly Trp Tyr Tyr Thr Ala Glu Tyr Phe Leu Lys Glu Leu His
    130                 135                 140
Phe Glu Leu Cys Asp Ala Val Ser Gly Glu Lys Ile Pro Ala Ala Thr
145                 150                 155                 160
Gln Asn Thr Asn Thr Asn Ser Asn Arg Tyr Glu Gly Lys Val Ile Asp
                165                 170                 175
Ser Ala Pro Leu Leu Pro Lys Met Asp Phe Lys Ser Ser Pro Phe Arg
            180                 185                 190
Met Tyr Lys Val Gly Thr Glu Phe Leu Val Tyr Asp His Asn Gln Tyr
        195                 200                 205
Trp Tyr Lys Thr Tyr Ile Asp Asp Lys Leu Tyr Tyr Met Tyr Lys Ser
    210                 215                 220
Phe Cys Asp Val Val Ala Lys Lys Asp Ala Lys Gly Arg Ile Lys Val
225                 230                 235                 240
Arg Ile Lys Ser Ala Lys Asp Leu Arg Ile Pro Val Trp Asn Asn Ile
                245                 250                 255

Lys Leu Asn Ser Gly Lys Ile Lys Trp Tyr Ala Pro Asn Val Lys Leu
            260                 265                 270
Ala Trp Tyr Asn Tyr Arg Arg Gly Tyr Leu Glu Leu Trp Tyr Pro Asn
        275                 280                 285
Asp Gly Trp Tyr Tyr Thr Ala Glu Tyr Phe Leu Lys
    290                 295                 300

<210>  102
<211>  903
<212>  DNA
<213>  artificial sequence

<220>
<223>  CBD500-500:

<400>  102
atgcaaaaca ctaatacaaa ttcaaatcgt tacgagggta aagtcattga tagcgcacca      60
ctgctaccga aaatggactt taaatcatca ccattccgca tgtataaggt aggaactgag     120
ttcttagtat atgatcataa tcaatattgg tacaagacat acattgatga caaactttac     180
tacatgtata aaagcttttg cgatgttgta gctaaaaaag acgcaaaagg tcgcatcaaa     240
gttcgaatta aaagcgcgaa agacttgcgt attccagtct ggaataacat aaaattgaat     300
tctgggaaaa ttaaatggta tgcacccaat gtaaaactag cgtggtacaa ctatcgaaga     360
ggatatttag agctatggta tccgaacgac ggctggtatt acacagcaga atacttctta     420
aaagagctcc attttgaact atgtgatgct gtaagtggtg agaaaatccc tgctgcaaca     480
caaaacacta atacaaattc aaatcgttac gagggtaaag tcattgatag cgcaccactg     540
ctaccgaaaa tggactttaa atcatcacca ttccgcatgt ataaggtagg aactgagttc     600
ttagtatatg atcataatca atattggtac aagacataca ttgatgacaa actttactac     660
atgtataaaa gcttttgcga tgttgtagct aaaaaagacg caaaaggtcg catcaaagtt     720
cgaattaaaa gcgcgaaaga cttgcgtatt ccagtctgga ataacataaa attgaattct     780
gggaaaatta aatggtatgc acccaatgta aaactagcgt ggtacaacta tcgaagagga     840
tatttagagc tatggtatcc gaacgacggc tggtattaca cagcagaata cttcttaaaa     900
taa                                                                   903

## Description

The present invention relates to recombinant polypeptides having the activity of binding and lysing of bacteria, comprising at least one enzymatically active domain and at least two bacterial cell binding domains. The present invention further relates to recombinant polypeptide having the activity of binding bacteria, comprising at least two bacterial cell binding domain. Further the present inventions relates to nucleic acid molecules comprising a nucleotide sequence encoding the recombinant polypeptides, vectors and host cells.

In recent years, peptidoglycan-degrading enzymes like bacteriophage endolysins have received increasing attention as antimicrobial agents. In view of emerging and spreading resistance of pathogenic bacteria against classical antibiotics, the demand for alternative ways of controlling these organisms is rising. Especially in case of Gram-positive bacteria, the application of phage endolysins as so called enzybiotics is a promising approach. Due to the absence of an outer membrane in Gram-positives, these enzymes also work as exolysins, i.e. they can cause lysis of susceptible cells from without. This property can be exploited e.g. in molecular biology for the efficient recovery of nucleic acids and proteins from bacterial cells, as it was demonstrated for endolysins from phages infecting *Listeria.* Aiming towards an application for control of the foodborne pathogen *Listeria monocytogenes,* genes coding for these lysins were introduced into a number of lactic acid bacteria including *Lactococcus lactis* and several lactobacilli, which are used as starter organisms in cheese production. Overexpressing and secreting the endolysins, these bacteria consequently showed lytic activity against *L. monocytogenes* cells. Medical applications of phage encoded peptidoglycan hydrolases reported so far include the detection and killing of *Bacillus anthracis,* and the control of *Streptococcus pneumoniae* in vitro and in mouse models.

Endolysins are cell wall lytic enzymes which are encoded in the late gene region of dsDNA phages and produced at the end of the lytic multiplication cycle. The same enzymes are also found within prophage genomes integrated into bacterial genomes. Their function is degradation of the bacterial peptidoglycan from within, resulting in lysis of the host cell and release of the phage progeny. According to their different target bonds within the peptidoglycan, endolysins can be divided into 5 different classes: (i) N-acetyl-β-D-muramidases (also known as lysozymes) and (ii) N-acetyl-β-D-glucosaminidases, which are both glycosidases and cleave one of the two β-1,4 glycosidic bonds of the glycan strands each; (iii) lytic transglycosylases, which cleave the same bond as muramidases, but by a different mechanism; (iv) N-acetylmuramoyl-L-alanine amidases, which cut between the glycan and the peptide moieties; and (v) endopeptidases, which cleave within the peptide moiety. All endolysins except for lytic transglycosylases are hydrolases. The same enzymatic activities are also found in bacterial enzymes which lyse cell walls of its own or closely related bacteria, the so-called autolysins, and other bacterial cell wall lysing polypeptides like the bacteriocins. Bacterial autolysins are cell wall lytic enzymes, which play important roles in cell wall remodeling, cell division, transformation, or as virulence factors. Together they can be summarized as peptidoglycan lysing enzymes. The enzymatic degradation of the peptidoglycan due to the action of peptidoglycan lysing enzymes results in loss of integrity of the cell wall and finally in cell disruption caused by the high internal pressure.

The feature of killing bacterial cells makes the peptidoglycan lysing enzymes interesting candidates for a use as a prophylactic or therapeutic agent against bacterial infections in humans and animals, as an antimicrobial or enzybiotics for use as a disinfectant in medical, public or private environment, for use as a decontaminant of bacterial contamination in food industry, animal feed or cosmetic industry or as a general surfactanct against bacterially contaminated surfaces. Peptidoglycan lysing enzymes are as well useful in bacterial diagnostics as they specifically bind and lyse bacterial cells from distinct bacterial groups, genera, species, strains or serovars. Specific cell lysis is often combined with additional detection methods relying on the cellular content of the bacterial cells to be detected like nucleic acid based methods or immunological methods.

Endolysins, autolysins and other peptidoglycan lysing enzymes from a Gram-positive background show a modular organization in which catalytic activity and substrate recognition are separated and localized in at least two distinct functional domains, the enzymatically active domain (EAD) and the cell binding domain (CBD). Most endolysins from phages infecting Gram-negative hosts are single-domain globular proteins. However, recently two lysins from a Gram-negative background that consist of two functional domains were reported: The endolysins of the *Pseudomonas aeruginosa* phages ΦKZ and EL consist of an N-terminal cell binding domain and a C-terminal catalytic domain. In contrast, the majority of endolysins from phages infecting Gram-positive bacteria feature a reverse orientation of the domains, with an N-terminal EAD and a C-terminal CBD.

For only very few endolysins, the ligands in the bacterial cell wall recognized by the binding domain are known. The pneumococcal phage Cpl-1 lysozyme specifically recognizes choline containing teichoic acids in the cell wall of *Streptococcus pneumoniae,* which places it in the family of Choline-Binding Proteins (CBP). The cholin binding modules (CBM) of these proteins are formed by a repeat of about 20 amino acids found in multiple tandem copies, ranging from 4 to 18. Cpl-1 exemplifies the modular design of these enzymes with two separate domains - an EAD and a CBD- usually connected via a short linker region. Although the majority of modular phage endolysins consist of one catalytic and one substrate binding domain, there are a number of proteins that harbor two different enzymatic activities, as e.g. the endolysins of *Streptococcus agalactiae* phage B30 (muramidase and endopeptidase), *Staphylococcus aureus* phage Φ11 (endopeptidase and amidase), *Streptococcus agalactiae* phage NCTC 11261 (endopeptidase and muramidase), and *Staphylococcus warneri* M phage ΦWMY (endopeptidase and amidase).

The fact that peptidoglycan lysing enzymes with phage and bacterial origin, endolysins as well as autolysins or bacteriocins show similar modular architectures, and that high homologies between distinct domains of bacterial and phage derived lytic proteins can be found, suggests a common ancestry and co-evolution of these proteins by interchange of functional domains (Garcia et al., 1990, Gene 86, 81-88). Diaz et al. (1990, Proc. Natl. Acad. Sci., 87, 8125-8129) created chimaeras of phage and bacterial pneumococcal enzymes which exhibited combined biochemical properties. Recombinant chimaeras from genes lacking nucleotide homology were constructed in Diaz et al. (1991, J. Biol. Chem., 266, 5464-6571), confirming also the function of the CBDs in substrate recognition. Croux et al. (1993, Mol. Microbiol., 9, 1019-1025) even created chimaeras based on pneumococcal and clostridial cell wall lytic enzymes which led to the switch in enzymatic activity of endolysins towards cells from other bacterial families. Sanz et al. (1996, Eur. J. Biochem., 235, 601-605) constructed multifunctional pneumococcal murein hydrolases by module assembly which comprised two EADs and one CBD. Recently, fusion proteins consisting of lysostaphin, a peptidoglycan hydrolase from *Staphylococcus simulans,* and the *Streptococcus agalactiae* phage B30 endolysin, as well as a C-terminally truncated version thereof, were reported (Donovan et al. 2006, Appl. Environ. Microbiol., 72, 2988-2996). Also in this case, the artificial constructs combined properties of both enzymes, lysing both *Staphylococcus* and *Streptococcus* cells. Loessner et al. (2002, Mol. Microbiol., 44, 335-349) described the concept of CBDs determining the specific recognition and high-affinity binding to bacterial cell wall carbohydrates using *Listeria monocytogenes* as a role model. US 2004/0197833 teaches the use of immobilized isolated CBDs in a method for the enrichment of target cells.

The object of the present invention is to provide improved and advantageous proteins which allow the reliable detection and enrichment and/or lysis of bacterial cells.

The object is solved by the subject matter as defined in the claims.

The following figures illustrate the present invention.

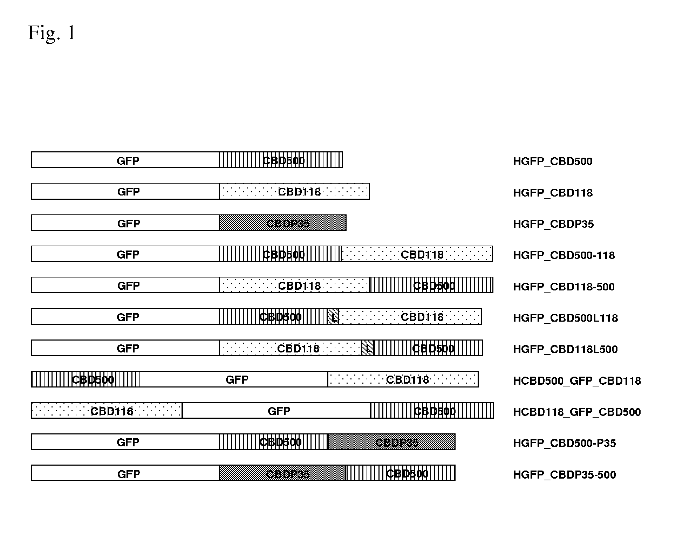

**Figure 1****:** Schematic representation of the GFP-double CBD fusion proteins against *Listeria* cells, as well as the GFP-single CBD constructs serving as references. GFP = Green fluorescent protein; CBD500, CBD118, CBDP35 = Cell wall binding domains of *Listeria* phage endolysins Ply500, Ply118, and PlyP35, respectively; L = linker region of the PlyPSA endolysin.

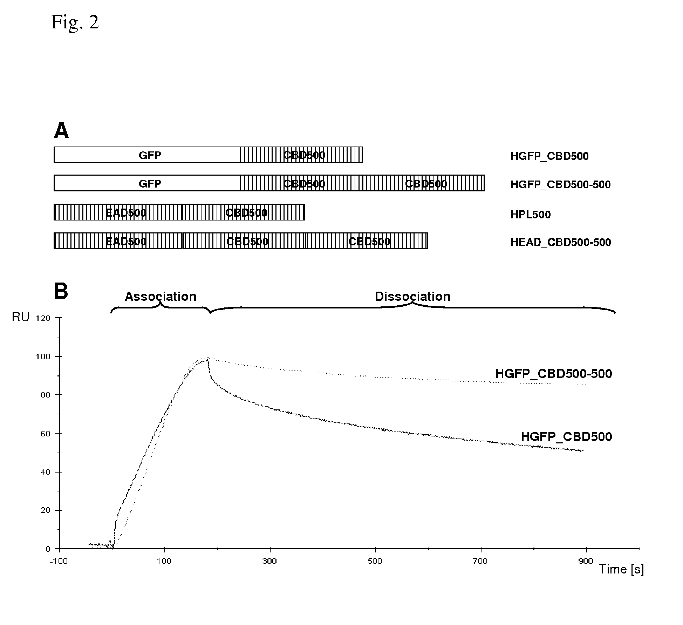

**Figure 2****:** Peptidoglycan binding proteins with duplicated CBD resulting in higher affinity due to reduced dissociation from the cell wall. **(A)** Schematic representations of double CBD500 fusion proteins as well as the respective single CBD500 constructs serving as references. GFP = Green fluorescent protein; EAD500 = Enzymatically active domain of *Listeria* phage endolysin Ply500; CBD500 = Cell wall binding domain of Ply500. **(B)** Overlay of the SPR sensograms of HGFP_CBD500 (black) and HGFP_CBD500-500 (grey), measured at a concentration of 50 nM. Association and dissociation phases are indicated. RU = relative response units.

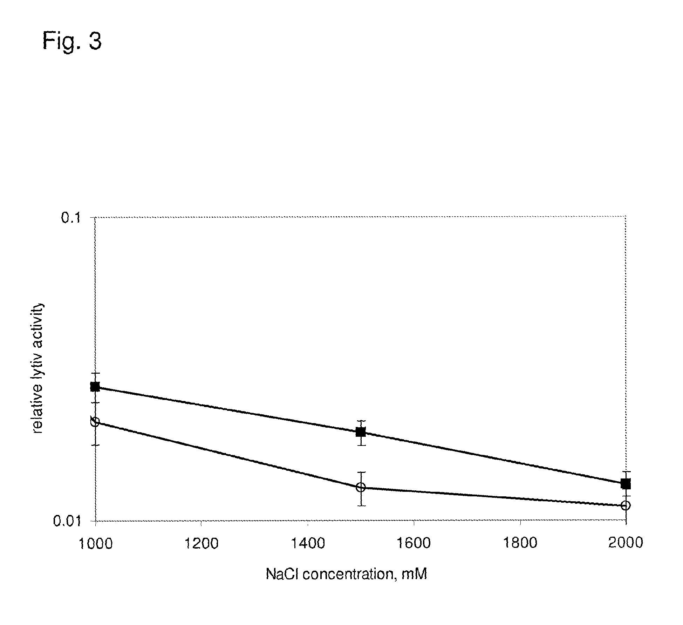

**Figure 3****:** Relative lytic activities of wild-type ply500 with N-terminal His-tag (open circles) and H_EAD_CBD500-500 (solid squares) against cells of *Listeria monocytogenes* WSLC 1042 measured with the photometric lysis assay at different NaCl concentrations. The optimum activity of wild-type ply500 at 200 mM NaCl corresponds to 1.0. All assays were carried out in triplicate.

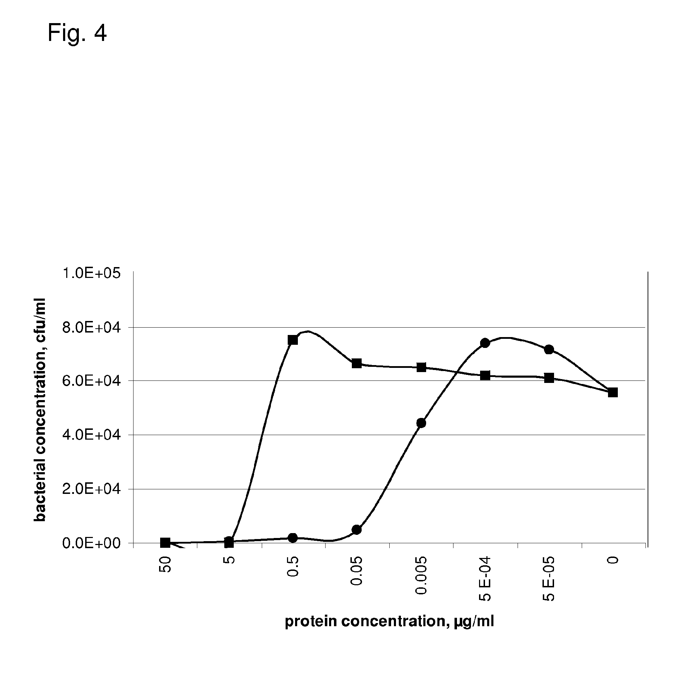

**Figure 4****:** Determination of the minimal bactericidal concentration (MBC) of peptidoglycan lysing enzymes against enterococci. The bacterial concentration of surviving cells of *Enterococcus faecalis* strain 17 is shown in dependence of the protein concentration of Fab25 VL (squares) or EADFab25_CBD25_CBD20 (circles) present in the cell lysis assay.

The term "peptidoglycan lysing enzyme" as used herein refers to an enzyme which is suitable to lyse bacterial cell walls. The enzyme comprises at least one of the following activities of which the "enzymatically active domains" (EADs) of the peptidoglycan lysing enzymes are constituted: endopeptidase, N-acetyl-muramoyl-L-alanine-amidase (amidase), N-acetylmuramidase (lysozyme or lytic transglycosylase) or N-acetyl-glucosaminidase. Either, the enzyme is phage or prophage encoded, the so-called "endolysins" or it is derived from related cell wall lysing enzymes coded by bacteria, the so-called "autolysins" or other bacterial peptiglycan lysing enzymes like bacteriocins, virulence factors or other antimicrobial polypeptides (e.g. lysostaphin, ALE-1 lysin, mutanolysin, enterolysin). In addition, the peptidoglycan lysing enzymes contain also regions which are enzymatically inactive, and bind to the cell wall of the host bacteria, the so-called CBDs (cell wall binding domains).

The term "peptidoglycan binding protein" as used herein refers to an artificially constructed bacterial cell binding protein which has none of the enzymatic activities described for the peptidoglycan lysing enzyme. The peptidoglycan binding protein comprises more than one CBD derived from a CBD. The peptidoglycan binding protein is constructed by shuffling of naturally occurring CBDs and/or by multiplication of naturally occurring.

The term "domain" as used herein refers to a subunit of a peptidoglycan lysing enzyme which is ascribed a specific function, and can also coincide with a structural or evolutionary conserved domain. Specific functions associated with a domain are for example bacterial peptidoglycan lysis or bacterial cell binding. The functional domains are sometimes also called "modules".

The term "CBD" as used herein refers to the cell wall binding domain of a peptidoglycan lysing enzyme, which is often found at the C-terminus of the protein. CBD domains have no enzymatic acitivity in terms of hydrolyzing the cell wall, but mediate binding of the peptidoglycan lysing enzyme to the bacterial cell wall. The term CBD as used herein describes a segment within a polypeptide chain which is derived from a naturally occurring peptidoglycan lysing enzyme.

The term "EAD" as used herein refers to the enzymatically active domain of a peptidoglycan lysing enzyme which is responsible for hydrolysis of the bacterial peptidoglycan. It contains at least one of the enzymatic activities described for a peptidoglycan lysing enzyme. The term EAD as used herein describes a segment within a polypeptide chain which is derived from a naturally occurring peptidoglycan lysing enzyme.

A "CHAP" domain (cysteine, histidine-dependent amidohydrolases/peptidases) is a region between 110 and 140 amino acids that is found in proteins from bacteria, bacteriphages, archaea and eukaryotes of the Trypanosomidae family. The proteins may function mainly in peptidoglycan hydrolysis. The CHAP domain is commonly associated with bacterial type SH3 domains and with several families of amidase domains. CHAP domain containing proteins may utilize a catalytic cysteine residue in a nucleophilic-attack mechanism. The CHAP domain contains two invariant amino acid residues, a cysteine and a histidine. These residues form part of the putative active site of CHAP domain containing proteins.

The term "ami" as used herein describes an enzymatically defined domain which exhibits amidase activity, i.e. it hydrolyzes the amide bond between N-acetylmuramine in the peptidoglycan backbone and the adjacent amino acid which is usually L-ala in the peptide linker. The amidase are often metal ion dependent for activity.

The term "SH3" domain which is sometimes also called Src homology 3 domain as used herein describes a small non-catalytic protein domain of about 60 amino acids which is characteristic for proteins which interact with other binding partners. It is identified via a proline-rich consensus motif. The SH3 domain is located within the CBD. SH3 domains found in peptidoglycan lysing enzymes are often of the SH3b or SH3_5 type.

The term "wild-type" refers to the naturally occurring form of a protein or a nucleic acid with respect to the sequence.

The term "shuffling" as used herein refers to the combination of different fragments of polypeptides from different wild-type enzymes into new chimaeric polypeptide constructs. In this context, the enzymes are preferentially peptidoglycan lysing enzymes, and the fragments are preferentially EADs and CBDs. Usually, the fragments are combined by molecular biological methods on nucleic acid level. Additional linker sequences may be introduced between the fragments for structural or cloning reasons.

One object of the present invention refers to peptidoglycan lysing enzymes that are composed of at least one EAD and at least two CBDs. Artificially created peptidoglycan lysing enzymes according to the invention exhibit new properties like an extended or altered binding range compared to naturally occurring proteins or an increased binding affinity to the bacterial cell wall or an increased or altered lytic activity or combinations thereof.

Another object of the present invention refers to peptidoglycan binding proteins that are composed of at least two CBDs. Artificially created peptidoglycan binding proteins according to the invention exhibit new properties like an extended or altered binding range compared to naturally occurring proteins or an increased binding affinity to the bacterial cell wall or both.

In the peptidoglycan lysing enzymes or peptidoglycan binding proteins according to the invention the at least two CBDs may be derived from two different peptidoglycan lysing enzymes (domain shuffling) or by multiplication of one CBD naturally occurring in an endolysin. If more than one EAD is present in the peptidoglycan lysing enzyme according to the invention the EADs may be derived from two different peptidoglycan lysing enzymes.

Meanwhile, a large number of peptidoglycan lysing proteins against different genera, species or strains of gram positive and gram negative bacteria is described in the art. The modular nature of the peptidoglycan lysing proteins, and the distinction between EAD and CBD is well known. Lots of conserved domains existing in peptidoglycan lysing proteins are characterized functionally, and their existence within a polypeptide or nucleotide sequence can be predicted by suitable computer programs which use respective protein or nucleic acid databases, e.g. CDD (Marchler-Bauer et al., 2005; Nucleic Acids Research, 33, D192-D196); Pfam (Finn et al., 2006, Nucleic Acids Research 34, D247-D251) or SMART (Schultz et al., 1998, Proc. Natl. Acad. Sci. USA 95, 5857-5864, Letunic et al., 2006, Nucleic Acids Res 34, D257-D260) or by binding assays with deletion mutants (Loessner et al., 2002, Mol. Microbiol., 44, 335-349). The artificial peptidoglycan lysing enzymes according to the invention are constructed by combining the desired enzymatic activity derived from an EAD with at least two CBDs for the cell binding activity using standard techniques for cloning and production of recombinant proteins as described in Sambrook et al. (Molecular cloning. A laboratory manual; 2nd ed. Cold Spring Harbor Laboratory Press 1989). The artificial peptidoglycan binding proteins according to the invention are constructed by combining at least two CBDs for the cell binding activity using standard techniques for cloning and production of recombinant proteins as described in Sambrook et al. (Molecular cloning. A laboratory manual; 2nd ed. Cold Spring Harbor Laboratory Press 1989). The at least two CBDs can derive from different peptidoglycan lysing enzymes which leads to shuffled chimaeric enzymes, or they can derive from a multiplication of CBDs from one naturally occurring enzyme, or combinations of both. Principally, all naturally occurring peptidoglycan lysing enzymes are potential candidates for the supply of EAD and CBD domains.

The peptidoglycan lysing enzymes preferably comprise at least one EAD selected from the group composed of Amidase_5 (bacteriophage peptidoglycan hydrolase, pfam05382), Amidase_2 (N-acetylmuramoyl-L-alanine amidase, pfam01510), Amidase_3 (N-acetylmuramoyl-L-alanine amidase, pfam01520), Transgly (transglycosylase, pfam00912), Peptidase_M23 (peptidase family M23, pfam01551), endolysin_autolysin (CD00737), Hydrolase_2 (cell wall hydrolase, pfam07486), CHAP (amidase, pfam05257), Transglycosylase (transglycosylase like domain, pfam06737), MtlB (membrane-bound lytic murein transglycosylase B, COG2951), MtlA (membrane-bound lytic murein transglycosylase A, COG2821), MtlE (membrane-bound lytic murein transglycosylase E, COG0741), bacteriophage_lambda_lysozyme (lysis of the bond between N-acetylmuramic acid and N-acetylglucosamine, CD00736), Peptidase_M74 (penicillin-insensitive murein endopeptidase, pfam03411), SLT (transglycosylase SLT, pfam01464), Lys (C-type lysozyme/alpha-lactalbumin family, pfam00062), COG5632 (N-acetylmuramoyl-L-alanine amidase, COG5632), MepA (murein endopeptidase, COG3770), COG1215 (glycosyltransferase, COG1215), AmiC (N-acetylmuramoyl-L-alanine amidase, COG0860), Spr (cell wall-associated hydrolase, COG0791), bacteriophage_T4-like_lysozyme (lysis of the bond between N-acetylmuramic acid and N-acetylglucosamine, cd00735), LT_GEWL (lytic transglycosylase (LT) and goose egg white lysozyme (GEWL) domain, cd00254), peptidase_S66 (LD-carboxypeptidase, pfam02016), Glyco_hydro_70 (glycosyl hydrolase family 70, pfam02324), Glyco_hydro_25 (glycosyl hydrolase familiy 25), VanY (D-alanyl-D-alanine carboxypeptidase, pfam02557), and LYZ2 (lysozyme subfamily 2, smart 00047).

The peptidoglycan lysing enzymes preferably comprise at least one CBD selected from the group composed of SH3_5 (bacterial SH3 domain, pfam08460), SH3_4 (bacterial SH3 domain, pfam06347), SH3_3 (bacterial SH3 domain, pfam08239), SH3b (bacterial SH3 domain homologue, smart00287), LysM (LysM domain found in a variety of enzymes involved in cell wall degradation, pfam01476 and cd00118), PG_binding_1 (putative peptidoglycan binding domain, pfam01471), PG_binding_2 (putative peptidoglycan binding domain, pfam08823), MtlA (peptidoglycan binding domain from murein degrading transglycosylase, pfam03462), Cpl-7 (C-terminal domain of Cpl-7 lysozyme, pfam08230), CW_binding_1 (putative cell wall binding repeat, pfam01473), LytB (putative cell wall-binding domain, COG2247), and LytE (LysM repeat, COG1388).

Preferably, the domains described above have amino acid lengths in the range of about 15 to about 250 amino acids, preferred are lengths in the range of about 20 to about 200 amino acids. As an example, about 15 to about 40 amino acid long domains are found in peptidoglycan binding domains like the LysM domain or the CW_binding_1 motif which is responsible for cholin binding. These small domains are often found as naturally repeated motifs also in wild-type cell wall lysing enzymes. These domains can be combined with additional CBDs from other cell wall lysing enzymes in order to create chimaeric shuffled artificial peptidoglycan lysing enzymes or peptidoglycan binding proteins.

Usually, complete EAD or CBD domains of peptidoglycan lysing enzymes are larger than the conserved domains described above. Preferentially, an EAD or CBD is in the range of about 50 residues to about 400 residues long. Each EAD and CBD contains at least one functional domain in order to exhibit their functions of peptidoglycan lysis or bacterial cell binding, but can also comprise more than one functional domain and additional sequence segments with unknown function. EAD and CBD domains of peptidoglycan binding enzymes are not always defined by the conserved domains described above. There are also peptidoglycan binding enzymes known (e.g. Ply118) which bind and lyse bacterial cells although none of the above described conserved domains is found. Whether potential domains function as an EAD or CBD can be tested with suitable functional assays (e.g. photometric lysis assay, plate lysis assay or determination of minimal bactericidal concentration (MBC) for peptidoglycan lysis (EAD), and cell binding assay, fluorescence microscopy or determination of binding affinity for cell binding (CBD)). The domain borders of EADs and CBDs can be defined by local alignment search tools (e.g. BLAST at the NCBI, Altschul et al., 1997, Nucleic Acids Res. 17, 3389-3402) which find regions of local similarity between sequences. In addition, a multitude of peptidoglycan lysing enzymes are already described with respect to their EAD and CBD domains.

Preferably, the peptidoglycan lysing enzymes and peptidoglycan binding proteins of the present invention are composed of EADs and CBDs derived from wild-type peptidoglycan lysing enzymes selected from the group consisting of Ply500, Ply511, Ply118, Ply100, PlyP40, Ply3626, phiLM4 endolysin, PlyCD119, PlyPSAa, Ply21, PlyBA, Ply12, PlyP35, PlyPH, PlyL, PlyB, phi11 endolysin, phi MR11 endolysin, phi12 endolysin, S. *aureus* phage PVL amidase , plypitti26, ΦSA2usa endolysin, endolysin of *Staphylococcus warneri* M phage ΦWMY PlyGBS, B30 endolysin, Cpl-1, Cpl-7, Cpl-9, PlyG, PlyC, pal amidase, Fab25, Fab20, endolysins from the *Enterococcus faecalis* V583 prophage, lysostaphin, phage PL-1 amidase, *S. capitis* ALE-1 endopeptidase, mutanolysin (N-acetylmuramidase of *Streptomyces globisporus* ATCC 21553), enterolysin A (cell wall degrading bacteriocin from *Enterococcus faecalis* LMG 2333), LysK, LytM, Ami autolysin from *L. monocytogenes,* endolysins of the *Pseudomonas aeruginosa* phages ΦKZ and EL, T4 lysozyme, gp61 muramidase, and STM0016 muramidase.

The fragments derived from naturally occurring peptidoglycan lysing enzymes in order to construct the enzymes and proteins according to the invention may not combine the mere sequence segments determined from the prediction of the conserved functional domains as described above, but preferably add suitable linker sequences which connect the different functional modules. The linker sequences can be derived from the wild-type sequences in neighbourhood to the defined functional domains or can be external suitable linker sequences known from the art. A suitable linker is for example the short domain linker with the sequence AAKNPN or TGKTVAAKNPNRHS (SEQ IDs No: 61 and 11) from the *Listeria* endolysin PlyPSA (Korndörfer et al., 2006, J. Mol. Biol., 364, 678-689) defined from the x-ray structure. Polyglycine linkers are also known in the art to serve as flexible domain linkers. Preferred linkers are also glycine and alanine rich linkers. Specific sequences for glycine and alanine rich linkers are given as SEQ ID NO:63, 64 and 65. Preferred are also proline and threonine rich sequences which occur as natural linkers, e.g. in enterolysin A SEQ ID NO:66. Proline and threonine rich linker sequences can be described by the consensus motif (PT)ₓP or (PT)ₓT, where x stands for an integer in the range of 1 to 10. Another linker possibility are the so-called "junction zones" between EADs and CBDs described in Croux et al. (1993, Molec. Microbiol., 9, 1019-1025. A skilled person knows several methods how to predict a suitable boundary for a functional domain to be taken out of a wild-type enzyme, e. g. secondary structure prediction, prediction of domain linkers, inspection of 3D-models of proteins or inspection of domain linkers and boundaries in highly resolved X-ray and NMR structures of proteins. Suitable methods are for example described in Garnier et al., 1996, Methods in Enzymology 266, 540-553; Miyazaki et al., 2002, J. Struct. Funct. Genomics, 15, 37-51; George und Heringa, 2003, Protein Eng. 15, 871-879; Bae et al., 2005, Bioinformatics, 21, 2264-2270, Altschul et al., 1997, Nucleic Acids Res. 17, 3389-3402; Schwede et al., 2003, Nucleic Acids Research 31, 3381-3385. Lund et al, CPHmodels 2.0: X3M a Computer Program to Extract 3D Models. Abstract at the CASP5 conferenceA102, 2002. The length of polypeptide linker between EAD and CBD domains or between CBD and CBD domains are in the range of about 5 to about 150 amino acid residues, preferentially of about 6 to about 60 amino acid residues.

Preferably, the order for the combination of EAD and CBDs in the peptidoglycan lysing enzymes according to the invention is EAD-CBD1-CBD2(-CBDN, N = 3 or more) from the N-terminus to the C-terminus. Preferred are also variants where an at least second EAD is added next to the EAD at the N-terminus or at the C-terminus. Preferred are also variants where the at least two CBDs are positioned at the N-terminus or at the N- and C-terminus with the EADs positioned in the middle. In addition, marker sequences or tags can be included, which can both be positioned N-terminal, C-terminal or in the middle, but especially preferred at the N-terminus.

Peptidoglycan lysing enzymes and peptidoglycan binding proteins according to the invention exhibit new properties compared to the wild-type enzymes from which they are derived.

The binding range of a peptidoglycan lysing enzyme or peptidoglycan binding protein determines the bacterial host range which is recognized. Most of the naturally occurring peptidoglycan lysing enzymes exhibit a relatively narrow host range. For technical application of the peptidoglycan lysing enzymes or peptidoglycan binding proteins it is often advantageous to extend the host range of the proteins so that an increased number of bacterial strains or species can be killed, captured or detected depending on the respective application. An extended host range comprised within one protein avoids the use of two or more proteins for the same application which has the advantages of reduced costs for protein production, reduced effort to optimize conditions for different proteins, simpler medical approval proceedings, and reduced immunogenicity. An extended host range which combines for example *Staphylococci* and *Enterococci* is useful in the therapy or prevention of nosocomial infections where multiresistant strains of both genera are an increasing problem. An extended host range is also useful in bacterial detection or a method to remove harmful bacteria from food. For example, pathogenic strains are found within all serovars of *Listeria.* None of the naturally occurring *Listeria* endolysins, however, is able to lyse cells from all serovars. A peptidoglycan lysing enzyme combining more than one CBD according to the invention is able to lyse all serovars. A host range which is not extended, but somehow altered compared to naturally occurring proteins, may be useful for applications which need tailored proteins for a given set of bacterial cells to be lysed, captured or detected. The binding range of peptidoglycan lysing enzymes or peptidoglycan binding proteins can be determined with assays known from the art or with the plate lysis assay, photometric lysis assay, binding assay or fluorescence microscopy described in the examples.

An increased binding affinity of peptidoglycan lysing enzymes or peptidoglycan binding proteins compared to wild-type proteins helps to reduce the amount of protein needed for any technical application which relies on the binding of the bacterial cells like cell lysis, cell capture, and detection. This reduces costs and minimizes immunological reactions and potential side effects in therapeutical applications. In applications relying on bacterial cell capture, the assays are less sensitive for washing steps which decreases background signals, incubation times can be reduced, and detection assays are more sensitive. An increased binding affinity can be measured with assays known from the art or with the surface plasmon resonance analysis or the assay for determination of the minimal bactericidal concentration described in the examples.

An increased lytic activity of peptidoglycan lysing enzymes compared to wild-type enzymes is useful in all applications relying on the lysis of bacterial cells like protection and therapy of infections, sanitation, cell lysis as an initial step in bacterial detection, or removal of pathogenic bacteria from food, feed, cosmetics etc.. The amount of protein needed for the respective application is reduced compared to the wild-type protein which reduces costs and minimizes immunological reactions and potential side effects in therapeutical applications. An altered lytic activity compared to wild-type could be for example a different pH-optimum of the artificial enzyme or a higher lysis activity at other buffer compositions (e.g. high ionic strength, activity in the presence of organic solvents, activity in the presence of specific ions). This also includes a higher activity in specific samples like blood, human serum, or other medical samples. A pH-optimum of an artificial enzyme which is shifted to lower pH is for example interesting for an application of the artificial enzyme in food industry as food products or intermediate products in food processing often have a low pH-value, e.g. in dairy farming. Enzyme function under high salt concentration is also important in food industry, e.g. in cheese production. An increased or altered lytic activity can be determined with assays known from the art or with the plate lysis assay, photometric lysis assay, or the assay for determination of the minimal bactericidal concentration described in the examples.

In one aspect the present invention relates to artificial peptidoglycan lysing enzymes and peptidoglycan binding proteins which can be used to lyse, capture and/or detect *Listeria* bacteria. The inventors combined domains of the *Listeria* endolysins ply500 (SEQ ID NO:1), ply118 (SEQ ID NO:3) and plyP35 (SEQ ID NO:5) using the method described above in order to create artificial peptidoglycan lysing enzymes and peptidoglycan binding proteins which exhibit new properties compared to the wild-type enzymes. Ply500 comprises a conserved D-alanyl-D-alanine carboxypeptidase (VanY; pfam02557) domain as an EAD. The CBD of ply500 begins with an amino acid in the range of about H133 to Q150 and ends with K289. For Ply118 no conserved domains were found within the amino acid sequence. From sequence alignments with homologous peptidoglycan lysing enzymes, however, it was derived that the CBD of ply118 begins with an amino acid in the range of about D90 to K180 and ends with amino acid K289. Preferred N-terminal starting amino acid residues for CBD118 are D90, K100, G127, S151, N161 or K180. PlyP35 also comprises a conserved D-alanyl-D-alanine carboxypeptidase (VanY; pfam02557) domain as an EAD. The CBD of plyP35 begins with an amino acid in the range of about P130 to N156 and ends with an amino acid in the range of Y281 to K291. Preferred N-terminal starting amino acid residues for CBDP35 are P130, A134, K143 and N156. Preferred C-terminal amino acids for CBDP35 are Y281, L286, and K291.

Preferred peptidoglycan binding proteins according to the invention are CBD500-118 (SEQ ID NO:7) which comprise the CBD of ply500 (amino acid H133 to K289) in the N-terminal position and the CBD of ply118 (amino acids D90 to I281) in the C-terminal position connected without an additional linker sequence, and CBD500L118 (SEQ ID NO:9) which comprises the CBD of ply500 (amino acids Q150 to K289) and the CBD of ply118 (amino acids K100 to I281) with a linker (L) connecting the two domains. The domain linker in this case is the plyPSA linker region with the amino acid sequence TGKTVAAKNPNRHS (SEQ ID NO:11), which correspond to amino acids 173 to 186 from the *Listeria* endolysin PlyPSA (Korndörfer et al., 2006, J. Mol. Biol., 364, 678-689).

Further preferred embodiments according to the invention are the artificial peptidoglycan binding proteins CBD118-500 (SEQ ID NO:13) which comprise the CBD of ply500 (amino acid H133 to K289) in N-terminal position and the CBD of ply118 (amino acids D90 to I281) in C-terminal position connected without an additional linker sequence, and CBD118L500 (SEQ ID NO:15) which comprises the CBD of ply118 (amino acids K100 to I281) and the CBD of ply500 (amino acids Q150 to K289) with a linker (L) connecting the two domains. The domain linker in this case is the plyPSA linker region (SEQ ID NO: 11).

The peptidoglycan binding proteins CBD500-118, CBD500L118, CBD118-500, and CBD118L500 all exhibit altered cell binding activities with respect to host range and binding activity compared to the wild-type enzymes ply500 and ply118 from which the CBD domains were derived. The cell binding activity of the constructs CBD500L118 and CBD118L500 discloses that a linker between two domains helps to achieve an extended host range which combines the binding specificities of the wt-enzymes.

Further preferred peptidoglycan binding proteins according to the present invention are CBD500-P35 (SEQ ID NO:17) which comprises the CBD of ply500 (amino acid Q150 to K289) in N-terminal position and the CBD of plyP35 (amino acids P130 to K291) in C-terminal position, and the protein with the inverse orientation of CBDs CBDP35-500 (SEQ ID NO:19) which comprises the CBD of plyP35 (amino acids P130 to K291) at the N-terminus, and the CBD of ply500 (amino acids Q150 to K289) at the C-terminus. In this case, the CBDs are not connected by an external linker sequence, as the fragment for the CBD of plyP35 includes the internal domain linker of plyP35.

The artificial peptidoglycan binding proteins CBD500-P35 and CBDP35-500 both exhibit an extended host range compared to the wild-type enzymes ply500 and plyP35 from which the CBD domains were derived. Both chimaeric proteins combine the different binding specificities of the two wt-enzymes within one protein. The orientation of the CBDs makes no difference in this case. Both CBDs can be positioned N-terminally as well as C-terminally.

Further preferred peptidoglycan binding proteins according to the present invention are CBD500-500 (SEQ ID NO:21) which exhibits a duplication of the CBD of ply500 (amino acid Q150 to K289), and the artificial peptidoglycan lysing enzyme EAD-CBD500-500 (SEQ ID NO:23) which exhibits a duplication of the naturally occurring CBD in ply500.

Both proteins according to the invention exhibit a higher binding affinity to *Listeria* cells compared to the wild-type, and EAD-CBD500-500 in addition exhibits an increased lysis activity under high salt conditions compared to ply500.

In another aspect the present invention relates to artificial peptidoglycan lysing enzymes which can be used to lyse, capture or detect *Enterococcus* bacteria. The inventors combined domains of the *Enterococcus* endolysins Fab25VL (SEQ ID NO:25) and Fab20VL (SEQ ID NO:27) using the method described above in order to create artificial peptidoglycan lysing enzymes and peptidoglycan binding proteins which exhibit new properties compared to the wild-type enzymes.

Fab25VL is an endolysin of 317 amino acids length which preferentially binds and lysis bacteria from the species *E. faecium,* but also some strains from the species *E. faecalis.* The N-terminally positioned EAD of Fab25VL (amino acids 1 to 167) exhibits a conserved Amidase_2 domain which functions as an N-acetylmuramoyl-L-alanine-amidase. The CBD comprises the amino acids 200 to 317. Between the two domains, a linker region comprising amino acids 168 to 199 is observed. Fab20VL is an endolysin of 365 amino acids length which preferentially binds and lysis bacteria from the species *E. faecalis.* The N-terminally positioned EAD (amino acids 40 to 194) of Fab20VL also exhibits a conserved Amidase_2 domains which functions as an N-acetylmuramoyl-L-alanine-amidase. The CBD of Fab20VL (amino acids 215 to 365) comprises a bacterial SH3 domain in its C-terminal part which shows homologies to peptidoglycan lysing enzymes from *Staphylococcus* and *Streptococcus* phages. An N-terminally truncated variant of Fab20VL with a deletion of amino acids 1 to 19 - Fab20K (SEQ ID NO:29) was constructed which showed better expression in E. coli compared to Fab20VL.

A preferred peptidoglycan lysing enzyme according to the present invention is SEQ ID NO:31 which combines the EAD and CBD of Fab25 (amino acids 1 to 317) with the CBD of Fab20VL (amino acids 215 to 365) and a short linker segment derived from Fab20VL (amino acids 200 to 214). The construct is denoted EADFab25_CBD25_CBD20. EADFab25_CBD25_CBD20 exhibits an extended host range, an increased lysis activity with respect to living cells and an increased binding affinity as new features compared to the wt-enzymes.

Further preferred peptidoglycan binding enzymes according to the present invention are composed of at least two EADs and at least two CBD capable of detecting and binding Staphylococcus bacteria.

Preferably the peptidoglycan lysing proteins according to the present invention comprise tags such as His-tag (Nieba et al., 1997, Anal. Biochem., 252, 217-228), Strep-tag (Voss & Skerra, 1997, Protein Eng., 10, 975-982), Avi-tag (US 5,723,584; US 5,874239), Myc-tag (Evan et al., Mol&Cell Biol, 5, 3610-3616), GST-tag (Peng et al. 1993, Protein Expr. Purif., 412, 95-100), JS-tag (WO 2008/077397), cystein-tag (EP1399551, SEQ IDs No:6 and 7), HA-tag (amino acid sequence EQKLISEEDL), FLAG-tag (Hopp et al., Bio/Technology. 1988;6:1204-1210) or other tags known in the art. Preferably the tag is coupled to the C-terminus or the N-terminus of the peptidoglycan lysing protein according to the invention, most preferably to the N-terminus. Tags can be useful to facilitate expression and/or purification of the peptidoglycan lysing protein, to immobilize the peptidoglycan lysing protein according to the invention to a surface or to serve as a marker for detection of the peptidoglycan lysing protein, e.g. by antibody binding in different ELISA assay formats.

Preferably the peptidoglycan lysing proteins according to the invention comprise marker or label moieties such as biotin, streptavidin, GFP (green fluorescent protein), YFP (yellow fluorescent protein), cyan fluorescent protein, RedStar protein or other fluorescent markers, alkaline phosphatase, horse radish peroxidase, immuno-gold labels, spin labels or other markers and labels known in the art. The markers can be attached in a recombinant way if they are of polypeptide nature or post-translationally by chemical modification of the polypeptide residues. The markers or labels are especially useful to detect the peptidoglycan lysing proteins according to the invention when they are used in diagnostics.

Further preferred peptidoglycan lysing proteins are the constructs HGFP-CBD118-500 (SEQ ID NO:33), HGFP-CBD500-118 (SEQ ID NO:35), HGFP-CBD118L500 (SEQ ID NO:37), HGFP-CBD500L118 (SEQ ID NO:39), HGFP-CBD500-P35 (SEQ ID NO:41), HGFP-CBDP35-500 (SEQ ID NO:43), HCBD500-GFP-CBD118 (SEQ ID NO:45), HCBD118-GFP-CBD500 (SEQ ID NO:47), HGFP-CBD500-500 (SEQ ID NO:49), and HEAD-CBD500-500 (SEQ ID NO:51). H denotes a his-tag including six histidines (SEQ ID NO:53), and GFP denotes green fluorescent protein introduced as a fluorescent marker (SEQ ID NO:55).

All of the above mentioned fusion constructs including a his-tag and a GFP marker show *Listeria* cell binding activity and the peptidoglycan lysing enzymes additionally lysis activity. The constructs HCBD500-GFP-CBD118 and HCBD118-GFP-CBD500 however show that an N-terminal position of the GFP marker is preferred compared to a positioning between the two CBD domains, as the cell binding activity, especially the cell binding activity of CBD118, is reduced in these constructs.

In summary, the object of the present invention is to alter and improve the properties of wild-type peptidoglycan lysing enzymes by artificial combination of functional domains by shuffling or by multiplication of naturally occurring domains. Peptidoglycan lysing enzymes according to the invention are composed of at least one EAD and at least two CBDs in order to extend the binding range of naturally occurring proteins, and/or to increase the binding affinity to the bacterial cell wall, and/or to increase or modify the lytic activity. Peptidoglycan binding proteins according to the invention are composed of at least two CBDs in order to extend the binding range of naturally occurring proteins, and/or to increase the binding affinity to the bacterial cell wall. In the polypeptides according to the invention the at least two CBDs are derived from two different peptidoglycan lysing enzymes (domain shuffling) or by multiplication of naturally occurring CBDs.

Preferred is a recombinant polypeptide as depicted in SEQ ID NO: 7, 9, 13, 15, 17, 19, 21, 23, 31, 33, 35, 37, 39, 41, 43, 45, 47, 49, 51, 71, 73, 75, 77, 79, 81, 83, 85, 87, 89, 91, 93, 95, 97, 99, 101.

In a further aspect the present invention relates to a nucleic acid molecule comprising a nucleotide sequence encoding the polypeptides according to the present invention.

Preferred is a nucleic acid molecule, wherein the nucleic acid molecule comprises a nucleotide sequence as depicted in SEQ ID NO: 8, 10, 14, 16, 18, 20, 22, 24, 32, 34, 36, 38, 40, 42, 44, 46, 48, 50, 52, 72, 74, 76, 78, 80, 82, 84, 86, 88, 90, 92, 94, 96, 98, 100, 102.

In a further aspect the present invention relates to a vector comprising a nucleic acid sequence of the invention. Preferably, said vector provides for the expression of said polypeptide of the invention in a suitable host cell. Said host cell may be selected due to mere biotechnological reasons, e.g. yield, solubility, costs, etc. but may be also selected from a medical point of view, e.g. a non-pathological bacteria or yeast, human cells, if said cells are to be administered to a subject. Said vector may provide for the constitutive or inducible expression of said polypeptides according to the present invention.

In a further aspect of the present invention the above mentioned polypeptides and/or cells are employed in a method for the treatment or prophylaxis of bacterial infections in a subject, in particular for the treatment or prophylaxis of infections caused by gram positive bacteria like staphylococci (e. g. S. *aureus, S. aureus (MRSA), S. epidermidis, S. haemolyticus, S. simulans, S. saprophyticus, S. chromogenes, S. hyicus, S. warneri* and/or *S. xylosus*), enterococci (*e.g. Enterococcus faecium, E. faecium* (VRE) *Enterococcus faecalis*), streptococci (*Streptococcus pyogenes, S. pneumoniae, S. mutans, S. uberis, S. agalactiae, S. dysgalactiae, Streptococci of the Lancefield groups A, B, C*), clostridia (e. g. *C. perfringens, C. difficile, C. tetani, C. botulinum, C. tyrobutyricum*), bacilli (e. g. *Bacillus anthracis, B. cereus), Listeria* (e.g.*L. monocytogenes, L. innocua*), *Haemophilus influenza, Corynebacterium diphteriae, Propionibacterium acne,* mycobacteria (e.g. *Mycobacterium tuberculosis, M. bovis).* Alternatively, the polypeptides and/or cells according to the invention are employed in a method for the treatment or prophylaxis of bacterial infections in a subject, in particular for the treatment or prophylaxis of infections caused by gram negative bacteria of bacterial groups, families, genera or species comprising strains pathogenic for humans or animals like *Enterobacteriaceae* (*Escherichia,* especially *E. coli, Salmonella, Shigella, Citrobacter, Edwardsiella, Enterobacter, Hafnia, Klebsiella, especially K. pneumoniae, Morganella, Proteus, Providencia, Serratia, Yersinia*), *Pseudomonadaceae* (*Pseudomonas, especially P. aeruginosa, Burkholderia, Stenotrophomonas, Shewanella, Sphingomonas, Comamonas*), *Neisseria, Moraxella, Vibrio, Aeromonas, Brucella, Francisella, Bordetella, Legionella, Bartonella, Coxiella, Haemophilus, Pasteurella, Mannheimia, Actinobacillus, Gardnerella, Spirochaetaceae* (*Treponema and Borrelia*), *Leptospiraceae, Campylobacter, Helicobacter, Spirillum, Streptobacillus, Bacteroidaceae (Bacteroides, Fusobacterium, Prevotella, Porphyromonas), Acinetobacter,* especially *A. baumanii.*

Said subject may be a human subject or an animal, in particular animals used in livestock farming and/or dairy farming such as cattle. Said method of treatment encompasses the application of said polypeptide of the present invention to the site of infection or site to be prophylactically treated against infection in a sufficient amount.

In particular said method of treatment may be for the treatment or prophylaxis of infections of the skin, of soft tissues, the respiratory system, the lung, the digestive tract, the eye, the ear, the teeth, the nasopharynx, the mouth, the bones, the vagina, of wounds of bacteremia and/or endocarditis.

In a further preferred embodiment a polypeptide according to the present invention is used in a method of treatment (or prophylaxis) of staphylococcal infections in animals, in particular in livestock and dairy cattle. In particular a polypeptide of the present application is suitable for use in methods of treatment (or prophylaxis) of bovine mastitis, in particular of bovine mastitis caused by *S. aureus, S. epidermidis, S. simulans, S. chromogenes, S. hyicus, S. warneri* and *S. xylosus.*

Furthermore, a polypeptide of the present invention may be used prophylactically as sanitizing agent, in particular before or after surgery, or for example during hemodialysis. Similarly, premature infants and immunocompromised persons, or those subjects with need for prosthetic devices can be treated with a polypeptide of the present invention, either prophylactically or during acute infection. In the same context, nosocomial infections, especially by antibiotic resistant strains like *Staphylococcus aureus (MRSA), Enterococcus faecium (VRE), Pseudomonas aeruginosa* (FQRP) or antibiotica resistant *Clostridium difficile* may be treated prophylactically or during acute phase with a polypeptide of the present invention. In this embodiment, a polypeptide of the present invention may be used as a disinfectant also in combination with other ingredients useful in a disinfecting solution like detergents, tensids, solvents, antibiotics, lanthibiotics, or bacteriocins.

In a particularly preferred embodiment a polypeptide of the present invention is used for medical treatment, if the infection to be treated (or prevented) is caused by multiresistant bacterial strains, in particular by strains resistant against one or more of the following antibiotics: penicillin, streptomycin, tetracycline, methicillin, cephalothin, gentamicin, cefotaxime, cephalosporin, vancomycin, linezolid, ceftazidime, imipenem or daptomycin. Furthermore, a polypeptide of the present invention can be used in methods of treatment by administering them in combination with conventional antibacterial agents, such as antibiotics, lanthibiotics, bacteriocins other endolysins, etc.

The dosage and route of administration used in a method of treatment (or prophylaxis) according to the present invention depends on the specific disease/site of infection to be treated. The route of administration may be for example in particular embodiments oral, topical, nasopharyngeal, parenteral, intravenous, rectal or any other route of administration.

For application of a polypeptide of the present invention to a site of infection (or site endangered to be infected) a polypeptide of the present invention may be formulated in such manner that the peptidoglycan lysing enzyme is protected from environmental influences such as proteases, oxidation, immune response etc., until it reaches the site of infection.

Therefore, a polypeptide of the present invention may be formulated as capsule, dragee, pill, suppository, injectable solution or any other medical reasonable galenic formulation. In some embodiments these galenic formulation may comprise suitable carriers, stabilizers, flavourings, buffers or other suitable reagents.

For example, for topical application a polypeptide of the present invention may be administered by way of a lotion or plaster.

For nasopharyngeal application a polypeptide according to the present invention may be formulated in saline in order to be applied via a spray to the nose.

For treatment of the intestine, for example in bovine mastitis, suppository formulation can be envisioned. Alternatively, oral administration may be considered. In this case, the polypeptide of the present invention has to be protected from the harsh digestive environment until the site of infection is reached. This can be accomplished for example by using bacteria as carrier, which survive the initial steps of digestion in the stomach and which secret later on a polypeptide of the present invention into the intestinal environment.

All medical applications rely on the effect of the polypeptides of the present invention to lyse specifically and immediately pathogenic bacteria when encountered. This has an immediate impact on the health status of the treated subject by providing a reduction in pathogenic bacteria and bacterial load and simultaneously relieves the immune system. Thus, the major task a person skilled in the art faces is to formulate the polypeptides of the present invention accurately for the respective disease to be treated. For this purpose usually the same galenic formulation as employed for conventional medicaments for these applications can be used.

In a further aspect of the present invention the above mentioned polypeptides and/or cells are a component of a pharmaceutical composition, which optionally comprises a carrier substance.

In an even further aspect the polypeptides and/or cells are part of a cosmetics composition. As mentioned above, several bacterial species can cause irritations on environmentally exposed surfaces of the patient's body such as the skin. In order to prevent such irritations or in order to eliminate minor manifestations of said bacterial pathogens, special cosmetic preparations may be employed, which comprise sufficient amounts of polypeptides of the present invention in order to lyse already existing or freshly settling pathogenic bacteria.

In a further aspect the present invention relates to the use of said polypeptides according to the present invention in foodstuff, on food processing equipment, in food processing plants, on surfaces coming into contact with foodstuff such as shelves and food deposit areas and in all other situations, where pathogenic, facultative pathogenic or other undesirable bacteria can potentially infest food material.

A further aspect the present invention relates to the use of said polypeptides according to the present invention in diagnostics of bacterial infections. In this aspect the polypeptides according to the invention are used as a tool to specifically lyse pathogenic bacteria. The lysis of the bacterial cells by the polypeptides according to the present invention can be supported by the addition of detergents like Triton X-100 or other additives which weaken the bacterial cell envelope like polymyxin B. Specific cell lysis is needed as an initial step for subsequent specific detection of bacteria using nucleic acid based methods like PCR, nucleic acid hybridization or NASBA (Nucleic Acid Sequence Based Amplification), immunological methods like IMS, immunfluorescence or ELISA techniques, or other methods relying on the cellular content of the bacterial cells like enzymatic assays using proteins specific for distinct bacterial groups or species (e.g. β-galactosidase for enterobacteria, coagulase for coagulase positive strains).

Another aspect of the present invention is the use of a peptidoglycan binding protein according to the present invention for binding, enrichment, removing, capture and detection of pathogenic of otherwise undesirable bacteria from a sample. A sample with regard to the methods according to the present invention is any material supposed to or containing bacteria, whereas the bacteria are a target for detection, binding, enrichment, removing or capture. Samples can be e.g. food or feed materials, surface materials or human or veterinary diagnostic probes. Bacteria detection is performed via detection of markers attached to the peptidoglycan binding protein according to the present invention or by detection of said protein itself, e.g. by immunological methods like ELISA. For the methods according to the present invention the peptidoglycan binding proteins according to the present invention may be immobilised on suitable supporting structures, e.g., microtiter plates, test stripes, slides, wafers, filter materials, reaction tubes, magnetic, glass or latex particles, pipette tips or flow-through cell chambers. The supporting structures may consist of, e.g., polystyrene, polypropylene, polycarbonate, PMMA, cellulose acetate, nitrocellulose, glass, silicium wafer, latex. The immobilisation may be accomplished by adsorption, by covalent binding or by further proteins, wherein the covalent binding is preferred. It is relevant that immobilisation is a functional one, that is, said peptidoglycan binding proteins exhibit structures accessible for bacteria although they are bound to the support material.

### Examples

### Example 1. DNA techniques and cloning procedures

DNA techniques and cloning procedures according to Sambrook et al. (Molecular cloning. A laboratory manual; 2nd ed. Cold Spring Harbor Laboratory Press 1989) were employed for construction of plasmids coding for endolysin based fusion proteins. The plasmid pQE-30 (QIAGEN) and its derivatives pHGFP, pHGFP_CBD118, pHGFP_CBD500 (Loessner et al. 2002), and pHEADPSA (Korndoerfer et al. 2006), were used as vector backbones for the construction of plasmids coding for N-terminally 6xHis-tagged artificial fusion proteins (H stands for His-tag). Restriction sites needed for insertion of the fragments into the plasmids were introduced via the primers. Double CBD fusion constructs were created either by separate amplification of the two CBD fragments and subsequent ligation or alternatively by fusing the two fragments via the PCR based Gene Splicing by Overlap Extension (SOE PCR) method (Horton et al. 1990). CBD118 (SEQ ID NO:57) and CBD500 (SEQ ID NO:58) coding fragments were ligated via *Eco*RI/*Mun*I sites in both orientations and then inserted into *Sac*I/*Sal*I sites of pHGFP, yielding pHGFP_CBD118-500 and pHGFP_CBD500-118. The plasmids pHGFP_CBDP35-500 and pHGFP_CBD500-P35 were created the same way. In case of pHGFP_CBD118L500 and pHGFP_CBD500L118, the fragment coding for the PlyPSA linker was introduced between the two CBDs by SOE PCR before insertion into pHGFP. As for pHCBD500_GFP_118 and pHCBD118_GFP_500, the 5' CBD and the GFP fragments were first fused via *Kpn*I sites or by SOE PCR, and then ligated into *Bam*HI/*Sac*I sites of pHGFP_CBD118 and pHGFP_CBD500, respectively, replacing the mere GFP fragments of these plasmids. For construction of pHGFP_CBD500-500, the CBD500 fragment was cloned into the *Sac*I site of pHGFP_CBD500, resulting in a duplication of CBD500. pHEAD_CBD500-500 was created by inserting the complete *ply*500 gene into *Bam*HI/*Sac*I sites of pHGFP_CBD500, replacing the GFP fragment. For all constructs, all stop codons except the ones at the 3' ends were omitted to allow genetic fusions. TAA was generally introduced as stop codon at the 3' ends. All constructs were verified by nucleotide sequencing.

### Example 2. Overexpression and purification of His-tagged recombinant proteins

Overexpression of His-tagged (abbreviated in the respective constructs by "H") fusion proteins was performed in *E. coli* XL1-Blue MRF' (Stratagene). The respective strains were grown in modified LB medium (15 g/l tryptose, 8 g/l yeast extract, 5 g/l NaCl) containing 100 µg/ml ampicillin and 30 µg/ml tetracycline for plasmid selection at 30 °C, with 0.1 to 1 mM IPTG added as inducer once an OD₆₀₀ of 0.5 was reached. After further incubation at 30 °C for 4 h cultures producing proteins that contain a GFP domain were stored overnight at 4°C before harvesting and resuspension in 5 ml buffer A (500 mM NaCl, 50 mM Na₂HPO₄, 5 mM imidazole, 0.1 % Tween 20, pH 8.0) per 250 ml culture. If no GFP was present, cells were pelleted 4 h after induction. The cells were disrupted by two passages through a French Press 20K cell (SLM Aminco) at 100 MPa, and cell debris was removed by centrifugation and filtration (0.2 µM PES membrane, Millipore).

The 6xHis-tagged target proteins in the raw extracts were purified by Immobilized Metal Affinity Chromatography (IMAC) with Ni-NTA Superflow resin (QIAGEN) using Micro Biospin columns (BIORAD). Buffer B (500 mM NaCl, 50 mM Na₂HPO₄, 250 mM imidazole, 0.1 % Tween 20, pH 8.0) served as elution buffer. The purified proteins were dialyzed against two changes of dialysis Buffer (100 mM NaCl, 50 mM NaH₂PO₄, 0.005% to 0.1% Tween 20, pH 8.0), filtered (0.2 µM PES membrane, Millipore), and stored at -20 °C after addition of 50 % (v/v) of glycerol. For each protein, the course of overexpression and purification was analyzed by SDS-PAGE and the protein concentration was determined spectrophotometrically (NanoDrop ND-1000 Spectrophotometer).

### Example 3. Binding assays and fluorescence microscopy

The binding properties of GFP-CBD fusion proteins were examined by binding using a representative set of *Listeria* strains (table 1) of all species and serovars. Late log phase cells of each strain in PBST buffer (50 mM NaH₂PO₄, 120 mM NaCl, pH 8.0, 0.01% Tween 20) were incubated with GFP-CBD protein in excess for 5 min at room temperature. After washing twice with buffer, the cells were prepared for fluorescence microscopy, using an Axioplan microscope and a filter set with excitation BP 450-490 nm, beamsplitter FT 510 nm, and emission LP 520 nm (Carl Zeiss AG). Pictures of labelled cells were obtained by using a Leica DFC320 camera. For each assay, binding intensity was evaluated by visual inspection, using a four score system: ++, +, (+), and - indicates strong, weak, very weak and no binding, respectively.

**Table 1: Binding of GFP-tagged CBDs and double CBD fusion proteins from different Listeria endolysins to Listeria cells from different species and serovars. 500G118 and 118G500 stand for HCBD500_GFP_118 and HCBD118_GFP_500, respectively. "L" stands for a linker introduced between the shuffled CBDs. ++ strong, + weak, (+) very weak, - no binding; WLSC: Weihenstephan Listeria Strain Collection; SV: Listeria serovar**

| **Species** | **WLSC code** | **Source** | **SV** | **Binding of HGFP_CBD** | | | | | | | | | | |
|---|---|---|---|---|---|---|---|---|---|---|---|---|---|---|
| | | | | **500** | **118** | **P35** | **500-118** | **118-500** | **500L118** | **118L500** | **500G118** | **118G000** | **500-P35** | **P35-500** |
| *L. monocytogenes* | EGDe | J. Kreft | 1/2a | - | ++ | ++ | - | - | + | (+) | (+) | - | ++ | ++ |
| *L. monocytogenes* | 10403S | D. Portnoy | 1/2a | - | ++ | ++ | - | - | - | - | (+) | - | ++ | + |
| *L. monocytogenes* | 1442 | Food | 1/2a | - | +++ | - | - | - | + | + | (+) | + | - | - |
| *L. monocytogenes* | 1066 | SLCC 8800 | 1/2b | - | ++ | ++ | - | - | - | - | (+) | - | ++ | ++ |
| *L. monocytogenes* | 1001 | ATCC 19112 | 1/2c | - | ++ | ++ | - | - | - | - | (+) | - | ++ | ++ |
| *L. seeligeri* | 4007 | ATCC 35967 | 1/2b | - | ++ | ++ | - | - | + | + | (+) | - | ++ | ++ |
| *L. welshimeri* | 50149 | SLCC 5877 | 1/2b | - | ++ | + | - | - | + | + | (+) | - | (+) | (+) |
| *L. monocytogenes* | 1485 | soft cheese | 3a | - | + | + | - | - | - | - | - | - | ++ | ++ |
| *L. monocytogenes* | 1031 | SLCC1694 | 3b | - | + | ++ | - | - | - | - | - | - | ++ | ++ |
| *L. monocytogenes* | 1032 | SLCC 2479 | 3c | - | + | ++ | - | - | - | - | - | - | ++ | ++ |
| *L. seeligeri* | 40127 | SLCC 8604 | 3b | - | + | ++ | - | - | - | - | - | - | ++ | ++ |
| *L. monocytogenes* | 1034 | SLCC 2482 | "7" | - | + | - | - | - | - | - | - | - | - | - |
| *L. monocytogenes* | 1020 | ATCC 19114 | 4a | ++ | - | ++ | + | + | + | ++ | + | ++ | ++ | ++ |
| *L. monocytogenes* | 1042 | ATCC 23074 | 4b | ++ | - | - | + | + | + | ++ | + | ++ | ++ | ++ |
| *L. monocytogenes* | ScottA | J. Jay | 4b | ++ | - | - | + | + | + | ++ | + | ++ | ++ | ++ |
| *L. monocytogenes* | 1019 | ATCC 19116 | 4c | ++ | - | ++ | + | + | + | ++ | + | ++ | ++ | ++ |
| *L. monocytogenes* | 1033 | ATCC 19117 | 4d | ++ | - | + | + | + | + | ++ | + | ++ | ++ | ++ |
| *L. monocytogenes* | 1018 | ATCC 19118 | 4e | ++ | - | (+) | + | + | + | ++ | + | ++ | ++ | ++ |
| *L. ivanovii* | 3009 | SLCC 4769 | 5 | ++ | - | ++ | + | + | + | ++ | + | ++ | ++ | ++ |
| *L. ivanovii (ssp. Ivanovii)* | 3010 | ATCC 19119 | 5 | ++ | - | ++ | + | + | + | ++ | + | ++ | ++ | ++ |
| *L. ivanovii (ssp. londoniensis)* | 3060 | SLCC 3765 | 5 | ++ | - | - | + | + | + | + | (+) | + | + | + |
| *L. innocua* | 2011 | ATCC 33090 | 6a | ++ | - | - | + | + | + | + | (+) | + | ++ | ++ |
| *L. innocua* | 2012 | ATCC 33091 | 6b | ++ | - | ++ | + | + | + | ++ | + | ++ | ++ | ++ |
| *L. welshimeri* | 50146 | SLCC 7622 | 6a | ++ | - | + | + | + | + | ++ | + | ++ | ++ | ++ |
| *L. grayi (ssp. Grayi)* | 6036 | ATCC 19120 | - | (+) | (+) | ++ | - | - | - | - | - | - | + | ++ |
| *L. grayi (ssp. Murrayi)* | 6037 | ATCC 25401 | - | (+) | (+) | ++ | - | - | - | - | - | - | ++ | ++ |

The constructs HGFP_CBD500-118 and HGFP_CBD118-500, in which both cell wall binding domains were directly fused to each other in both orientations, and attached to an N-terminal GFP domain, both showed weak binding to all strains of serovars 4, 5, and 6 strains tested. As this corresponds to the binding pattern of the mere CBD500, these results suggested that CBD118 is not functional in these constructs. On the other hand it showed that CBD500 does not need to be located at the C-terminus of a protein to retain functionality. Assuming that enhanced flexibility of both binding domains in a fusion construct might render CBD118 functional, we created the proteins HGFP_CBD500L118 and HGFP_CBD118L500, which include the linker peptide of PlyPSA separating the CBDs. Again, both constructs labelled all strains belonging to serovar 4, 5, and 6 strains, but additionally also four out of seven serovar 1/2 strains tested. From fluorescence microscopy it was observed that the latter were predominantly marked at the poles and septa as observed for HGFP_CBD118. In contrast, both proteins decorated strains of serovars 4, 5, and 6 in even distribution over the cell surfaces like HGFP_CBD500. Thus, these double CBD constructs combined properties of both CBDs, although their binding ranges within serovars 1/2, 3, and "7" were narrower than that of HGFP_CBD118. Introduction of a short linker not only enabled CBD118 to access its ligands, but it also enhanced binding of CBD500 in C-terminal position. The fusion protein HGFP_CBD118L500 displayed equally strong decoration of most of the serovar 4, 5, and 6 cells as HGFP_CBD500. In addition, two fusion constructs in which the GFP was placed in central position, whereas CBD500 and CBD118 were either N- or C-terminally located. In HCBD500_GFP_CBD118, CBD118 was directly attached to the C-terminus of the GFP, placing it in the same environment as in HGFP_CBD118. This protein was able to mark all serovar 1/2 strains tested, although the decoration was very weak. The construct HCBD118_GFP_CBD500, in which the CBDs were inversely oriented, strongly bound to most of the serovar 4, 5, and 6 strains, but only weakly labelled one serovar 1/2 strain. Again, CBD500 showed stronger binding when located at the C-terminus. However, it was demonstrated to be functional also in N-terminal position (HCBD500_GFP_CBD118).

In a further approach, CBD118 in double CBD fusion constructs was replaced by CBDP35 (SEQ ID NO:59). The CBD of the endolysin of phage P35 strongly labelled most strains of serovars 1/2 and 3 as well as some strains of serovars 4, 5, and 6, binding in even distribution over the complete cell surface. The binding patterns of the newly constructed proteins HGFP_CBD500-P35 and HGFP_CBDP35-500 represented almost exact combinations of those of the single CBDs of Ply500 and PlyP35: They displayed strong binding to all strains which were either bound by CBD500 or by CBDP35 or by both, regardless of the location of the single CBDs within the fusions. These results proved that a combination of two cell wall binding domains from different peptidoglycan lysing enzymes can be fully functional in artificial fusion proteins, even when they are not in C-terminal position.

### Example 4. Determination of binding affinity by surface plasmon resonance analysis (SPR)

Affinities of HGFP_CBD500 (SEQ ID NO:60) and HGFP_CBD500-500 to the cell wall of L. *monocytogenes* WSLC 1042 were determined by surface plasmon resonance analysis, using a BIAcore X instrument and C1 sensor chips (BIAcore, Uppsala, Sweden). The chip surface was activated with the amine coupling method and coated with HGFP-CBD500 molecules in both flow cells (70 µl of 0.5 mg/ml protein in 10 mM sodium acetate buffer, pH 5, at a flow rate of 5 µl/min). Heat inactivated WSLC 1042 cells in HBS buffer (10 mM HEPES, 150 mM NaCl, 3.4 mM EDTA, 0.005% Tween 20, pH 7.8) were then bound to the immobilized CBDs in flow cell Fc2 (3.0 x 10¹⁰ cells per ml; 15 µl at a flow rate of 3 µl/min). Finally, interactions between the immobilized cells and 3 different concentrations of both HGFP_CBD500 (50 nM, 100 nM, 200 nM) and HGFP_CBD500-500 (12.5 nM, 25 nM, 50 nM) in HBS buffer were measured (30 µl at 10 µl/min), Fc1 serving as reference cell. The association phase was measured for 3 min, the dissociation phase for 12 min. All steps were carried out at 25 °C. Evaluation of kinetic data was performed with the BIAevaluation software, version 4.1 (BIAcore), employing a "1:1 binding with mass transfer" model. The equilibrium association constants obtained for three concentrations measured for each protein are given in table 2.

**Table 2: Equilibrium affinity constants (K_{A}) of HGFP_CBD500 and HGFP_CBD500-500 binding to the cell wall of Listeria monocytogenes WSLC 1042.**

| HGFP_CBD500 | | HGFP_CBD500-500 | |
|---|---|---|---|
| Concentration (nM) | K_{A} (M⁻¹) | Concentration (nM) | K_{A} (M⁻¹) |
| 200 | 5.61x10⁸ | 50 | 1.00x10¹⁰ |
| 100 | 6.50x10⁸ | 25 | 5.61x10¹⁰ |
| 50 | 5.96x10⁸ | 12.5 | 2.19x10¹⁰ |
| mean | 6.02x10⁸ | mean | 2.93x10¹⁰ |

The construct HGFP_CBD500-500 comprising the artificial double CBD was shown to bind to the immobilized *Listeria* cells with an approximately 50 fold higher affinity compared to HGFP_CBD500 comprising the natural CBD of the endolysin of phage A500 - ply500. Comparing sensograms of both the single and double CBD protein constructs, it was obvious that both constructs mainly differed in the dissociation phase. Once bound to the cell surface, HGFP_CBD500 detached much more rapidly than HGFP_CBD500-500, resulting in the higher overall affinity of the double CBD construct.

### Example 5. Photometric lysis assays

The lytic activity of wild-type and chimaeric peptidoglycan lysing enzymes was determined by a photometric lysis assay. Substrate cells of *Listeria monocytogenes* strains WSLC 1001 (serovar 1/2 c) and WSLC 1042 (serovar 4 b) were prepared by growing the bacteria in TB medium until late log phase and freezing them in 50-fold concentration in PBS buffer (50 mM NaH₂PO₄, 120 mM NaCl, pH 8.0). The assay was carried out in a total volume of 1 ml, with cells diluted to an initial OD₆₀₀ of approximately 1.0 in PBS. All purified native endolysins and chimeric proteins to be compared were added to the cells in equimolar amounts in a volume of 20 µl, and the OD at 600 nm was measured at intervals of 15 s for maximum 10 minutes. Enzymatic concentrations used ranged from 30 to 152 pmol/ml. For negative control, 20 µl buffer were added to the cells. All assays were carried out in triplicate. Loessner et al. (2002, Mol. Microbiol. 44, 335-349) suggested ionic interaction as the molecular basis for binding of CBDs to their ligands in the cell wall. The CBD of endolysin ply500 showed optimum binding at a NaCl concentration of approximately 100 mM and decreasing binding capacity with increasing salt concentration. Based on that, the lytic activity of his-tagged wild-type ply500 (HPL500) and a construct according to the invention using a duplication of the naturally occurring CBD500 of ply500 H_EAD_CBD500-500 was compared under high salt conditions. The assays were carried out as described above, but using NaCl concentrations (between 1M and 2 M). Photometric curves were normalized and corrected by the data of the control assays (corrected value = value + (1 - control value)). The resulting curves were fitted with the following sigmoid function, using the software SigmaPlot 9.0 (Systat Software, Inc.): f = y0 + a / (1 + exp(-(x - x0) / b))^c. The steepest slope of the function was determined, which corresponds to the relative enzymatic activity.

Surprisingly, at salt concentrations of 1 M NaCl or higher, the peptidoglycan lysing enzyme according to the invention H_EAD_CBD500-500 showed higher lytic activity than the naturally occurring enzyme ply500.

### Example 6. Protein expression and purification of Enterococcus endolysins

The *Enterococcus* endolysins Fab25VL, Fab20VL, Fab20K, and the peptidoglycan lysing enzyme according to the invention EADFab25_CBD25_CBD20 were expressed in and isolated from *E. coli* HMS 174 DE3. Protein expression was performed for 3 h at 37 °C after induction with 1 mM IPTG. The bacterial cell pellet was harvested by centrifugation (5000 rpm, 15 min, 4 °C), resuspended in 25 ml buffer A (25mM Tris, pH 8.0, 500 mM NaCl, 20 mM imidazol, 0.1 % Tween 20, 10 % glycerol), and the cells disrupted in a microfluidizer. Bacterial cell debris was removed by centrifugation (12000 rpm, 5 min, 4 °C). The supernatant was submitted to an ammonium sulphate precipitation to 30 % saturation. The precipitate was collected by centrifugation (12000 rpm, 5 min, 4 °C). The supernatant including the endolyins was applied to hydrophobic chromatography using a 5 ml phenylsepharose column (High Sub FF, Amersham). The column was washed with 10 volumes of buffer B (25mM Tris, pH 7.0, 500 mM NaCl, 30 % ammonium sulphate, 10 % glycerol). The endolysins were eluted with 10 column volumes of buffer C (25mM Tris, pH 7.0, 500 mM NaCl, 10 % glycerol). Protein containing fractions were analyzed for endolysin on Coomassie stained SDS-gels. Endolysin containing fractions were pooled and analyzed for lysis activity in plate lysis assays according to example 7.

### Example 7. Plate lysis assay to test the lysis activity and host range of peptidoglycan lysing proteins against Enterococcus bacteria.

A variety of *Enterococcus* bacteria from the medically relevant species *Enterococcus faecium* and *Enterococcus faecalis* were grown over night at 37 °C in precultures of 3 ml BHI medium. For each strain, 2 ml of the preculture was inoculated into 25 ml fresh medium and incubated up to an OD_{600 nm} of around 1. Bacterial cells were harvested by centrifugation at 4500 rpm for 15 min at 4 °C. The cell pellet was resuspended in 500 µl BHI medium. For the test of lysis activity against heat inactivated cells (table 3), the cells were incubated at 85 °C for 45 min and collected by centrifugation at 1400 rpm. The cell pellet was resuspended in 10 ml LB top agar, and the top agar poured onto LB plates. For the test of lysis activity against living cells (table 4), *Enterococcus* precultures were grown in 1 ml BHI medium over night, the preculture mixed with 10 ml BHI top agar, poured onto LB plates, and incubated for 2 h at 30 °C. The *Enterococcus* endolysins Fab25VL, Fab20K, an equimolar combination of the endolysins Fab25VL and Fab20K, and the artificial enzyme EADFab25_CBD25_CBD20 according to the invention were used. 5 µl peptidoglycan lysing protein solution each were pipetted in spots onto the bacterial lawn immersed in the top agar. The appearance of lysis zones around the spotted protein solutions was analysed after 18 h of incubation of the plates at 30 °C using a four score system: +++, ++, +, and - indicates strong, medium,weak, weak and no lysis, respectively.

**Table 3. Plate lysis assay using peptidoglycan lysing enzymes against heat inactivated Enterococcus cells**

| Species | Strain (ProCC) | Source | Fab25VL | Fab20K | 1:1 25VL:20K | EADFab25_CBD25_CBD20 |
|---|---|---|---|---|---|---|
| E. faecium | 880 | Profos | +++ | - | +++ | +++ |
| E. faecium | 1177 | University hospital | +++ | - | +++ | +++ |
| E. faecium | S1506 | ATCC 20477 | +++ | - | +++ | +++ |
| E. faecium | S1553 | DSMZ 2146 | +++ | - | +++ | +++ |
| E. faecium | S1563 | University hospital | +++ | - | +++ | +++ |
| E. faecium | S1564 | University hospital | +++ | - | +++ | +++ |
| E. faecium | S1565 | University hospital | +++ | - | +++ | +++ |
| E. faecium | S1568 | University hospital | +++ | - | +++ | +++ |
| E. faecium | S1570 | University hospital | +++ | - | +++ | +++ |
| E. faecium | S1634 | Robert Koch Institute | +++ | - | +++ | +++ |
| E. faecalis | 17 | Prof. Stetter | ++ | +++ | +++ | +++ |
| E. faecalis | 1176 | ATCC 19433 | - | +++ | +++ | +++ |
| E. faecalis | S1505 | University hospital | ++ | +++ | +++ | +++ |
| E. faecalis | S1507 | University hospital | ++ | +++ | +++ | +++ |
| E. faecalis | S1552 | DSMZ 2570 | - | +++ | +++ | +++ |
| E. faecalis | S1566 | University hospital | +++ | +++ | +++ | +++ |
| E. faecalis | S1567 | University hospital | ++ | +++ | +++ | +++ |
| E. faecalis | S1569 | University hospital | +++ | +++ | +++ | +++ |
| E. faecalis | S1571 | University hospital | ++ | +++ | +++ | +++ |
| E. faecalis | S1578 | University hospital | - | +++ | +++ | +++ |
| E. faecalis | S2465 | University hospital | +++ | +++ | +++ | +++ |

**Table 4. Plate lysis assay using peptidoglycan lysing enzymes against living Enterococcus cells**

| Species | Strain (ProCC) | Source | Fab25VL | Fab20K | 1:1 25VL:20K | EADFab25_CBD25_CBD20 |
|---|---|---|---|---|---|---|
| E. faecium | 880 | Profos | +++ | - | ++ | ++ |
| E. faecium | 1177 | University hospital | ++ | - | ++ | +++ |
| E. faecium | S1506 | ATCC 20477 | + | - | + | ++ |
| E. faecium | S1553 | DSMZ 2146 | ++ | - | + | +++ |
| E. faecium | S1563 | University hospital | - | - | - | +++ |
| E. faecium | S1564 | University hospital | ++ | - | ++ | +++ |
| E. faecium | S1565 | University hospital | ++ | - | ++ | ++ |
| E. faecium | S1568 | University hospital | ++ | - | ++ | ++ |
| E. faecium | S1570 | University hospital | + | - | - | ++ |
| E. faecium | S1634 | Robert Koch Institute | ++ | - | ++ | +++ |
| E. faecalis | 17 | Prof. Stetter | + | - | - | +++ |
| E. faecalis | 1176 | ATCC 19433 | - | - | - | + |
| E. faecalis | S1505 | University hospital | + | - | + | +++ |
| E. faecalis | S1507 | University hospital | - | - | - | +++ |
| E. faecalis | S1552 | DSMZ 2570 | - | - | - | +++ |
| E. faecalis | S1566 | University hospital | + | - | + | + |
| E. faecalis | S1567 | University hospital | + | - | + | +++ |
| E. faecalis | S1569 | University hospital | + | - | - | +++ |
| E. faecalis | S1572 | University hospital | + | - | - | +++ |
| E. faecalis | S1578 | University hospital | - | - | - | +++ |

It turned out that using heat inactivated *Enterococcus* cells (table 3), the endolysin Fab20K lysed all *E. faecalis* strains with high efficiency, but no strains of *E. faecium.* Fab20 Endolysin seemed to be specific for *E. faecalis* strains. Fab25VL lysed all strains of *E. faecium* with high efficiency, but only two strains of *E. faecalis.* The other *E. faecalis* strains where lysed with medium efficiency or were not lysed at all. The naturally occurring endolysin Fab25VL therefore was not strictly specific to *E. faecium,* but lysed strains from this species more reliably than strains from the species *E. faecalis.* A 1:1 mixture of the two endolysins Fab25VL and Fab20K lysed all strains tested with high efficiency, but the same was achieved using only one enzyme according to the invention, namely EADFab25_CBD25_CBD20 which combines the CBDs of Fab25VL and Fab20K, but has only the EAD of Fab25VL. Using only one enzyme instead of two facilitates enzyme production, reduces costs, and minimizes immunological reactions in therapeutic applications. Performing the assay using living cells (table 4), the advantages using the enzyme according to the invention EADFab25_CBD25_CBD20 were more pronounced. Whereas Fab25VL lysed mainly *E. faecium* cells under these assay conditions, and only some *E. faecalis* cells with low efficiency, the endolysin Fab20K did not work at all. None of the living cells were lysed at all, suggesting that maybe the the cell surface receptors for endolysin binding or the substrate molecules for peptidoglycan lysis were not accessible in living cells. A combination of the two enzymes did not improve the situation, but cell lysis using EADFab25_CBD25_CBD20 gave much better results. All strains tested were lysed at least with low efficiency, but most of the strains were lysed with high efficiency. This unexpected result shows that the artificial peptidoglycan lysing enzymes according to the invention can have favourable effects in specific uses even if a first characterization suggests a pure additive effect of the more than one CBD used in terms of the bacterial host range.

### Example 8. Determination of the minimal bactericidal concentration (MBC) of peptidoglycan lysing enzymes against enterococci.

*Enterococcus faecalis* strain 17 was grown over night at 37 °C in BHI medium. The preculture was diluted 1:10 into 25 ml fresh medium and incubated at 37 °C up to an OD_{600 nm} of around 1. Bacterial cells were harvested by centrifugation at 4500 rpm for 5 min at 4 °C, and the cell pellet was resuspended in lysis buffer (PBS (2.25 mM NaH₂PO₃, 7.75 mM Na₂HPO₃, 150 mM NaCl) including, 2 mM CaCl₂, 10 mM BSA) to a concentration of 10⁵ cfu/ml. Protein solutions of Fab25VL and EADFab25_CBD25_CBD20 (1 mg/ml) were serially diluted to 50 µg/ml, 5 µg/ml, 0.5 µg/ml, 0.05 µg/ml, 0.005 µg/ml, 0.0005 µg/ml, and 0.00005 µg/ml in lysis buffer. 450 µl cell suspension and 50 µl protein solution of each concentration were mixed and incubated for 1 h at 37 °C. As a control, lysis buffer without protein added was incubated. 100 µl of 1:10 and 1:100 dilutions of the lysis samples were plated to LB agar plates, incubated for 1 day at 37 °C and counted for cells surviving the lysis by the peptidoglycan lysing enzymes. The MBC99.9 %, for example, is defined as the lowest enzyme concentration at which the initial bacterial cell concentration is reduced by a factor of 1000. In this case, the cfu/ml of surviving cells had to be lower than 10². It was observed that the MBC against *Enterococcus faecalis* strain 17 was lower using the peptidoglycan lysing enzyme according to the invention EADFab25_CBD25_CBD20 than the naturally occurring enzyme Fab25 VL. The MBC99.9 % was 0.05 µg/ml with EADFab25_CBD25_CBD20 whereas it was 5 µg/ml using Fab25VL. This result suggests a higher binding affinity of EADFab25_CBD25_CBD20 to the bacterial cells and/or an increased lysis activity. Using the polypeptide according to the invention a factor 100 less protein has to be used in order to kill the pathogenic bacteria.

## Claims

1. Recombinant polypeptide having the activity of binding and lysing of bacteria, comprising at least one enzymatically active domain and at least two bacterial cell binding domain.

2. Recombinant polypeptide having the activity of binding bacteria, comprising at least two bacterial cell binding domain.

3. Recombinant polypeptide according to claim 1, comprising two enzymatically active domains.

4. Recombinant polypeptide according to anyone of claims 1, 2 or 3, wherein the enzymatically active domain(s) and/or bacterial cell bindings domain are derived from two different peptidoglycan lysing enzymes.

5. Recombinant polypeptide according to anyone of claims 1 to 4, wherein the enzymatically active domain(s) is/are selected from the group consisting of Amidase_5 (bacteriophage peptidoglycan hydrolase, pfam05382), Amidase_2 (N-acetylmuramoyl-L-alanine amidase, pfam01510), Amidase_3 (N-acetylmuramoyl-L-alanine amidase, pfam01520), Transgly (transglycosylase, pfam00912), Peptidase_M23 (peptidase family M23, pfam01551), endolysin_autolysin (CD00737), Hydrolase_2 (cell wall hydrolase, pfam07486), CHAP (amidase, pfam05257), Transglycosylase (transglycosylase like domain, pfam06737), MtlB (membrane-bound lytic murein transglycosylase B, COG2951), MtlA (membrane-bound lytic murein transglycosylase A, COG2821), MtlE (membrane-bound lytic murein transglycosylase E, COG0741), bacteriophage_lambda_lysozyme (lysis of the bond between N-acetylmuramic acid and N-acetylglucosamine, CD00736), Peptidase_M74 (penicillin-insensitive murein endopeptidase, pfam03411), SLT (transglycosylase SLT, pfam01464), Lys (C-type lysozyme/alpha-lactalbumin family, pfam00062), COG5632 (N-acetylmuramoyl-L-alanine amidase, COG5632), MepA (murein endopeptidase, COG3770), COG1215 (glycosyltransferase, COG1215), AmiC (N-acetylmuramoyl-L-alanine amidase, COG0860), Spr (cell wall-associated hydrolase, COG0791), bacteriophage_T4-like_lysozyme (lysis of the bond between N-acetylmuramic acid and N-acetylglucosamine, cd00735), LT_GEWL (lytic transglycosylase (LT) and goose egg white lysozyme (GEWL) domain, cd00254), peptidase_S66 (LD-carboxypeptidase, pfam02016), Glyco_hydro_70 (glycosyl hydrolase family 70, pfam02324), Glyco_hydro_25 (glycosyl hydrolase familiy 25), VanY (D-alanyl-D-alanine carboxypeptidase, pfam02557), and LYZ2 (lysozyme subfamily 2, smart 00047).

6. Recombinant polypeptide according to anyone of claims 1 to 4, wherein the bacterial cell bindings domains are selected from the group consisting of SH3_5 (bacterial SH3 domain, pfam08460), SH3_4 (bacterial SH3 domain, pfam06347), SH3_3 (bacterial SH3 domain, pfam08239), SH3b (bacterial SH3 domain homologue, smart00287), LysM (LysM domain found in a variety of enzymes involved in cell wall degradation, pfam01476 and cd00118), PG_binding_1 (putative peptidoglycan binding domain, pfam01471), PG_binding_2 (putative peptidoglycan binding domain, pfam08823), MtlA (peptidoglycan binding domain from murein degrading transglycosylase, pfam03462), Cpl-7 (C-terminal domain of Cpl-7 lysozyme, pfam08230), CW_binding_1 (putative cell wall binding repeat, pfam01473), LytB (putative cell wall-binding domain, COG2247), and LytE (LysM repeat, COG1388).

7. Recombinant polypeptide according to anyone of claims 1 to 6, wherein said domains are in the range of about 15 to about 250 amino acid residues long, particular in the range of about 20 to about 200 amino acid residues long and more particular about 15 to about 40 amino acid residues long.

8. Recombinant polypeptide according to anyone of claims 1 to 7, wherein the enzymatically active domain(s) and/or the bacterial cell binding domains are derived from wild-type peptidoglycan lysing enzymes selected from the group consisting of Ply500, Ply511, Ply118, Ply100, PlyP40, Ply3626, phiLM4 endolysin, PlyCD119, PlyPSAa, Ply21, PlyBA, Ply12, PlyP35, PlyPH, PlyL, PlyB, phi11 endolysin, phi MR11 endolysin, phi12 endolysin, *S. aureus* phage PVL amidase , plypitti26, ΦSA2usa endolysin, endolysin of *Staphylococcus warneri* M phage ΦWMY PlyGBS, B30 endolysin, Cpl-1, Cpl-7, Cpl-9, PlyG, PlyC, pal amidase, Fab25, Fab20, endolysins from the *Enterococcus faecalis* V583 prophage, lysostaphin, phage PL-1 amidase, *S. capitis* ALE-1 endopeptidase, mutanolysin (N-acetylmuramidase of *Streptomyces globisporus* ATCC 21553), enterolysin A (cell wall degrading bacteriocin from *Enterococcus faecalis* LMG 2333), LysK, LytM, Ami autolysin from L. *monocytogenes,* endolysins of the *Pseudomonas aeruginosa* phages ΦKZ and EL, T4 lysozyme, gp61 muramidase, and STM0016 muramidase.

9. Recombinant polypeptide as depicted in SEQ ID NO: 7, 9, 13, 15, 17, 19, 21, 23, 31, 33, 35, 37, 39, 41, 43, 45, 47, 49, 51, 71, 73, 75, 77, 79, 81, 83, 85, 87, 89, 91, 93, 95, 97, 99, 101.

10. Nucleic acid molecule comprising a nucleotide sequence sequence encoding for a recombinant polypeptide according to anyone of claims 1 to 9.

11. Nucleic acid molecule according to claim 9, wherein the nucleic acid molecule comprises a nucleotide sequence as depicted in SEQ ID NO: 8, 10, 14, 16, 18, 20, 22, 24, 32, 34, 36, 38, 40, 42, 44, 46, 48, 50, 52, 72, 74, 76, 78, 80, 82, 84, 86, 88, 90, 92, 94, 96, 98, 100, 102.

12. Vector comprising a nucleic acid molecule according to claims 10 or 12.

13. Polypeptide according to anyone of claims 1 to 9 for use as a medicament.

14. Use of the polypeptide according to anyone of claims 1 to 4 as a disinfectant in medical, public or private environment, as a decontaminant of bacterial contamination in food industry, animal feed or cosmetic industry or as a surfactant against bacterially contaminated surfaces.

15. Use of the polypeptide according to anyone of claims 1 to 4 as a diagnostic means in the medicine, food or feed diagnostic or environmental diagnostic.
